# EUROPÄISCHE PATENTSCHRIFT

(11) **EP 2 516 426 B1**
(45) Veröffentlichungstag und Bekanntmachung des Hinweises auf die Patenterteilung: **16.09.2015**
(21) Anmeldenummer: 10793261.8
(22) Anmeldetag: 17.12.2010
(51) Int. Cl.: C07D 417/14, A61K 31/454, A01N 43/78

(54) **BIS(DIFLUORMETHYL)PYRAZOLE ALS FUNGIZIDE**
BIS(DIFLUORMETHYL)PYRAZOLES USED AS FUNGICIDES
BIS(DIFLUOROMÉTHYL)PYRAZOLES UTILISÉS COMME FONGICIDES

(30) Priorität: 21.12.2009 US 288484 P; 21.12.2009 EP 09180073
(43) Veröffentlichungstag der Anmeldung: 31.10.2012
(73) Patentinhaber: Bayer CropScience AG, 40789 Monheim (DE)
(72) Erfinder: CRISTAU, Pierre, F-69009 Lyon (FR); HOFFMANN, Sebastian, 41470 Neuss (DE); KLUTH, Joachim, 40764 Langenfeld (DE); TSUCHIYA, Tomoki, 69009 Lyon (FR); WASNAIRE, Pierre, 40225 Düsseldorf (DE); BENTING, Jürgen, 42799 Leichlingen (DE); PORTZ, Daniela, 52391 Vettweiß (DE); WACHENDORFF-NEUMANN, Ulrike, 56566 Neuwied (DE); HILLEBRAND, Stefan, 41462 Neuss (DE)
(86) Internationale Anmeldenummer: PCT/EP2010/070156
(87) Internationale Veröffentlichungsnummer: WO 2011/076699

(56) Entgegenhaltungen:
- WO-A1-2010/037479
- WO-A2-2008/013622
- WO-A2-2009/094407

## Beschreibung

Die Erfindung betrifft Bis(difluormethyl)pyrazol-Derivate, deren agrochemisch wirksamen Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, in der Tiergesundheit, im Materialschutz sowie im Bereich Haushalt und Hygiene. Die vorliegende Erfindung betrifft weiterhin ein Verfahren zur Herstellung von Bis(difluormethyl)pyrazol-Derivaten.

Es ist bereits bekannt, dass bestimmte substituierte Pyrazol-Derivate als fungizide Pflanzenschutzmittel benutzt werden können (siehe WO 07/014290, WO 08/013925, WO 08/013622, WO 08/091594, WO 08/091580, WO 09/055514, WO 09/094407, WO 09/094445, WO 09/132785, WO 10/037479, WO10/065579, WO10/066353, WO10/123791 siehe auch Patentanmeldungen mit der Anmeldenummer: DE102010000662.9, PCT/EP2010/003499, EP09174510.9, EP09174614.9; EP09180073.0, EP10161264.6, EP10163067.1, EP10164099.3, EP10172486.2, EP10174012.4, EP10189067.1). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Bis(difluormethyl)pyrazol-Derivate die genannten Aufgaben zumindest in Teilaspekten lösen und sich als Pflanzenschutzmittel, insbesondere als Fungizide eignen.

Gegenstand der Erfindung sind Verbindungen der Formel (I), 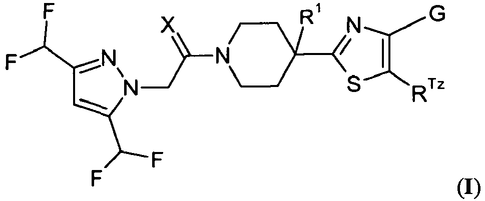 in welcher die Symbole folgende Bedeutungen haben:
- X: steht für Sauerstoff oder Schwefel,
- G: steht für G1 = 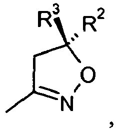 G2 = 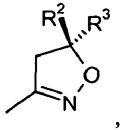 G3 = 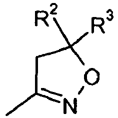 oder G4 = 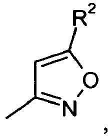
- R¹: steht für Wasserstoff oder Halogen,
- R²: steht für C₁-C₆-Alkyl oder C₁-C₆-Halogenalkyl
oder
- R²: steht für ein C₃-C₈-Cycloalkyl, das bis zu zwei Substituenten enthalten kann, wobei die Substituenten unabhängig voneinander ausgewählt sind aus folgender Liste:
Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
oder
- R²: steht für ein C₅-C₈-Cycloalkenyl, das bis zu zwei Substituenten enthalten kann, wobei die Substituenten unabhängig voneinander ausgewählt sind aus folgender Liste:
Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
oder
- R²: steht für ein Phenyl, das bis zu zwei Substituenten enthalten kann, wobei die Substituenten unabhängig voneinander ausgewählt sind aus folgender Liste:
Amino, Halogen, Cyano, Hydroxy, SH, Nitro, C(=O)H, C₁-C₆-Alkyl, C₂-C₆-Alkenyl, C₂-C₆-Alkinyl, C₁-C₆- Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Aklthio, C₁-C₆- Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -LQ,
oder
- R²: steht für Naphthyl, Dihydronaphthalenyl, Tetrahydronaphthalenyl, Hexahydronaphthalenyl, Octahydronaphthalenyl oder Indenyl, das bis zu zwei Substituenten enthalten kann, wobei die Substituenten unabhängig voneinander ausgewählt sind aus folgender Liste:
Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
- R²: steht für einen 5- oder 6-gliedrigen Heteroarylrest, der bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆- Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -LQ,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halo-gencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
oder
R² und R³ bilden zusammen mit dem Kohlenstoffatom, an das sie gebunden sind, einen 5- bis 12-gliedriges, unsubstituiertes oder substituiertes, teilweise gesättigtes oder ungesättigtes, mono- oder bicyclisches Ringsystem, das bis zu drei weitere Heteroatome, ausgewählt aus N, O und S enthalten kann, wobei zwei Sauerstoffatome nicht benachbart sind und wobei mögliche Substituenten unabhängig voneinander ausgewählt sind aus Halogen, C₁-C₄-Alkyl, C₁-C₄-Halogenalkyl, C₁-C₄-Alkoxy, C₁-C₄-Halogenalkoxy, Oxo, Hydroxy, Benzyl oder Phenyl,
R³ steht für Wasserstoff, Cyano, C₁-C₃-Alkyl oder C₁-C₃-Halogenalkyl,
R^{Tz} steht für Halogen oder Wasserstoff
L steht für eine direkte Bindung, -CH₂-, -(C=O)-, Schwefel oder Sauerstoff,
Q steht für ein Phenyl, das bis zu zwei Substituenten enthalten kann, wobei die Substituenten unabhängig voneinander ausgewählt sind aus folgender Liste:
Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halo-gencycloalkyl, C₂-C₆-Alkoxyalkyl, C₂-C₆-Alkylcarbonyl, C₂-C₆-Halogenalkylcarbonyl, C₂-C₆-Alkoxycarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Alkinyloxy, C₂-C₆-Alkoxyalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Tri(C₁-C₄-alkyl)silyl oder Phenyl,
oder
Q steht für ein 5- oder 6-gliedrigen Heteroarylrest, das bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₁-C₄-Alkoxyalkyl, C₂-C₄-Alkylcarbonyl, C₂-C₆-Alkoxycarbonyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl, C₁-C₄-Alkylsulfonyl, C(=O)H, C(=O)Me, C(=O)OMe oder Phenyl,
sowie agrochemisch wirksame Salze, Metallkomplexe und N-Oxide davon.

Ein weiterer Gegenstand ist die Verwendung der Verbindungen der Formel (I) als Fungizid.

Erfindungsgemäße Bis(difluormethyl)pyrazol-Derivate der Formel (I) sowie deren agrochemisch wirksame Salze, Metallkomplexe und N-Oxide eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel (**I**) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäß verwendbaren Bis(difluormethyl)pyrazol-Derivate sind durch die Formel (**I**) allgemein definiert. Bevorzugte Restedefinitionen der vorstehenden und nachfolgend genannten Formeln sind im Folgenden angegeben. Diese Definitionen gelten für die Endprodukte der Formel (**I**) wie für alle Zwischenprodukte (siehe auch unten unter "Erläuterungen der Verfahren und Zwischenprodukte) gleichermaßen.
- X: steht bevorzugt für Sauerstoff,
- R¹: steht bevorzugt für Wasserstoff oder Fluor,
- G: steht bevorzugt für G1, G3 und G4,
- G: steht besonders bevorzugt für 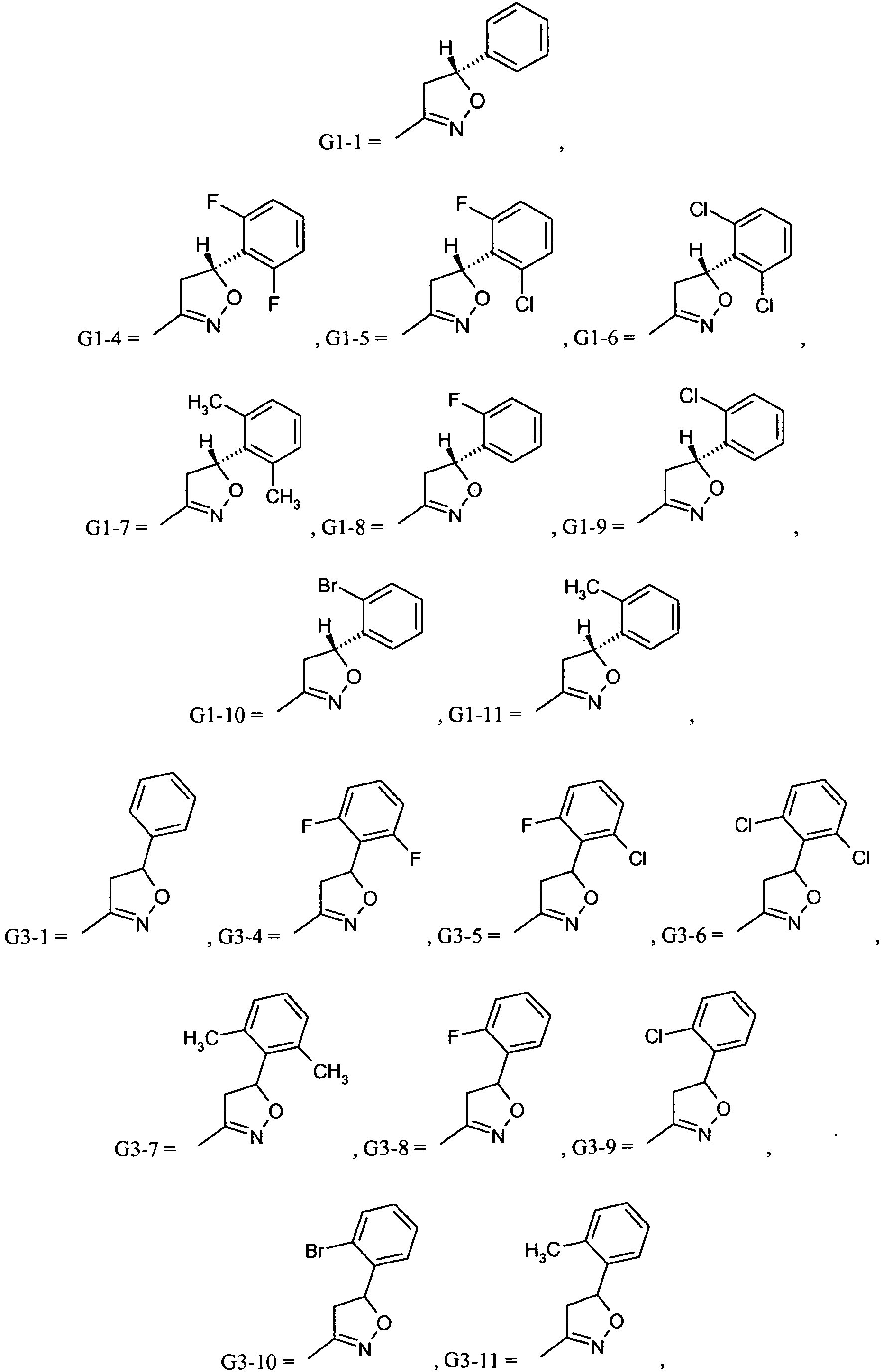 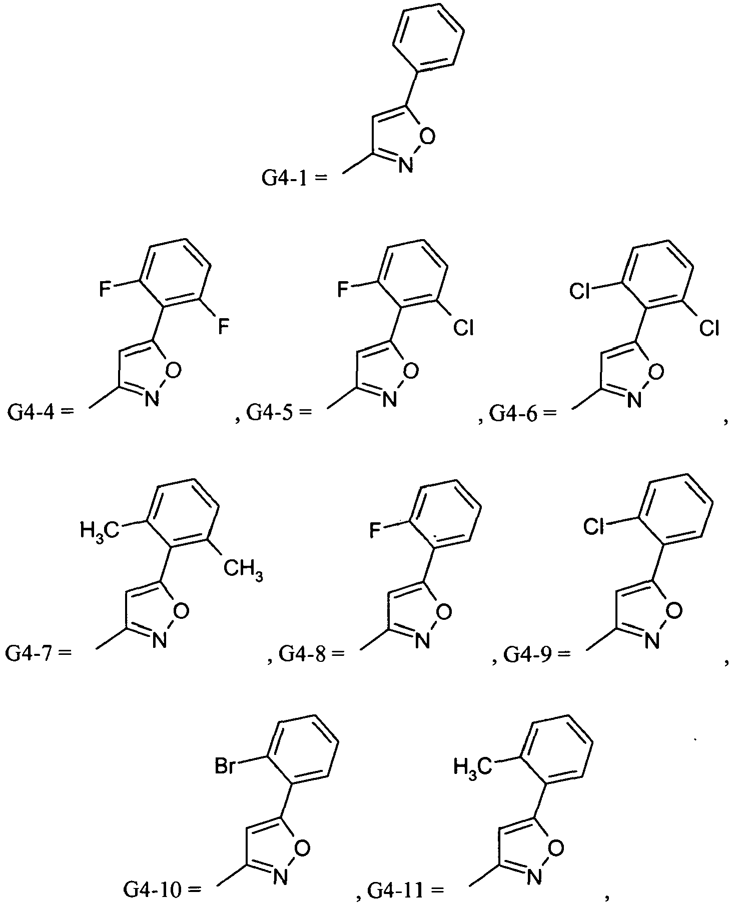
- R²: steht bevorzugt für ein C₃-C₈-Cycloalkyl, das bis zu zwei Substituenten enthalten kann, wobei die Substituenten unabhängig voneinander ausgewählt sind aus folgender Liste:
Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
- R²: steht außerdem bevorzugt für ein Phenyl, das bis zu zwei Substituenten enthalten kann, wobei die Substituenten unabhängig voneinander ausgewählt sind aus folgender Liste:
Amino, Halogen, Cyano, Hydroxy, SH, Nitro, C(=O)H, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -LQ,
- R²: steht außerdem bevorzugt für einen 5- oder 6-gliedrigen Heteroarylrest, der bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -LQ,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halo-gencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
- R²: steht besonders bevorzugt für ein Phenyl, das bis zu zwei Substituenten enthalten kann, wobei die Substituenten unabhängig voneinander ausgewählt sind aus folgender Liste:
Amino, Halogen, Cyano, Nitro, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halo-gencycloalkyl, C₂-C₆-Alkoxyalkyl, C₂-C₆-Alkylcarbonyl, C₂-C₆-Halogenalkylcarbonyl, C₂-C₆-Alkoxycarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Alkinyloxy, C₂-C₆-Alkoxyalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl oder Phenyl,
- R²: steht ganz besonders bevorzugt für ein Phenyl, das bis zu zwei Substituenten enthalten kann, wobei die Substituenten unabhängig voneinander ausgewählt sind aus folgender Liste:
Chlor, Fluor, Brom, Iod, Cyano, Nitro, -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)CH₃, -C(CH₃)₃, -CH=CH₂, -CH=CHCH₃, -CH₂CH=CH₂, -CH=CHCH₂CH₃, -CH₂CH=CHCH₃, -CH₂CH₂CH=CH₂, -C≡CH, -C≡CCH₃, -CH₂C≡CH, - C≡CCH₂CH₃, -CH₂C≡CCH₃, -CH₂CH₂C≡CH, -CF₃, -CFH₂, -CF₂H, -CF₂CF₃, -CCl₃, Cyclopropyl, Cyclobutyl, Cyclopentyl, Cyclohexyl, -CH₂OCH₃, -CH₂OCH₂CH₃, -CH₂CH₂OCH₃, -CH₂OCH₂CH₂CH₃, -CH₂CH₂OCH₂CH₃, -CH₂CH₂CH₂OCH₃, -C(=O)CH₃, -C(=O)CH₂CH₃, C(=O)CH₂CH₂CH₃, C(=O)CH(CH₃)₂, -C(=O)CF₃, -C(=O)OCH₃, -C(=O)OCH₂CH₃, -C(=O)OCH₂CH₂CH₃, -C(=O)OCH(CH₃)₂ -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, -OCF₃, -OCF₂H, -OCH₂CF₃, -OCF₂CF₃, O-Cyclohexyl, O-Cyclopentyl, O-Cyclopropyl, -SCH₃, -SCH₂CH₃, -SCH₂CH₂CH₃, -SCH(CH₃)₂, -SCH₂CH₂CH₂CH₃, -SCH₂CH(CH₃)₂, -SCH(CH₃)CH₂CH₃, -SC(CH₃)₃, -SCF₃, -SCF₂H, -SCH₂CF₃, -SCF₂CF₃, -S(=O)Me, -S(O)CF₃, -S(=O)₂Me, -S(O)₂CF₃, -OCH₂CH=CH₂, -OCH₂C≡CH, -OCH₂OCH₃, -OCH₂OCH₂CH₃, -OCH₂CH₂OCH₃, -OCH₂OCH(CH₃)₂, Trimethylsilyl oder Phenyl,
- R²: steht insbesondere bevorzugt für ein Phenyl, das bis zu zwei Substituenten enthalten kann, wobei die Substituenten unabhängig voneinander ausgewählt sind aus folgender Liste:
Fluor, Chlor, Brom, -CH₃ oder Phenyl,
- R³: steht bevorzugt für Wasserstoff, Cyano oder C₁-C₃-Alkyl,
- R³: steht besonders bevorzugt für Wasserstoff oder Methyl, Ethyl, n-Propyl, iso-Propyl,
- R³: steht ganz besonders bevorzugt für Wasserstoff,
- R^{Tz}: steht bevorzugt für Chlor oder Wasserstoff und besonders bevorzugt für Wasserstoff,
- L: steht bevorzugt für eine direkte Bindung oder Sauerstoff,
- Q: steht bevorzugt für ein Phenyl, das bis zu zwei Substituenten enthalten kann, wobei die Substituenten unabhängig voneinander ausgewählt sind aus folgender Liste:
Halogen, Cyano, Hydroxy, Nitro, SH, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
- Q: steht außerdem bevorzugt für ein 5- oder 6-gliedrigen Heteroarylrest, das bis zu zwei Substituenten enthalten kann, wobei die Substituenten unabhängig voneinander ausgewählt sind aus folgender Liste:
Substituenten am Kohlenstoff:
Halogen, Cyano, Hydroxy, Nitro, SH, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
Substituenten am Stickstoff: C₁-C₆-Alkyl, C₂-C₆-Alkenyl, C₂-C₆-Alkinyl, C₃-C₆-Cycloalkyl oder Phenyl,
sowie agrochemisch wirksame Salze, Metallkomplexe und N-Oxide davon.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Ganz besonders bevorzugt sind solche Verbindungen der Formel (I), in welcher alle Reste jeweils die oben genannten ganz besonders bevorzugten Bedeutungen haben.

Insbesondere bevorzugt sind solche Verbindungen der Formel (I), in welcher alle Reste jeweils die oben genannten insbesondere bevorzugten Bedeutungen haben.

Weiterhin bevorzugt sind Verbindungen der Formel (I), in welcher
- X: für Sauerstoff steht,
- G: für G3 und/oder G4 und insbesondere für G3 steht,
- R¹: für Fluor oder Wasserstoff und insbesondere für Wasserstoff steht,
- R²: für Phenyl, 2-Fluorphenyl, 2,6-Difluorphenyl, 2-Acetylphenyl, 3-Acetylphenyl, 2-Hydroxyphenyl, 2-Nitrophenyl, 2-[(2-Methoxyethoxy)methyl]phenyl, 2-[(Ethylsulfanyl)methyl]phenyl, 2-[(Cyclopropylmethoxy)carbonyl]phenyl, 2-(Allyloxy)phenyl, 3-(But-2-in-1-yloxy)phenyl, 2-(Butoxymethyl)phenyl, 2-Fluor-6-formylphenyl, 2-[(2-Methylprop-2-en-1-yl)oxy]phenyl, 2-(2-Methoxyethoxy)phenyl, 2-[(3-Methylbut-2-en-1-yl)oxy]phenyl, 3-(Prop-2-in-1-yloxy)phenyl, 4-(Prop-2-in-1-yloxy)phenyl, 3-Formylphenyl, 2-(Cyclohexylmethoxy)phenyl, 2-(Pent-2-in-1-yloxy)phenyl, 2-Formylphenyl, 2-(Cyclopropylcarbamoyl)phenyl, 2-(But-2-in-1-yloxy)-6-fluorphenyl, 2-Fluor-6-(prop-2-in-1-yloxy)pheny, 2-[(Cyclohexylcarbonyl)oxy]phenyl, 2-[(Cyclopropylcarbonyl)oxy]phenyl, 3-(Pent-2-in-1-yloxy)phenyl, 2-(But-2-in-1-yloxy)phenyl, 2-[(3,3,3-Trifluorpropanoyl)oxy]phenyl, 2-[(Methylsulfonyl)amino]phenyl, 2-Ethinylphenyl, 2-(Prop-2-in-1-yloxy)phenyl, 4-[(Methylsulfonyl)amino]phenyl, 2-Aminophenyl, 3-Hydroxyphenyl, 2-(Methoxycarbonyl)phenyl, 2-(Chloromethyl)phenyl, 4-(Pent-2-in-1-yloxy)phenyl, 4-(But-2-in-1-yloxy)phenyl, 2-Chlor-6-(prop-2-in-1-yloxy)phenyl, 2-Chlor-6-(2-methoxyethoxy)phenyl, 2-(Allyloxy)-6-chlorphenyl, 2-[(2,2,2-Trifluorethoxy)methyl]phenyl, 2-[(Ethylsulfonyl)methyl]phenyl oder 2-(Hydroxymethyl)phenyl und insbesondere steht R² für Phenyl, 2-Fluorphenyl oder 2,6-Difluorphenyl steht,
- R³: für Wasserstoff steht,
- R^{Tz}: für Wasserstoff steht,
sowie agrochemisch wirksame Salze, Metallkomplexe und N-Oxide davon.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide, herbizide und insektizide Eigenschaften auf

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc. in Frage.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod;
**Alkyl:** gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Alkenyl:** ungesättigte, geradkettige oder verzweigte Kohlenwasserstoffreste mit 2 bis 8 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl:** geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkoxy:** gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methyl-propoxy;
**Alkylthio:** gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Alkylsulfinyl:** gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dünethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
**Alkylsulfonyl:** gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
**Cycloalkyl:** monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl;
**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluor-ethoxy und 1,1,1-Trifluorprop-2-oxy;
**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, Pentafluorethylthio und 1,1,1-Trifluorprop-2-ylthio;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes be.nzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome: :** z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph, -OSO₂PhMe);

Bei der Nennung von Kombinationen von mehreren Resten, wie zum Beispiel Cx-Cy-Alkylcarbonyl oder Cx-Cy-Alkoxyalkyl, bezeichnet der Ausdruck Cx-Cy jeweils die Summe aller Kohlenstoffatome, die jeweils in dem gesamten Fragment vorhanden sind. X und Y stehen dabei jeweils für eine ganze Zahl, wobei die Zahl Y größer ist als diejenige von X.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Verfahren und Zwischenprodukte

Die Bis(difluormethyl)pyrazol-Derivate der Formel (I) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

### Verfahren A

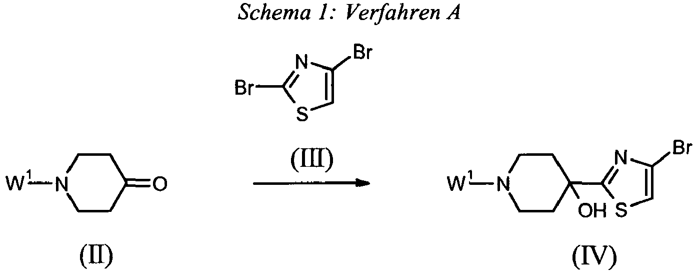

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

Das Verfahren A beschreibt die Herstellung von Verbindungen der Struktur (**IV**) durch Reaktion von Verbindungen der Struktur (**II**) mit (**III**).

Eine Möglichkeit das Zwischenprodukt (**IV**) aus Verbindung (**II**) herzustellen ist in Schema 1 (Verfahren A) gezeigt. Eine Verbindung der allgemeinen Formel (**IV**) wird aus einer Verbindung der allgemeinen Formel (**III**) durch Halogen-Metall Austausch und folgender Zugabe einer Verbindung der Formel (**II**) erhalten (s. z.B. Org. Lett. 2004, 6, 3083-3085).

Das Verfahren A wird in Gegenwart einer geeigneten metallorganischen Verbindung durchgeführt. Bevorzugte metallorganische Verbindungen sind Organolithium-Verbindungen (wie z.B. Butyllithium) oder Grignard Reagenzien (wie z.B. Isopropylmagnesiumhalogenid).

Das Verfahren A wird vorzugsweise unter Verwendung eines oder mehrer Verdünnungsmittel durchgeführt. Bei der Durchführung vom Verfahren kommen vorzugsweise aprotische Lösungsmittel infrage, wie z.B. Dioxan, Glyme, Diethylether oder Tetrahydrofuran. Besonders bevorzugt ist die Verwendung von Tetrahydrofuran.

Die Reaktionstemperaturen können bei der Durchführung des Verfahrens A in einem größeren Bereich variiert werden. Im Falle der Halogen-Metall Austauschreaktionen arbeitet man im Allgemeinen bei Temperaturen von -120 °C bis +150 °C, vorzugsweise bei Temperaturen von -120 °C bis +60 °C, ganz besonders bevorzugt bei -120 °C bis 0 °C. Nach der Zugabe der Verbindung (**II**) arbeitet man bevorzugt bei -80°C bis +50°C.

Zur Durchführung des Verfahrens A setzt man pro Mol an Verbindung der Formel (**III**) im Allgemeinen 1 bis 2 Mol, vorzugsweise 1 Mol der metallorganischen Verbindung ein. Die Reaktionsdauer beträgt 1 bis 48 Stunden. Die Aufarbeitung erfolgt nach den üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren B

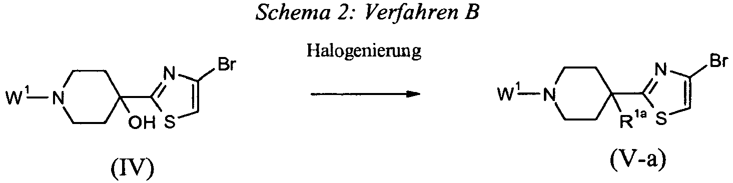

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

R^{1a} steht für F, Cl, Br, I

Das Verfahren B beschreibt die Herstellung von Verbindungen der Struktur (**V**-**a**) durch Halogenierung von Verbindungen der Struktur (**IV**).

Eine Verbindung der allgemeinen Formel (**V-a**), bei der R^{1a} = F, Cl, Br und I ist, wird aus einer Verbindung der allgemeinen Formel (**IV**) durch Halogenierung erhalten (s. z.B. WO 06/133216, WO 04/108692, J. Med. Chem., 1991, 34, 108-122, EP0796846, J. Antibiot., 1988, 41, 134-138, Bioorg. Med. Chem. Lett., 2008, 18, 5209-5212, Chem. Eur. J., 2004, 5640-5648, Russ. J. Org. Chem., 2007, 50-55).

Als Lösungsmittel können alle üblichen, unter den Reaktionsbedingungen inerten Lösungsmittel, verwendet werden oder die Reaktion kann in Mischungen von zwei oder mehreren dieser Lösungsmittel ausgeführt werden. Bevorzugt wird das Lösungsmittel Dichlormethan verwendet.

Als Halogenquelle kann z.B. Diethylaminoschwefeltrifluorid, Selectfluor, Deoxofluor, Thionylchlorid, PBr₃ und Methansulfonylchlorid verwendet werden.

Die Ausgangsmaterialien und das Halogenierungsmittel werden in equimolaren Mengen eingesetzt. Das Halogenierungsmittel kann auch im Überschuss verwendet werden. Die Reaktion wird normalerweise bei Temperaturen von -80 °C bis +80 °C durchgeführt und vorzugsweise bei 0 °C bis +40 °C, aber sie kann auch bei Rückflussteniperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion werden die Verbindungen (V-a) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren C

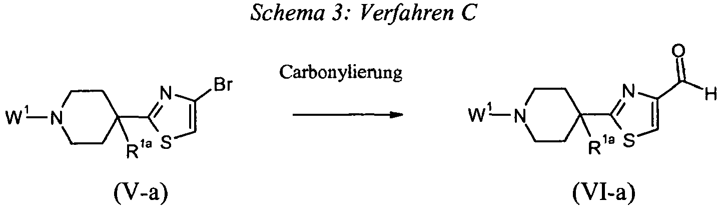

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

R^{1a} steht für F, Cl, Br, I

Eine Möglichkeit das Zwischenprodukt (**VI-a**) aus Verbindung (**V-a**) herzustellen ist in Schema 3 (Verfahren C) gezeigt.

Eine Verbindung der allgemeinen Formel (**VI-a**), wird aus einer Verbindung der allgemeinen Formel (**V-a**) durch Halogen-Metall Austausch und folgender Zugabe eines Elektrophils (z.B. DMF) erhalten, siehe beispielsweise Tetrahedron, 2006, 62, 9017-9037; Org. Lett. 2005, 7, 339-342; Synthesis, 1987, 11, 998-1001.

Das Verfahren C wird in Gegenwart einer geeigneten metallorganischen Verbindung durchgeführt. Bevorzugte metallorganische Verbindungen sind Organolithium-Verbindungen (wie z.B. Butyllithium).

Das Verfahren C wird vorzugsweise unter Verwendung eines oder mehrer Verdünnungsmittel durchgeführt. Bei der Durchführung des Verfahrens C kommen vorzugsweise aprotische Lösungsmittel infrage (wie z.B. Dioxan, Glyme, Diethylether oder Tetrahydrofuran). Besonders bevorzugt ist Diethylether.

Die Reaktionstemperaturen können bei der Durchführung des Verfahrens A in einem größeren Bereich variiert werden. Im Falle der Halogen-Metall Austauschreaktionen arbeitet man im Allgemeinen bei Temperaturen von -120 °C bis +150 °C, vorzugsweise bei Temperaturen von -120 °C bis +60 °C, ganz besonders bevorzugt bei -120 °C bis -70 °C. Nach der Zugabe der Verbindung (II) arbeitet man bevorzugt bei -80°C bis +50°C.

Zur Durchführung des Verfahrens C setzt man pro Mol an Verbindung der Formel (**V-a**) im Allgemeinen 1 bis 2 Mol, vorzugsweise 1 Mol der metallorganischen Verbindung und des Elektrophils ein. Die Reaktionsdauer beträgt 1 bis 48 Stunden. Die Aufarbeitung erfolgt nach üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren D

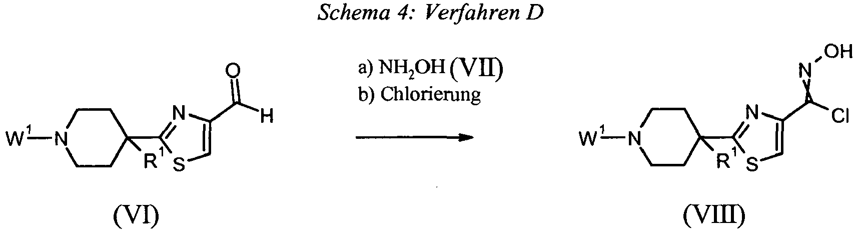

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

Eine Möglichkeit das Zwischenprodukt (**VIII**) aus Verbindung (**VI**) herzustellen ist in Schema 4 (Verfahren D) gezeigt

Eine Verbindung der allgemeinen Formel (**VIII**) wird durch Kondensation eines Aldehyds der Formel (**VI**) mit Hydroxylamin (**VII**) und anschließender Chlorierung erhalten (s. z.B. WO 05/0040159, WO 08/013622 und Synthesis 1987, 11, 998-1001).

Im Verfahren D wird zunächst Aldehyd (**VI**) (**VI** mit R¹ = H ist verfügbar bei Maybridge) und Hydroxylamin (**VII**) zur Reaktion gebracht (Schema 4, Schritt (a)). Das korrespondierende Oxim wird anschließend in Gegenwart eines geeigneten Chlorierungsmittels chloriert. Bevorzugte Chlorierungsreagenzien sind N-Chlorsuccinimid, HClO und Chlor. Nach dem Schritt (a) des Verfahrens D kann die Reaktionsmischung nach üblichen Methoden aufgearbeitet oder direkt im Schritt (b) weiter umgesetzt werden.

Das Verfahren D wird vorzugsweise unter Verwendung eines oder mehrer Verdünnungsmittel durchgeführt. Im Schritt (a) des erfindungsgemäßen Verfahrens D werden bevorzugt protische Lösungsmittel, wie z.B. Ethanol, als Lösungsmittel verwendet. Nach der Bildung des korrespondierenden Oxims aus Verbindung (**VI**) wird die Reaktionsmischung im Schritt (b) mit einem weiteren Lösungsmittel verdünnt, z.B. Tetrahydrofuran, und anschließend mit wässrigem Natriumhypochlorit versehen. Ebenso kann die Chlorierung mit Hilfe von N-Chlorsuccinimid in DMF erfolgen.

Die Reaktionstemperaturen können bei der Durchführung des Verfahrens D in einem größeren Bereich variiert werden. Im Allgemeinen arbeitet man bei Temperaturen von -10 °C bis +150 °C, vorzugsweise bei Temperaturen von 0°C bis +100°C, ganz besonders bevorzugt bei Rückflusstemperatur des Lösungsmittels im Schritt (a) und bei 0 °C bis 30 °C im Schritt (b).

Zur Durchführung des Verfahrens D setzt man pro Mol an Verbindung der Formel (**VI**) im Allgemeinen 1 bis 2 Mol, vorzugsweise 1 Mol Hydroxylamin (**VII**) und im Allgemeinen 1 bis 5 Mol, vorzugsweise 1 Mol eines Chlorierungsreagenz ein. Die Reaktionsdauer beträgt 1 bis 48 Stunden. Die Aufarbeitung erfolgt nach üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren E

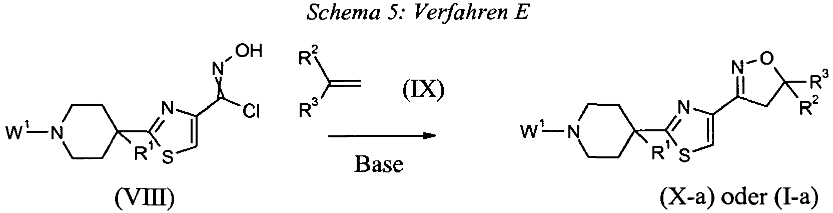

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

Eine Möglichkeit das Zwischenprodukt (**X**-**a**) oder die erfindungsgemäßen Verbindungen der Formel (**I-a**) aus Verbindung (**VIII**) herzustellen ist in Schema 5 (Verfahren E) gezeigt.

Eine Verbindung der allgemeinen Formel (**X-a**) oder (**I-a**) wird aus einem Alken der allgemeinen Formel (**IX**) und Verbindung (**VIII**) durch eine Zykloadditionsreaktion erhalten (s. z.B. WO08/013622 und Synthesis, 1987, 11, 998-1001).

Die Alkene (**IX**) sind kommerziell verfügbar oder können aus kommerziell erhältlichen Vorstufen nach literaturbeschriebenen Vorschriften hergestellt werden (z.B. aus Ketone oder Aldehyde durch eine Wittig oder Horner-Wadsworth-Emmons Olefinierung: Chem. Rev. 1989, 89, 863-927 und Julia Olefinierung: Tetrahedron Lett., 1973, 14, 4833-4836; Peterson-Olefinierung: J. Org. Chem. 1968, 33, 780).

Das Verfahren E wird in Gegenwart einer geeigneten Base durchgeführt. Bevorzugte Basen sind tertiäre Amine (z.B. Triethylamin), Alkalimetall- oder Erdalkalimetallcarbonate, Hydrogencarbonate und Phosphate.

Das Verfahren E wird vorzugsweise unter Verwendung eines oder mehrer Verdünnungsmittel durchgeführt. Bei der Durchführung des Verfahrens E kommen vorzugsweise inerte organische Lösungsmittel infrage (wie z.B. Toluol und Hexan). Ebenso kommt Wasser als Lösungsmittel infrage. Alternativ kann das Verfahren E in einem Überschuß des Alkens (**IX**) durchgeführt werden.

Typischerweise wird eine geeignete Base und das Olefin (**IX**) vorgelegt und Verbindung (**VIII**) zugegeben. Alternativ werden die Verbindungen (**VIII**) und (**IX**) vorgelegt und eine geeignete Base zugegeben.

Die Reaktionstemperaturen können bei der Durchführung des Verfahrens E in einem größeren Bereich variiert werden. Im Allgemeinen arbeitet man bei Temperaturen von -120 °C bis +150 °C, vorzugsweise bei Temperaturen von -10 °C bis +100 °C, ganz besonders bevorzugt bei 0 °C bis 30 °C.

Zur Durchführung des Verfahrens E setzt man pro Mol an Verbindung der Formel (**VIII**) im Allgemeinen 0,5 bis 5 Mol, vorzugsweise 1 Mol des Alkens (**IX**) ein. Die Reaktionsdauer beträgt 1 bis 48 Stunden. Die Aufarbeitung erfolgt nach üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren F

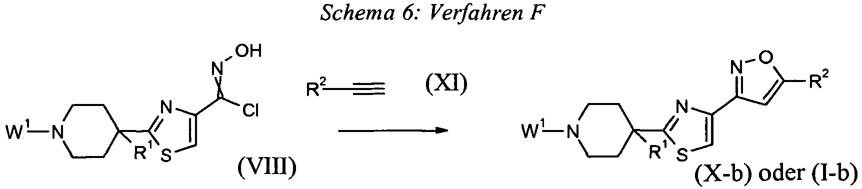

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

Eine Möglichkeit das Zwischenprodukt (**X-b**) oder die erfindungsgemäßen Verbindungen der Formel (**I-b**) aus Verbindung (**VIII**) herzustellen ist in Schema 6 (Verfahren F) gezeigt.

Eine Verbindung der allgemeinen Formel (**X-b**) oder (**I-b**) wird aus einem Alkin der allgemeinen Formel (**XI**) und Verbindung (**VIII**) durch eine Cykloadditionsreaktion erhalten (s. z.B. WO 08/013622, WO 05/040159 und Synthesis, 1987, 11, 998-1001).

Die Alkine **(XI)** sind kommerziell verfügbar oder können aus kommerziell erhältlichen Vorstufen nach literaturbeschriebenen Vorschriften hergestellt werden (z.B. aus Ketone oder Aldehyde durch eine Corey-Fuchs-Reaktion: Tetrahedron Lett. 1972, 36, 3769-3772, Seyferth-Gilbert-Homologisierung: J. Org. Chem., 1996, 61, 2540-2541, oder mit Bestmann-Ohira's Reagenz: Synthesis 2004, 1, 59-62).

Das Verfahren F wird in Gegenwart einer geeigneten Base durchgeführt. Bevorzugte Basen sind tertiäre Amine (z.B. Triethylamin), Alkalimetall- oder Erdalkalimetallcarbonate, Hydrogencarbonate und Phosphate.

Das Verfahren F wird vorzugsweise unter Verwendung eines oder mehrer Verdünnungsmittel durchgeführt. Bei der Durchführung des Verfahrens F kommen vorzugsweise inerte organische Lösungsmittel infrage, wie z.B. Toluol und Hexan. Ebenso kommt Wasser als Lösungsmittel infrage. Alternativ kann das Verfahren F in einem Überschuß des Alkins (**XI**) durchgeführt werden.

Typischerweise wird eine geeignete Base und das Alkin (**XI**) vorgelegt und Verbindung (**VIII**) zugegeben. Alternativ werden die Verbindungen (**VIII**) und (**XI**) vorgelegt und eine geeignete Base zugegeben.

Die Reaktionstemperaturen können bei der Durchführung des Verfahrens F in einem größeren Bereich variiert werden. Im Allgemeinen arbeitet man bei Temperaturen von -120 °C bis +150 °C, vorzugsweise bei Temperaturen von -10 °C bis +100 °C, ganz besonders bevorzugt bei 0 °C bis 30 °C.

Zur Durchführung des Verfahrens F setzt man pro Mol an Verbindung der Formel (**VIII**) im Allgemeinen 0,5 bis 5 Mol, vorzugsweise 1 Mol des Alkins (**XI**) ein. Die Reaktionsdauer beträgt 1 bis 48 Stunden. Die Aufarbeitung erfolgt nach üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren G

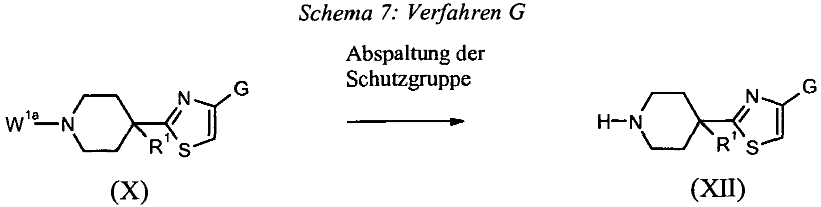

W^{1a} steht für Acetyl, C₁-C₄-Alkoxycarbonyl, Benzyl oder Benzyloxycarbonyl

Eine Möglichkeit Verbindungen der Formel (**XII**) aus entsprechenden Verbindungen (X) darzustellen ist in Schema 7 gezeigt.

Eine Verbindung der Formel (**X**) wird in eine Verbindung der Formel (**XII**) durch geeignete Methoden zur Entfernung von Schutzgruppen, die in der Literatur beschrieben sind ("Protective Groups in Organic Synthesis"; Third Edition; 1999; 494-653, und dort zitierte Literatur), überführt.

*tert*-Butoxycarbonyl und Benzyloxycarbonyl Schutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxan), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Säuren, die für diese Reaktion, der Entschützung von t-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis"; Third Edition; 1999; S. 494-653).

Die Reaktion wird normalerweise bei Temperaturen von 0° C bis +150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion werden die Verbindungen (**XII**) von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden. Es ist außerdem möglich, die Verbindung der allgemeinen Formel (**XII**) als Salz zu isolieren, z.B. als Salz der Salzsäure oder der Trifluoressigsäure.

### Verfahren H

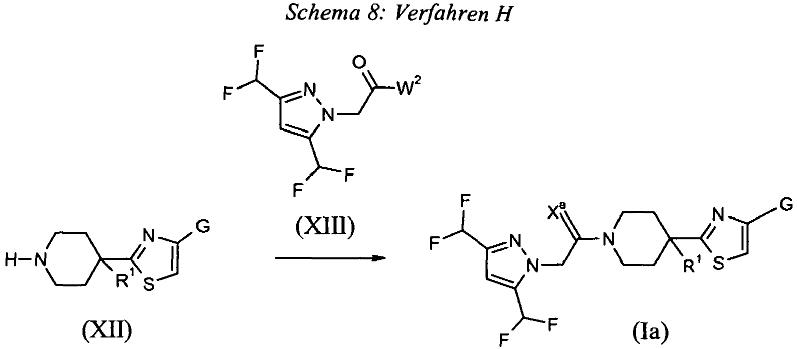

W² steht für Chlor oder OH

X^{a} steht für Sauerstoff

Eine Möglichkeit, Verbindungen der Formel (**Ia**), aus entsprechenden Verbindungen (**XII**) darzustellen, ist in Schema 8 gezeigt.

Eine Verbindung mit der allgemeinen Formel (**Ia**), kann synthetisiert werden, analog zu in der Literatur beschriebenen Vorschriften (s. z.B. WO 07/147336), durch eine Kupplungsreaktion einer Verbindung mit der entsprechenden allgemeinen Formel (**XII**) mit einem Substrat der allgemeinen Formel (**XIIIa**) mit W^{2a} = Chlor, gegebenenfalls in der Gegenwart eines Säurefängers / Base.

Verbindungen (**XIIIa**) (W^{2a} = Chlor) oder (**XIIIb**) (W^{2b} = OH) sind entweder kommerziell erhältlich oder durch in der Literatur beschriebene Prozesse darstellbar (s. z.B. WO 08/013622 und WO 08/013925). Außerdem kann ein Substrat mit der allgemeinen Formel (**XIIIa**), mit W^{2a} = Chlor, aus der entsprechenden Säure (W^{2b} = OH) durch Chlorierung unter Verwendung literaturbekannter Prozesse dargestellt werden. (z.B. Tetrahedron 2005, 61, 10827-10852, und darin zitierte Literatur).

Die Substituenten an R² können auf allen Stufen der Synthese, in denen sie vorkommen, nach für den Fachmann allgemein bekannten Reaktionenvorschriften modifiziert werden. So können beispielsweise OH-, NH2- oder SH-Funktionalitäten nach bekannten Methoden mit geeigneten Halogeniden oder Sulfaten alkyliert (siehe z.B. J. March: Advanced Organic Chemistry - Reactions, Mechanisms, and Structures, 4th Ed. (1992), Wiley, New York, Seiten 388-390, 406-407, 411-415), unter Verwendung von geeigneten Carbonsäuren, Carbonsäurechloriden oder Carbonsäureanhydriden acyliert (siehe J. March: Advanced Organic Chemistry - Reactions, Mechanisms, and Structures, 4th Ed. (1992), Wiley, New York, Seiten 392-400, 409, 417-419) oder unter Verwendung von geeigneten Sulfonylchloriden sulfonyliert werden (siehe J. March: Advanced Organic Chemistry- Reactions, Mechanisms, and Structures, 4th Ed. (1992), Wiley, New York, Seiten 496-500). Auch können durch Hydroxyverbindungen mit Halogenierungsmitteln in entsprechende Halogenide überführt werden (siehe z.B. J. March: Advanced Organic Chemistry - Reactions, Mechanisms, and Structures, 4th Ed. (1992), Wiley, New York, Seiten 431-434). Diese Halogenide wiederum können mit Hilfe von geeigneten Hydroxyverbindungen verethert werden (siehe z.B. J. March: Advanced Organic Chemistry - Reactions, Mechanisms, and Structures, 4th Ed. (1992), Wiley, New York, Seiten 388-390). Außerdem können Carbonyl-Funktionalitäten nach geläufigen Methoden zu entsprechenden Hydroxyverbindungen reduziert werden (siehe z.B. J. March: Advanced Organic Chemistry - Reactions, Mechanisms, and Structures, 4th Ed. (1992), Wiley, New York, Seiten 443, 910-918) oder im Falle von Aldehyden zu Carbonsäuren oxidiert werden (siehe z.B. J. March: Advanced Organic Chemistry - Reactions, Mechanisms, and Structures, 4th Ed. (1992), Wiley, New York, Seiten 701-703), die wiederum in die entsprechenden Ester überführt werden können (siehe z.B. J. March: Advanced Organic Chemistry - Reactions, Mechanisms, and Structures, 4th Ed. (1992), Wiley, New York, Seiten 393-396). Schließlich können Thioether unter Verwendung geeigneter Oxidationsmittel zu Sulfoxiden oder Sulfonen oxidiert werden (siehe z.B. J. March: Advanced Organic Chemistry - Reactions, Mechanisms, and Structures, 4th Ed. (1992), Wiley, New York, Seiten 1201-1202). Beispiele für derartige Reaktionen finden sich im Syntheseteil dieser Anmeldung.

Als Lösungsmittel können alle üblichen, unter den Reaktionsbedingungen inerten Lösungsmittel, wie z.B. zyklische und azyklische Ether (z.B. Diethylether, Tetrahydrofuran, Dioxan), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig Base, Triethylamin oder kommerziell erhältliche polymere Säurefänger) im Verhältnis zum Startmaterial der allgemeinen Formel (**XII**) wird verwendet. Ist das Startmaterial ein Salz, werden wenigstens zwei Äquivalente des Säurefängers benötigt.

Die Reaktion wird normalerweise bei Temperaturen von 0 °C bis 100 °C durchgeführt und vorzugsweise bei 20 °C bis 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden. Nach Beendigung der Reaktion, werden die Verbindungen (**I**), von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Alternativ kann eine Verbindung der Formel (**Ia**), auch aus der entsprechenden Verbindung der Formel (**XII**) mit einem Substrat der Formel (**XIIIb**) mit W^{2b} = OH in Gegenwart eines Kupplungsreagenzes synthetisiert werden analog zu in der Literatur beschriebenen Vorschriften (z.B. Tetrahedron 2005, 61, 10827-10852, und darin zitierte Referenzen).

Geeignete Kupplungsreagenzien sind beispielsweise Peptidkupplungsreagenzien (zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.)

Gegebenenfalls kann eine Base, wie z.B. Triethylamin oder Hünig-Base in der Reaktion verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxan), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und Amide (z.B. N,N-Dimethylformamid, N,N-Dimethylacetamid) verwendet werden oder die Reaktion kann in Mischungen von zwei oder mehreren dieser Lösungsmittel ausgeführt werden. Das bevorzugte Lösungsmittel ist Dichlormethan.

Die Reaktion wird normalerweise bei Temperaturen von 0 °C - 100 °C durchgeführt und vorzugsweise bei 0 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**Ia**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren I

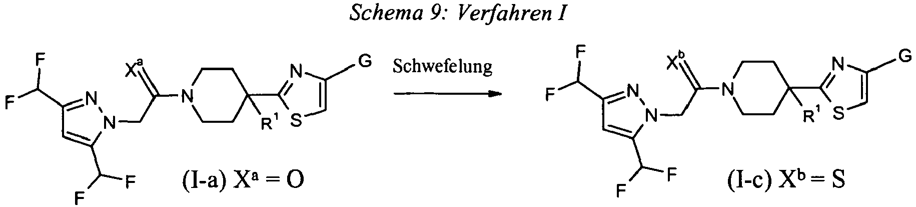

Eine Möglichkeit Verbindungen der Formel **(I-c)**, in denen X^{b} = Schwefel ist aus entsprechenden Verbindungen (**Ia**), in denen X^{a} gleich Sauerstoff ist, darzustellen ist in Schema 9 gezeigt.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxan), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester) und Amide (z.B. N,N-Dimethylformamid, N,N-Dimethylacetamid) verwendet werden und die Reaktion kann in Mischungen von zwei oder mehreren dieser Lösungsmittel ausgeführt werden. Die bevorzugten Lösungsmittel sind Chloroform und 1,2-Dimethoxyethan

Geeignete Schwefelungsreagenzien sind beispielsweise Lawesson's Reagenz (s. Tetrahedron 1986, 42, 6555-6564, Tetrahedron Lett. 1993, 46, 7459-7462) und Phosphorpentasulfid. Das Startmaterial und das Schwefelungsreagenz werden in equimolaren Mengen verwendet, aber das Schwefelungsreagenz kann gegebenenfalls auch im Überschuss verwendet warden.

Die Reaktion wird normalerweise bei Temperaturen von 0 °C bis 150 °C durchgeführt und vorzugsweise bei 0 °C bis 100 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **(Ib)**, in denen X^{b} = Schwefel, von der Reaktionsmischung durch eine der üblichen Trenntechniken getrennt. Falls notwendig werden die Verbindungen durch Umkristallisation, Destillation oder Chromatographie gereinigt.

### Verfahren J

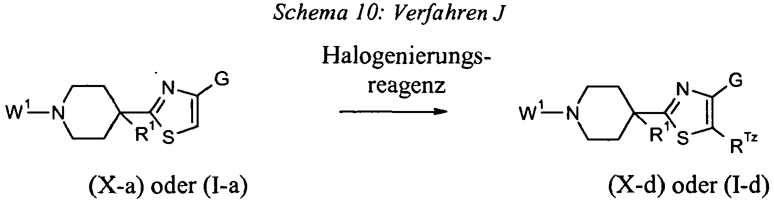

W¹ steht für Acetyl, C₁-C₄-Alkoxycarbonyl, Benzyl, Benzyloxycarbonyl oder [3,5-Bis(difluormethyl)-1H-pyrazol-1-yl]acetyl

R^{TZ} steht für ein Halogen

Eine Möglichkeit das Zwischenprodukt (**X-d**) oder oder die erfindungsgemäßen Verbindungen der Formel (**I-d**) aus Verbindung (**X-a**) oder (**I-a**) herzustellen ist in Schema 10 (Verfahren J) gezeigt.

Eine Verbindung der allgemeinen Formel (**X-d**) oder (**I-d**) wird aus Verbindung (**X**-**a**) oder (**I**-**a**) durch Halogenierung erhalten (s. z.B. WO 08/013622, WO 05/040159 und Synthesis, 1987, 11, 998-1001).

Als Lösungsmittel können alle üblichen, unter den Reaktionsbedingungen inerten Lösungsmittel, wie zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäuren (z.B. Essigsäure) und Carbonsäureester (z.B. Essigsäureethylester) verwendet werden und die Reaktion kann in Mischungen von zwei oder mehreren dieser Lösungsmittel ausgeführt werden. Die bevorzugten Lösungsmittel sind Chloroform und Essigsäure.

Bevorzugte Halogenierungsreagenzien sind beispielsweise N-Chlorsuccinimid, HClO und Chlor (Chlorierungsreagenzien), N-Bromsuccinimid, HBrO und Brom (Bromierungsreagenzien), N-Fluorodibenzenesulfonimide (NFSI) und F₂ (Fluorierungsreagenzien) oder N-Iodsuccinimid, ICl und Iod (Iodierungsreagenzien, siehe J. March: Advanced Organic Chemistry - Reactions, Mechanisms, and Structures, 4th Ed. (1992), Wiley, New York, Seiten 531-534; D. Kikelj, U. Urleb in Science of Synthesis, 11 (2001), Seiten 749-751). Das Startmaterial und das Halogenierungsreagenz werden in equimolaren Mengen verwendet, aber das Halogenierungsreagenz kann gegebenenfalls auch im Überschuss verwendet warden.

Die Reaktion wird normalerweise bei Temperaturen von von -10 °C bis +200 °C, durchgeführt und vorzugsweise bei 0 °C bis 100 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**X-d**) oder (**I**-**d**) von der Reaktionsmischung durch eine der üblichen Trenntechniken getrennt. Falls notwendig werden die Verbindungen durch Umkristallisation, Destillation oder Chromatographie gereinigt.

Ein weiterer Gegenstand der Erfindung betrifft die nichtmedizinische Verwendung der erfindungsgemäßen Bis(difluormethyl)pyrazol Derivate zum Bekämpfen unerwünschter Mikroorganismen.

Ein weiterer Gegenstand der Erfindung betrifft ein Mittel zum Bekämpfen unerwünschter Mikroorganismen, umfassend wenigstens ein Bis(difluormethyl)pyrazol Derivate gemäß der vorliegenden Erfindung.

Außerdem betrifft die Erfindung ein Verfahren zum Bekämpfen unerwünschter Mikroorganismen, dadurch gekennzeichnet, dass die erfindungsgemäßen Bis(difluormethyl)pyrazol Derivate auf die Mikroorganismen und/oder in deren Lebensraum ausgebracht werden.

Weiterhin betrifft die Erfindung ein Saatgut, welches mit wenigstens einem erfindungsgemäßen Bis(difluormethyl)pyrazol Derivate behandelt wurde.

Ein letzter Gegenstand der Erfindung betrifft ein Verfahren zum Schutz von Saatgut vor unerwünschten Mikroorganismen durch Verwendung eines mit wenigstens einem Bis(difluormethyl)pyrazol Derivate gemäß der vorliegenden Erfindung behandelten Saatgutes.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Bis(difluormethyl)pyrazol Derivate der Formel (I) besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp.*, *Juglandaceae sp.*, *Betulaceae sp.*, *Anacardiaceae sp.*, *Fagaceae sp.*, *Moraceae sp.*, *Oleaceae sp.*, *Actinidaceae sp.*, *Lauraceae sp.*, *Musaceae sp.* (beispielsweise Bananenbäume und - plantagen), *Rubiaceae sp.* (beispielsweise Kaffee), *Theaceae sp.*, *Sterculiceae sp.*, *Rutaceae sp.* (beispielsweise Zitronen, Organen und Grapefruit); *Solanaceae sp.* (beispielsweise Tomaten), *Liliaceae sp.*, *Asteraceae sp.* (beispielsweise Salat), *Umbelliferae sp.*, *Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp*. (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Bis(difluormethyl)pyrazol Derivate. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Bis(difluormethyl)pyrazol Derivate auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Erfindung umfasst weiterhin ein Verfahren zur Behandlung von Saatgut.

Ein weiterer Aspekt der vorliegenden Erfindung betrifft insbesondere ein Saatgut, welches mit wenigstens einem der erfindungsgemäßen Bis(difluormethyl)pyrazol Derivate behandelt ist. Die erfindungsgemäßen Saatgüter finden Anwendung in Verfahren zum Schutz von Saatgut vor pflanzenpathogenen Schadpilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe der Formel (I) können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylhamstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylhamstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil CrickmoreBt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_CrickmoreBt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (s i e he zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel **(I)** bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln **(I)** geht aus den folgenden Beispielen hervor. Die Erfindung ist jedoch nicht auf diese Beispiele beschränkt.

**Allgemeines:** Wenn nicht anders angegeben, wurden alle chromatografischen Reinigungs- bzw. Trennungsschritte an Kieselgel und mit einem Lösungsmittelgradienten von 0:100 Essigsäure-ethylester/Cyclohexan zu 100:0 Essigsäure-ethylester/Hexan durchgeführt.

### Herstellung von Verbindungen der Formel (I)

### 2-[3,5-Bis(difluormethyl)-1H-pyrazol-1-yl]-1-{4-fluor-4-[4-(5-phenyl-4,5-dihydro-1,2-oxazol-3-yl)1,3-thiazol-2-yl]piperidin-1-yl}ethanon (I-5)

### Verfahren A

### tert-Butyl-4-(4-brom-1,3-thiazol-2-yl)-4-hydroxypiperidin-1-carboxylat (IV-1)

Zu einer Lösung von 2,4-Dibrom-1,3-thiazol (8.8 g) in Dichloromethan (180 mL) wurde bei -78 °C unter Argon nButyllithium (1.6M in Tetrahydrofuran, 25 mL) getropft. Das Reaktionsgemisch wurde 20 Minuten bei -78 °C gerührt und dann wurde *tert*-Butyl-4-oxopiperidin-1-carboxylat zugegeben. Das Gemisch wurde bei Raumtemperatur für 30 Minuten gerührt. Das Reaktionsgemisch wurde anschließend mit gesättigter Ammoniumchloridlösung bei -30°C versetzt und die wässrige Phase abgetrennt. Nach Extraktion der wässrigen Phase mit Dichloromethan wurden die vereinigten organischen Phasen über Natriumsulfat getrocknet und unter vermindertem Druck eingeengt. Der Rückstand wurde chromatographisch gereinigt. Man erhielt *tert*-Butyl-4-(4-brom-1,3-thiazol-2-yl)-4-hydroxypiperidin-1-carboxylat (15.3 g).

¹H NMR (DMSO-d₆): δₚₚₘ : 1.43 (s, 9H), 1.70 (d, 2H), 1.88 (ddd, 2H), 3.11 (bs, 2H), 3.83 (d, 2H), 6.31 (s, 1H), 7.72 (s, 1H)

logP (HCOOH): 2.74

MS (ESI): 363 und 365 ([M+H]⁺)

### Verfahren B

### tert-Butyl-4-(4-brom-1,3-thiazol-2-yl)-4-fluorpiperidin-1-carboxylat (V-1)

*tert*-Butyl-4-(4-brom-1,3-thiazol-2-yl)-4-hydroxypiperidin-1-carboxylat (17.7 g) wurde unter Argon bei 0 °C in Dichloromethan in einem PE-Kolben vorgelegt und Diethylaminoschwefeltrifluorid (DAST) (7.08 mL) wurden zugetropft. Die Kühlung wurde entfernt. Nach Rühren über Nacht wurde mit gesättigter wässriger Natriumhydrogencarbonatlösung versetzt und mit Dichlromethan extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-(4-brom-1,3-thiazol-2-yl)-4-fluorpiperidin-1-carboxylat (18.0 g).

¹H NMR (DMSO-d₆): δₚₚₘ : 1.42 (s, 9H), 2.13-2.00 (m, 4H), 3.14 (bs, 2H), 3.95-3.87 (m, 2H), 7.95 (s, 1 H)

logP (HCOOH): 3.94

MS (ESI): 309 und 311 ([M-C(CH₃)₃+2H]⁺)

### Verfahren C

### tert-Butyl-4-fluor-4-(4-formyl-1,3-thiazol-2-yl)piperidin-1-carboxylat (VI-1)

Zu einer Lösung von *tert*-Butyl-4-(4-brom-1,3-thiazol-2-yl)-4-fluorpiperidin-1-carboxylat (245 mg) in Dichloromethan (5 mL) wurde bei -78 °C nButyllithium (1.6 M in Tetrahydrofuran, 0.42 mL) getropft. Nach 20 min wurde N,N-Dimethylformamid (0.16 mL) zugetropft. Nach Rühren für 30 Minuten bei -78 °C wurde mit gesättigter Ammoniumchloridlösung versetzt und mit Dichloromethan extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-fluor-4-(4-formyl-1,3-thiazol-2-yl)piperidin-1-carboxylat (75 mg).

¹H NMR (DMSO-d₆): δₚₚₘ : 1.43 (s, 9H), 2.18-2.04 (m, 4H), 3.17 (bs, 2H), 3.97-3.89 (m, 2H), 8.80 (s, 1 H), 9.92 (s, 1 H)

logP (HCOOH): 2.80

MS (ESI): 259 ([M-C(CH₃)₃+2H]⁺)

### Verfahren D und E

### tert-Butyl-4-fluor-4-{4-[(E/Z)-(hydroxyimino)methyl]-1,3-thiazol-2-yl}piperidin-1-carboxylat

Zu einer Lösung von *tert*-Butyl-4-fluor-4-(4-formyl-1,3-thiazol-2-yl)piperidin-1-carboxylat (3.49 g) in Ethanol (50 mL) wurde Hydroxylamin (50% in Wasser, 0.81 mL) bei Raumtemperatur getropft. Die Reaktionsmischung wurde bei 60 °C für 1 Stunde gerührt, dann wurde das Lösungsmittel unter vermindertem Druck entfernt. Man erhielt *tert*-Butyl-4-fluor-4-{4-[(E/Z)-(hydroxyimino)methyl]-1,3-thiazol-2-yl}piperidin-1-carboxylat (3.49 g).

¹H NMR (DMSO-d₆): δₚₚₘ : 1.42 (s, 9H), 2.17-2.03 (m, 4H), 3.16 (bs, 2H), 3.94-3.86 (m, 2H), 7.96 (s, 1H), 8.17 (s, 1H)

logP (HCOOH): 2.53

MS (ESI): 230 ([M-COOC(CH₃)₃+2H]⁺)

### tert-Butyl-4-fluor-4-[4-(5-phenyl-4,5-dihydro-1,2-oxazol-3-yl)-1,3-thiazol-2-yl]piperidin-1-carboxylat (X-a-1)

Zu einer Lösung von *tert*-Butyl-4-fluor-4-{4-[(E/Z)-(hydroxyimino)methyl]-1,3-thiazol-2-yl}piperidin-1-carboxylat (500 mg) in Tetrahydrofuran (5 mL) wurde bei Raumtemperatur Styrol (0.21 mL) getropft und anschließend Chlorlauge (13% in Wasser). Nach Rühren für 4 Stunden bei Raumtemperatur wurde das Lösungsmittel unter vermindertem Druck entfernt. Der Rückstand wurde mit Dichloromethan und Wasser versetzt und mit Dichloromethan extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-fluor-4-[4-(5-phenyl-4,5-dihydro-1,2-oxazol-3-yl)-1,3-thiazol-2-yl]piperidin-1-carboxylat (380 mg).

¹H NMR (DMSO-d₆): δₚₚₘ : 1.42 (s, 9H), 2.14-2.07 (m, 4H), 3.15 (bs, 2H), 3.38 (dd, 1H), 3.89 (dd, 1H), 3.93 (bs, 2H), 5.75 (dd, 1H), 7.34 (m, 1H), 7.42-7.39 (m, 4H), 8.21 (s, 1H)

logP (HCOOH): 4.28

MS (ESI): 332 ([M-COOC(CH₃)₃+2H]⁺)

### Verfahren G

### 4-Fluor-4-[4-(5-phenyl-4,5-dihydro-1,2-oxazol-3-yl)-1,3-thiazol-2-yl]piperidiniumchlorid (XII-1)

Zu einer Lösung von tert-Butyl-4-fluor-4-[4-(5-phenyl-4,5-dihydro-1,2-oxazol-3-yl)-1,3-thiazol-2-yl]piperidin-1-carboxylat (380 mg) wurde bei 0 °C eine 4-molare Lösung von Salzsäure in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-Fluor-4-[4-(5-phenyl-4,5-dihydro-1,2-oxazol-3-yl)-1,3-thiazol-2-yl]piperidiniumchlorid (374 mg).

¹H NMR (DMSO-d₆): δₚₚₘ: 2.58-2.30 (m, 4H), 3.23-3.14 (m, 2H), 3.38 (dd, 1H), 3.56 (s, 2H), 3.90 (dd, 1H), 5.76 (dd, 1H), 7.42-7.33 (m, 5H), 8.25 (s, 1H), 9.08 (bs, 1H), 9.24 (bs, 1H)

logP (HCOOH): 1.17

MS (ESI): 332 ([M-Cl]⁺)

### Verfahren H

### 2-[3,5-Bis(difluormethyl)-1H-pyrazol-1-yl]-1-{4-fluor-4-[4-(5-phenyl-4,5-dihydro-1,2-oxazol-3-yl)-1,3-thiazol-2-yl]piperidin-1-yl}ethanon (I-5)

Zu einer Lösung von [Bis-3,5-(difluormethyl)-1H-pyrazol-1-yl]essigsäure (230 mg) in Dichlormethan (5 mL) wurden Oxalylchlorid (387 mg) und ein Tropfen N,N-Dimethylformamid gegeben. Das Reaktionsgemisch wurde über Nacht bei Raumtemperatur gerührt. Nach Entfernen der Lösungsmittel unter vermindertem Druck wurde der Rückstand dann in Dichlormethan (5 mL) gelöst und bei 0 °C tropfenweise mit **einer Lösung von** 4-Fluor-4-[4-(5-phenyl-4,5-dihydro-1,2-oxazol-3-yl)-1,3-thiazol-2-yl]piperidiniumchlorid (374 mg) und von Hünigbase (394 mg) in Dichlormethan (5 mL) versetzt. Die Reaktionsmischung wurde bei Raumtemperatur für 3 Stunden gerührt. Nach Zugabe von konz. Ammoniumchloridlösung wurde die wässrige Phase abgetrennt und mit Essigsäureethylester extrahiert. Die vereinigten organischen Phasen wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1- {4-fluor-4-[4-(5-phenyl-4,5-dihydro-1,2-oxazol-3-yl)-1,3-thiazol-2-yl]piperidin-1-yl}ethanon (250 mg).

¹H NMR (DMSO-d₆): δₚₚₘ : 2.45-2.05 (m, 4H), 3.11 (m, 1H), 3.38 (dd, 1H), 3.48 (m, 1H), 3.95-3.86 (m, 2H), 4.26 (m, 1H), 5.39 (d, 1H), 5.50 (d, 1H), 5.76 (dd, 1H), 6.91 (s, 1H), 7.03 (t, 1H), 7.18 (t, 1H), 7.42-7.33 (m, 5H), 8.22 (s, 1H)

logP (HCOOH): 3.41

MS (ESI): 540 ([M+H]⁺)

### 1-(4-{4-[5-(2-acetylphenyl)-4,5-dihydro-1,2-oxazol-3-yl]-1,3-thiazol-2-yl}piperidin-1-yl)-2-[3,5-bis(difluoromethyl)-1H-pyrazol-1-yl]ethanone (I-7)

### Verfahren D und E

### tert-Butyl-4-{4-[5-(2-acetylphenyl)-4,5-dihydro-1,2-oxazol-3-yl]-1,3-thiazol-2-yl}piperidin-1-carboxylat (X-a-2)

Zu einer Lösung von *tert*-Butyl-4-{4-[(Z/E)-(hydroxyimino)methyl]-1,3-thiazol-2-yl}piperidin-1-carboxylat (4.5 g) und 1-(2-Vinylphenyl)ethanon (2.32 g) in Ethylacetat (70 mL) wurde bei Raumtemperatur Kaliumhydrogencarbonat (7.23 g) und N-Chlorsuccinimid (2.31 g) gegeben und anschließend drei Tropfen Wasser. Die Reaktionsmischung wurde für 3 Stunden bei 60 °C gerührt, dann mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(2-acetylphenyl)-4,5-dihydro-1,2-oxazol-3-yl]-1,3-thiazol-2-yl}piperidin-1-carboxylat (4.64 g).

¹H NMR (DMSO-d₆): δₚₚₘ : 1.41 (s, 9H), 1.60-1.48 (m, 2H), 2.04-1.98 (m, 2H), 2.63 (s, 3H), 2.89 (bs, 2H), 3.13 (dd, 1H), 3.27-3.18 (m, 1H), 4.07-3.95 (m, 3H), 6.12 (dd, 1H), 7.48 (dd, 1H), 7.56 (d, 1H), 7.61 (dd, 1H), 7.98 (s, 1H), 8.00 (d, 1H)

### Verfahren G

### 1-(2-{3-[2-(Piperidin-4-yl)-1,3-thiazol-4-yl]-4,5-dihydro-1,2-oxazol-5-yl}phenyl)ethanonhydrochlorid (XII-2)

Zu einer Lösung von *tert*-Butyl-4-{4-[5-(2-acetylphenyl)-4,5-dihydro-1,2-oxazol-3-yl]-1,3-thiazol-2-yl}piperidin-1-carboxylat (500 mg) in 1,4-Dioxan (5 mL) wurde bei 0 °C eine 4-molare Lösung von Chlorwasserstoff in 1,4-Dioxan (4.2 mL) getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 1-(2-{3-[2-(Piperidin-4-yl)-1,3-thiazol-4-yl]-4,5-dihydro-1,2-oxazol-5-yl}phenyl)ethanonhydrochlorid (430 mg).

¹H NMR (DMSO-d₆): δₚₚₘ : 1.99-1.85 (m, 2H), 2.23-2.15 (m, 2H), 2.63 (s, 3H), 3.08-2.97 (m, 2H), 3.14 (dd, 1H), 3.43-3.30 (m, 3H), 4.00 (dd, 1H), 6.12 (dd, 1H), 7.98 (dd, 1H), 7.56 (d, 1H), 7.62 (dd, 1H), 8.00 (d, 1H), 8.03 (s, 1H), 8.75 (bs, 1H), 9.03 (bs, 1H)

logP (HCOOH): 0.96

MS (ESI): 358 ([M-Cl]⁺)

### Verfahren H

### 1-(4-{4-[5-(2-acetylphenyl)-4,5-dihydro-1,2-oxazol-3-yl]-1,3-thiazol-2-yl}piperidin-1-yl)-2-[3,5-bis(difluoromethyl)-1H-pyrazol-1-yl]ethanone (I-7)

Lösung A: Zu einer Lösung von [3,5-Bis(difluormethyl)-1H-pyrazol-1-yl]essigsäure (280 mg) in Dichlormethan (10 ml) wurde bei Raumtemperatur ein Tropfen N,N-Dimethylformamid und Oxalylchlorid (0.295 mL) getropft. Nach 2-stündigem Rühren bei Raumtemperatur wurde das Lösungsmittel entfernt und der Rückstand wurde wieder in Dichlormethan (10 ml) gelöst (Lösung A).

Zu einer Lösung von 1-(2-{3-[2-(Piperidin-4-yl)-1,3-thiazol-4-yl]-4,5-dihydro-1,2-oxazol-5-yl}phenyl)ethanonhydrochlorid (441 mg) in Dichloromethan (5 mL) wurde bei Raumtemperatur Diisopropylethylamin (588 ml) zugesetzt. Nach 15 Minuten wurde die Lösung A zugetropft. Nach Rühren über Nacht bei Raumtemperatur wurde die Reaktionsmischung mit Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt 1-(4-{4-[5-(2-acetylphenyl)-4,5-dihydro-1,2-oxazol-3-yl]-1,3-thiazol-2-yl}piperidin-1-yl)-2-[3,5-bis(difluoromethyl)-1H-pyrazol-1-yl]ethanone (590 mg).

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzaldehyd (I-26)

### Verfahren D und E

### tert-Butyl-4-{4-[5-(2-formylphenyl)-4,5-dihydro-1,2-oxazol-3-yl]-1,3-thiazol-2-yl}piperidin-1-carboxylat (X-a-3)

*tert*-Butyl-4-{4-[(Z/E)-(hydroxyimino)methyl]-1,3-thiazol-2-yl}piperidin-1-carboxylat (5.6 g) und 2-Vinylbenzaldehyd (2.61 g) wurden analog zu I-7 (Verfahren D und E) umgesetzt. Man erhielt tert-Butyl-4-{4-[5-(2-formylphenyl)-4,5-dihydro-1,2-oxazol-3-yl]-1,3-thiazol-2-yl}piperidin-1-carboxylat (5.69 g).

¹H NMR (DMSO-d₆): δₚₚₘ : 1.41 (s, 9H), 1.60-1.48 (m, 2H), 2.04-1.98 (m, 2H), 2.89 (bs, 2H), 3.28-3.15 (m, 2H), 4.07-3.95 (m, 3H), 6.42 (dd, 1H), 7.64-7.57 (m, 2H), 7.72 (dd, 1H), 7.99 (s, 1H), 8.03 (d, 1H), 10.18 (s, 1H)

logP (HCOOH): 3.76

MS (ESI): 342 ([M-COOC(CH₃)₃+2H]⁺)

### Verfahren G

### 2-{3-[2-(Piperidin-4-yl)-1,3-thiazol-4-yl]-4,5-dihydro-1,2-oxazol-5-yl}benzaldehydhydrochlorid (XII-3)

*tert*-Butyl-4-{4-[5-(2-formylphenyl)-4,5-dihydro-1,2-oxazol-3-yl]-1,3-thiazol-2-yl}piperidin-1-carboxylat (5.1 g) wurde analog zu I-7 (Verfahren G) umgesetzt. Man erhielt 2-{3-[2-(Piperidin-4-yl)-1,3-thiazol-4-yl]-4,5-dihydro-1,2-oxazol-5-yl}benzaldehydhydrochlorid (4.35 g).

¹H NMR (DMSO-d₆): δₚₚₘ : 2.02-1.89 (m, 2H), 2.23-2.15 (m, 2H), 3.08-2.97 (m, 2H), 3.18 (dd, 1H), 3.35-3.29 (m, 2H), 3.41-3.35 (m, 1H), 4.05 (dd, 1H), 6.42 (dd, 1H), 7.64-7.57 (m, 2H), 7.72 (dd, 1H), 8.02 (d, 1H), 8.03 (s, 1H), 10.19 (s, 1H)

logP (HCOOH): 0.76

MS (ESI): 342 ([M-Cl]⁺)

### Verfahren H

### 2-{3-[2-(1-{[3,5-Bis(difluormethyl)-1H-pyrazol-1-yl]acetyl}piperidin4-yl)-1,3-thiazol-4-yl]-4,5-dihydro-1,2-oxazol-5-yl}benzaldehyd (I-26)

2-{3-[2-(Piperidin-4-yl)-1,3-thiazol-4-yl]-4,5-dihydro-1,2-oxazol-5-yl}benzaldehydhydrochlorid (432 mg) wurde analog zu I-7 (Verfahren G) umgesetzt. Man erhielt 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzaldehyd (330 mg).

### 2-[3,5-Bis(difluormethyl)-1H-pyrazol-1-yl]-1-(4-{4-[5-(2-ethinylphenyl)-4,5-dihydro-1,2-oxazol-3-yl]-1,3-thiazol-2-yl}piperidin-1-yl)ethanon (I-38)

Zu einer Lösung von 4-Acetamidobenzolsulfonylazid (140 mg) in Acetonitril (10 mL) wurde bei Raumtemperatur Dimethyl-2-oxopropylphosphonate (97 mg) gegeben. Nach Rühren über 2 Stunden wurde 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzaldehyd (267 mg) in Methanol (2 mL) zur Reaktionsmischung gegeben. Nach Rühren für 8 Stunden wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert-*2-[3,5-Bis(difluormethyl)-1H-pyrazol-1-yl]-1-(4-{4-[5-(2-ethinylphenyl)-4,5-dihydro-1,2-oxazol-3-yl]-1,3-thiazol-2-yl}piperidin-1-yl)ethanon (50 mg).

### N-(2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl)methansulfonamid (I-37)

### Verfahren D und E

### tert-Butyl-4-[4-(5-{2-[(methylsulfonyl)amino]phenyl}-4,5-dihydro-1,2-oxazol-3-yl)-1,3-thiazol-2-yl]piperidin-1-carboxylat (X-a-4)

*tert*-Butyl-4-{4-[(Z/E)-(hydroxyimino)methyl]-1,3-thiazol-2-yl}piperidin-1-carboxylat (1.35 g) und N-(2-Vinylphenyl)methansulfonamid (1.11 g) wurden analog zu I-7 (Verfahren D und E) umgesetzt. Man erhielt *tert*-Butyl-4-[4-(5-{2-[(methylsulfonyl)amino]phenyl}-4,5-dihydro-1,2-oxazol-3-yl)-1,3-thiazol-2-yl]piperidin-1-carboxylat (0.94 g).

¹H NMR (DMSO-d₆): δₚₚₘ : 1.41 (s, 9H), 1.51-1.49 (m, 2H), 2.08-2.00 (m, 2H), 2.95-2.84 (m, 2H), 3.03 (s, 3H), 3.38-3.21 (m, 2H), 3.90 (dd, 1H), 4.05-3.96 (m, 2H), 6.09 (dd, 1H), 7.43-7.30 (m, 4H), 8.00 (s, 1H), 9.18 (bs, 1H)

logP (HCOOH): 3.00

MS (ESI): 407 ([M-COOC(CH₃)₃+2H]⁺)

### Verfahren G

### 4-[4-(5-{2-[(Methylsulfonyl)amino]phenyl}-4,5-dihydro-1,2-oxazol-3-yl)-1,3-thiazol-2-yl]piperidiniumchlorid (XII-4)

*tert*-Butyl-4-[4-(5-{2-[(methylsulfonyl)amino]phenyl}-4,5-dihydro-1,2-oxazol-3-yl)-1,3-thiazol-2-yl]piperidin-1-carboxylat (900 mg) wurde analog zu I-7 (Verfahren G) umgesetzt. Man erhielt 4-[4-(5-{2-[(Methylsulfonyl)amino]phenyl}-4,5-dihydro-1,2-oxazol-3-yl)-1,3-thiazol-2-yl]piperidiniumchlorid (1.00 g).

logP (HCOOH): 0.72

MS (ESI): 407 ([M-Cl+2H]⁺)

### Verfahren H

### N-(2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl)methansulfonamid (I-37)

4-[4-(5-{2-[(Methylsulfonyl)amino]phenyl}-4,5-dihydro-1,2-oxazol-3-yl)-1,3-thiazol-2-yl]piperidiniumchlorid (299 mg) wurde analog zu I-7 (Verfahren G) umgesetzt. Man erhielt N-(2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl)methansulfonamid (215 mg).

### 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (I-9)

### Verfahren D und E

### tert-Butyl-4-{4-(5-(2-hydroxyphenyl)-4,5-dihydro-1,2-oxazol-3-yl]-1,3-thiazol-2-yl}piperidin-1-carboxylat (X-a-5)

Zu einer Lösung von *tert*-Butyl-4-{4-[(hydroxyimino)methyl]-1,3-thiazol-2-yl}piperidin-1-carboxylat (3.46 g) und 2-Vinylphenol (1.60 g) in Ethylacetat (50 mL) wurde bei Raumtemperatur Kaliumhydrogencarbonat (5.55 g) und N-Chlorsuccinimid (1.78 g) gegeben und anschließend ein Tropfen Wasser. Nach Rühren über Nacht bei 60 °C wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(2-hydroxyphenyl)-4,5-dihydro-1,2-oxazol-3-yl]-1,3-thiazol-2-yl}piperidin-1-carboxylat (1.70 g).

¹H NMR (DMSO-d₆, 400 MHz): δₚₚₘ : 1.41 (s, 9H), 1.49-1.61 (m, 2H), 1.98-2.06 (m, 2H), 2.82-2.96 (m, 2H), 3.80 (dd, 1H), 3.96-4.05 (m, 2H), 5.82 (dd, 1H), 6.79 (t, 1H), 6.85 (d, 1H), 7.13 (t, 1H), 7.20 (d, 1H), 7.98 (s, 1H), 9.70 (s, 1H)

logP (pH2.7): 3.22

MS (ESI): 330 ([M+H-C₄H₉OCO]⁺)

### Verfahren G

### 4-{4-[5-(2-Hydroxyphenyl)-4,5-dihydro-1,2-oxazol-3-yl]-1,3-thiazol-2-yl}piperidiniumchlorid (XII-5)

Zu einer Lösung *tert*-Butyl-4-{4-[5-(2-hydroxyphenyl)-4,5-dihydro-1,2-oxazol-3-yl]-1,3-thiazol-2-yl}piperidin-1-carboxylat (1.70 g) in Dichlormethan wurde bei 0 °C eine 4-molare Lösung von Chlorwasserstoff (4.0 Äq.) in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-{4-[5-(2-Hydroxyphenyl)-4,5-dihydro-1,2-oxazol-3-yl]-1,3-thiazol-2-yl}piperidiniumchlorid (1.45 g).

¹H NMR (DMSO-d₆, 400 MHz): δₚₚₘ : 1.85-1.98 (m, 2H), 2.15-2.23 (m, 2H), 2.98-3.09 (m, 2H), 3.26 (dd, 1H), 3.81 (dd, 1H), 5.83 (dd, 1H), 6.79 (t, 1H), 6.86 (d, 1H), 7.13 (t, 1H), 7.20 (d, 1H), 8.02 (s, 1H), 8.58 (bs, 1H), 8.87 (bs, 1H), 9.74 (s, 1H)

logP (pH2.7): 0.69

MS (ESI): 330 ([M+H]⁺)

### Verfahren H

### 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (I-9)

Zu einer Lösung von [3,5-Bis-(difluormethyl)-1H-pyrazol-1-yl]essigsäure (982 mg) in Dichlormethan (10 mL) werden bei 0 °C Oxalylchlorid (1.50 g) und ein Tropfen N,N-Dimethylformamid gegeben. Das Reaktionsgemisch wird bei Raumtemperatur für 60 Minuten gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-{4-[5-(2-Hydroxyphenyl)-4,5-dihydro-1,2-oxazol-3-yl]-1,3-thiazol-2-yl}piperidiniumchlorid (1.45 g) und Triethylamin (5.5 mL) in Dichlormethan (14 mL) gegeben. Die Reaktionsmischung wird für 3 h bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (900 mg).

### 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon (I-21)

Zu einer Lösung von 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (60 mg) und Kaliumcarbonat (23 mg) in DMF (3 mL) werden bei Raumtemperatur Kaliumiodid (10 mg) und 3-Bromprop-1-in (21 mg) gegeben. Das Reaktionsgemisch wird für 9 h bei 80 °C gerührt. Daraufhin wird das Gemisch mit verdünnter Salzsäure versetzt und mit Essigester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon (40 mg).

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylcyclohexancarboxylat (I-32)

Zu einer Lösung von 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (150 mg) und Triethylamin (33 mg) in Methylenchlorid (10 mL) wird bei 0 °C ein Lösung aus Cyclohexancarbonylchlorid in Methylenchlorid (2 mL) gegeben. Das Eisbad wird entfernt und das Reaktionsgemisch wird für 3 h bei 0 °C - RT gerührt. Daraufhin wird das Gemisch mit verdünnter Natriumhydrogencarbonat-Lösung versetzt und mit Methylenchlorid extrahiert. Die vereinigten organischen Phasen werden über Natriumsulfat getrocknet und eingeengt. Nach säulenchromatographischer Reinigung erhält man 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylcyclohexancarboxylat (150 mg).

### 2-[3,5-Bis(difluormethyl)-1H-pyrazol-1-yl]-1-(4-{4-[5-(2-nitrophenyl)-4,5-dihydro-1,2-oxazol-3-yl]-1,3-thiazol-2-yl}piperidin-1-yl)ethanon (I-10)

### Verfahren D und E

### tert-Butyl-4-{4-[5-(2-nitrophenyl)-4,5-dihydro-1,2-oxazol-3-yl]-1,3-thiazol-2-yl}piperidin-1-carboxylat (X-a-6)

Zu einer Lösung von *tert*-Butyl-4-{4-[(hydroxyimino)methyl]-1,3-thiazol-2-yl}piperidin-1-carboxylat (2.80 g) und 1-Nitro-2-vinylbenzol (1.60 g) in Ethylacetat (50 mL) wurde bei Raumtemperatur Kaliumhydrogencarbonat (4.50 g) und N-Chlorsuccinimid (1.44 g) gegeben und anschließend ein Tropfen Wasser. Nach Rühren über Nacht bei 60 °C wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt tert-Butyl-4-{4-[5-(2-nitrophenyl)-4,5-dihydro-1,2-oxazol-3-yl]-1,3-thiazol-2-yl}piperidin-1-carboxylat (2.10 g).

¹H NMR (DMSO-d₆, 400 MHz): δₚₚₘ : 1.40 (s, 9H), 1.48-1.60 (m, 2H), 1.98-2.06 (m, 2H), 2.81-2.96 (m, 2H), 3.40 (dd, 1H), 3.96-4.04 (m, 2H), 4.09 (dd, 1H), 6.24 (dd, 1H), 7.64 (t, 1H), 7.66 (d, 1H), 7.79 (t, 1H), 8.02 (s, 1H), 8.15 (d, 1H)

logP (pH2.7): 4.01

MS (ESI): 359 ([M+H-C₄H₉OCO]⁺)

### Verfahren G

### 4-{4-[5-(2-Nitrophenyl)-4,5-dihydro-1,2-oxazol-3-yl]-1,3-thiazol-2-yl}piperidiniumchlorid (XII-6)

Zu einer Lösung von *ter*t-Butyl-4-{4-[5-(2-nitrophenyl)-4,5-dihydro-1,2-oxazol-3-yl]-1,3-thiazol-2-yl}piperidin-1-carboxylat (2.10 g) in Dichlormethan wurde bei 0 °C eine 4-molare Lösung von Chlorwasserstoff (4.0 Äq.) in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-{4-[5-(2-Nitrophenyl)-4,5-dihydro-1,2-oxazol-3-yl]-1,3-thiazol-2-yl}piperidiniumchlorid (1.60 g).

¹H NMR (DMSO-d₆, 400 MHz): δₚₚₘ : 1.86-1.97 (m, 2H), 2.15-2.23 (m, 2H), 2.98-3.09 (m, 2H), 4.10 (dd, 1H), 6.24 (dd, 1H), 7.62 (t, 1H), 7.67 (d, 1H), 7.81 (t, 1H), 8.07 (s, 1H), 8.16 (d, 1H), 8.63 (bs, 1H), 8.91 (bs, 1H)

logP (pH2.7): 1.09

MS (ESI): 359 ([M+H]⁺)

### Verfahren H

### 2-[3,5-Bis(difluormethyl)-1H-pyrazol-1-yl]-1-(4-{4-[5-(2-nitrophenyl)-4,5-dihydro-1,2-oxazol-3-yl]-1,3-thiazol-2-yl}piperidin-1-yl)ethanon (I-10)

Zu einer Lösung von [3,5-Bis-(difluormethyl)-1H-pyrazol-1-yl]essigsäure (0.37 g) in Dichlormethan (10 mL) werden bei 0 °C Oxalylchlorid (0.57 g) und ein Tropfen N,N-Dimethylformamid gegeben. Das Reaktionsgemisch wird bei Raumtemperatur für 60 Minuten gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-{4-[5-(2-Nitrophenyl)-4,5-dihydro-1,2-oxazol-3-yl]-1,3-thiazol-2-yl}piperidiniumchlorid (0.59 g) und Triethylamin (2.1 mL) in Dichlormethan (14 mL) gegeben. Die Reaktionsmischung wird für 20 h bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-(4-{4-[5-(2-nitrophenyl)-4,5-dihydro-1,2-oxazol-3-yl]-1,3-thiazol-2-yl}piperidin-1-yl)ethanon (170 mg).

### 1-(4-{4-[5-(2-Aminophenyl)-4,5-dihydro-1,2-oxazol-3-yl]-1,3-thiazol-2-yl}piperidin-1-yl)-2-[3,5-bis(difluormethyl)-1H-pyrazol-1-yl]ethanon (1-41)

Zu einer Lösung von 2-[3,5-Bis(difluormethyl)-1H-pyrazol-1-yl]-1-(4-{4-[5-(2-nitrophenyl)-4,5-dihydro-1,2-oxazol-3-yl]-1,3-thiazol-2-yl}piperidin-1-yl)ethanon (80 mg) in Methanol (10 mL) wird bei Raumtemperatur Pd/C (20 mg, 10%ig) gegeben. Das Reaktionsgemisch wird 3 Stunden bei Raumtemperatur unter Wasserstoffatmosphäre gerührt, anschließend werden flüchtige Bestandteile unter vermindertem Druck entfernt. Nach säulenchromatographischer Reinigung erhält man 1-(4-{4-[5-(2-Aminophenyl)-4,5-dihydro-1,2-oxazol-3-yl]-1,3-thiazol-2-yl}piperidin-1-yl)-2-[3,5-bis(difluormethyl)-1H-pyrazol-1-yl]ethanon (25 mg).

### 2-[3,5-Bis(difluormethyl)-1H-pyrazol-1-yl]-1-[4-(4-{5-[2-(cyclohexylmethoxy)phenyl]-4,5-dihydro-1,2-oxazol-3-yl}-1,3-thiazol-2-yl)piperidin-1-yl]ethanon (I-24)

### Verfahren D und E

### tert-Butyl-4-(4-{5-[2-(cyclohexylmethoxy)phenyl]-4,5-dihydro-1,2-oxazol-3-yl}-1,3-thiazol-2-yl)piperidin-1-carboxylat (X-a-7)

Zu einer Lösung von *tert*-Butyl-4-{4-[(hydroxyimino)methyl]-1,3-thiazol-2-yl}piperidin-1-carboxylat (2.90 g) und 1-(Cyclohexylmethoxy)-2-vinylbenzol (2.40 g) in Ethylacetat (300 mL) wurde bei Raumtemperatur Kaliumhydrogencarbonat (4.60 g) und N-Chlorsuccinimid (1.48 g) gegeben und anschließend ein Tropfen Wasser. Die Reaktionsmischung wurde für 6 h bei 60 °C gerührt, anschließend wurde mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt tert-Butyl-4-(4-{5-[2-(cyclohexylmethoxy)phenyl]-4,5-dihydro-1,2-oxazol-3-yl}-1,3-thiazol-2-yl)piperidin-1-carboxylat (3.40 g).

¹H NMR (DMSO-d₆, 400 MHz): δₚₚₘ : 0.97-1.12 (m, 5H), 1.40 (s, 9H), 1.50-1.80 (m, 8H), 1.98-2.06 (m, 2H), 2.81-2.96 (m, 2H), 3.74-3.81 (m, 3H), 3.95-4.03 (m, 2H), 5.78 (dd, 1H), 6.92 (t, 1H), 7.00 (d, 1H), 7.26-7.33 (m, 2H), 7.97 (s, 1H)

logP (pH2.7): 6.30

MS (ESI): 426 ([M+H-C₄H₉OCO]⁺)

### Verfahren G

### 4-(4-{5-[2-(Cyclohexylmethoxy)phenyl]-4,5-dihydro-1,2-oxazol-3-yl}-1,3-thiazol-2-yl)piperidiniumchlorid (XII-7)

Zu einer Lösung von tert-Butyl-4-(4-{5-[2-(cyclohexylmethoxy)phenyl]-4,5-dihydro-1,2-oxazol-3-yl}-1,3-thiazol-2-yl)piperidin-1-carboxylat (3.20 g) in Dichlormethan wurde bei 0 °C eine 4-molare Lösung von Chlorwasserstoff (4.0 Äq.) in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-(4-{5-[2-(Cyclohexylmethoxy)phenyl]-4,5-dihydro-1,2-oxazol-3-yl}-1,3-thiazol-2-yl)piperidiniumchlorid (2.50 g).

¹H NMR (DMSO-d₆, 400 MHz): δₚₚₘ : 0.92-1.14 (m, 5H), 1.51-1.81 (m, 6H), 1.93-2.07 (m, 2H), 2.14-2.27 (m, 2H), 2.92-3.10 (m, 2H), 3.26-3.47 (m, 4H), 3.71-3.86 (m, 3H), 5.80 (dd, 1H), 6.91 (t, 1H), 7.01 (d, 1H), 7.25-7.34 (m, 2H), 8.01 (s, 1H), 9.30 (s, 1H), 9.52 (s, 1H)

logP (pH2.7): 1.90

MS (ESI): 426 ([M+H]⁺)

### Verfahren H

### 2-[3,5-Bis(difluormethyl)-1H-pyrazol-1-yl]-1-[4-(4-{5-[2-(cyclohexylmethoxy)phenyl]-4,5-dihydro-1,2-oxazol-3-yl}-1,3-thiazol-2-yl)piperidin-1-yl]ethanon (I-24)

Zu einer Lösung von [3,5-Bis-(difluormethyl)-1H-pyrazol-1-yl]essigsäure (1.49 g) in Dichlormethan (10 mL) werden bei 0 °C Oxalylchlorid (2.29 g) und ein Tropfen N,N-Dimethylformamid gegeben. Das Reaktionsgemisch wird bei Raumtemperatur für 60 Minuten gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-(4-{5-[2-(Cyclohexylmethoxy)phenyl]-4,5-dihydro-1,2-oxazol-3-yl}-1,3-thiazol-2-yl)piperidiniumchlorid (2.55 g) und Triethylamin (8.36 mL) in Dichlormethan (14 mL) gegeben. Die Reaktionsmischung wird für 20 h bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-[4-(4-{5-[2-(cyclohexylmethoxy)phenyl]-4,5-dihydro-1,2-oxazol-3-yl}-1,3-thiazol-2-yl)piperidin-1-yl]ethanon (1.87 g).

### 2-{3-[2-(1-{[3,5-Bis(difluormethyl)-1H-pyrazol-1-yl]acetyl}piperidin-4-yl)-1,3-thiazol-4-yl]-1,2-oxazol-5-yl}benzaldehyd (I-31)

### Verfahren D und E

### tert-Butyl-4-{4-[5-(2-formylphenyl)-1,2-oxazol-3-yl]-1,3-thiazol-2-yl}piperidin-1-carboxylat (X-b-1)

Zu einer Lösung von *tert*-Butyl-4-{4-[(hydroxyimino)methyl]-1,3-thiazol-2-yl}piperidin-1-carboxylat (1.00 g) in DMF (10 mL) wurde bei 50 °C N-Chlorsuccinimid (0.51 g) gegeben und für 30 Minuten gerührt. Zum Reaktionsgemisch wurde bei Raumtemperatur Triethylamin (1.34 mL) und 2-Ethinylbenzaldehyd (0.54 g) gegeben. Nach Rühren für 2 Stunden bei 50 °C wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(2-formylphenyl)-1,2-oxazol-3-yl]-1,3-thiazol-2-yllpiperidin-1-carboxylat (95 mg).

¹H NMR (DMSO-d₆): δₚₚₘ : 1.42-1.41 (m, 9H), 1.69-1.50 (m, 2H), 2.12-1.98 (m, 2H), 2.90 (bs, 2H), 3.40-3.28 (m, 1H), 4.10-3.95 (m, 2H), 7.44 (s, 1H), 7.77 (dd, 1H), 7.87 (dd, 1H), 7.94 (d, 1H), 8.02 (d, 1H), 8.29 (s, 1H), 10.29 (s, 1H)

logP (HCOOH): 4.10

MS (ESI): 340 ([M-COOC(CH₃)₃+2H]⁺)

### Verfahren G und H

### 2-{3-[2-(1-{[3,5-Bis(difluormethyl)-1H-pyrazol-1-yl]acetyl}piperidin-4-yl)-1,3-thiazol-4-yl]-1,2-oxazol-5-yl}benzaldehyd (I-31)

Zu einer Lösung *tert*-Butyl-4-{4-[5-(2-formylphenyl)-1,2-oxazol-3-yl]-1,3-thiazol-2-yl}piperidin-1-carboxylat (95 mg) in Dichlormethan wurde bei 0 °C eine 4-molare Lösung von Chlorwasserstoff (2 mL) in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-{4-[5-(2-Formylphenyl)-1,2-oxazol-3-yl]-1,3-thiazol-2-yl}piperidiniumchlorid (**XII-8**).

Zu einer Lösung von [3,5-Bis-(difluormethyl)-1H-pyrazol-1-yl]essigsäure (51 mg) in Dichlormethan (2 mL) werden bei 0 °C Oxalylchlorid (57 µL) und ein Tropfen N,N-Dimethylformamid gegeben. Das Reaktionsgemisch wird bei Raumtemperatur für 60 Minuten gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-{4-[5-(2-Formylphenyl)-1,2-oxazol-3-yl]-1,3-thiazol-2-yl}piperidiniumchlorid und Triethylamin (90 µL) in Dichlormethan (2 mL) gegeben. Die Reaktionsmischung wird für 3 h bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-{3-[2-(1-{[3,5-Bis(difluormethyl)-1H-pyrazol-1-yl]acetyl}piperidin-4-yl)-1,3-thiazol-4-yl]-1,2-oxazol-5-yl}benzaldehyd (36 mg).

### Cyclopropylmethyl-2-{3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}benzoat (I-13)

### Verfahren D und E

### tert-Butyl-4-(4-{5-[2-(methoxycarbonyl)phenyl]-4,5-dihydro-1,2-oxazol-3-yl}-1,3-thiazol-2-yl)piperidin-1-carboxylat (X-a-8)

*tert*-Butyl-{4-[(Z/E)-(hydroxyimino)methyl]-1,3-thiazol-2-yl}piperidin-1-carboxylat (600 mg) und Methyl-2-vinylbenzoat (406 mg) wurden analog zu I-31 (Verfahren D und E) umgesetzt. Man erhielt *tert-*Butyl-4-(4-{5-[2-(methoxycarbonyl)phenyl]-4,5-dihydro-1,2-oxazol-3-yl}-1,3-thiazol-2-yl)piperidin-1-carboxylat (643 mg).

¹H NMR (DMSO-d₆): δₚₚₘ : 1.40 (s, 9H), 1.60-1.49 (m, 2H), 2.05-1.96 (m, 2H), 2.98-2.75 (m, 2H), 3.20 (dd, 1H), 3.26-3.20 (m, 1H), 3.88 (s, 3H), 4.02 (dd, 1H), 4.05-3.93 (m, 2H), 6.30 (dd, 1H), 7.48 (dd, 1H), 7.55 (d, 1H), 7.65 (dd, 1H), 7.96 (d, 1H), 8.00 (s, 1H)

logP (HCOOH): 4.14

MS (ESI): 372 ([M-COOC(CH₃)₃+2H]⁺)

### 2-(3-{2-[1-(tert-Butoxycarbonyl)piperidin-4-yl]-1,3-thiazol-4-yl}-4,5-dihydro-1,2-oxazol-5-yl)benzoesäure (X-a-9)

Zu einer Lösung von *ter*t-Butyl-4-(4-{5-[2-(methoxycarbonyl)phenyl]-4,5-dihydro-1,2-oxazol-3-yl}-1,3-thiazol-2-yl)piperidin-1-carboxylat (643 mg) in Tetrahydrofuran (4 mL) und Wasser (0.8 mL) wird bei Raumtemperatur Lithiumhydroxidmonohydrat (86 mg) gegeben. Das Gemisch wird 2 h bei Raumtemperatur gerührt und dann mit eiskalter 1N HCl-Lösung versetzt. Die wässrige Phase wird mit Ethylacetat extrahiert und dann die vereinigten organischen Phasen mit Natriumsulfat getrocknet.

Der Feststoff wird abfiltriert und das Lösungsmittel abdestilliert. Man erhält 2-(3-{2-[1-(tert-Butoxycarbonyl)piperidin-4-yl]-1,3-thiazol-4-yl}-4,5-dihydro-1,2-oxazol-5-yl)benzoesäure (377 mg).

¹H NMR (DMSO-d₆): δₚₚₘ : 1.40 (s, 9H), 1.56-1.48 (m, 2H), 2.04-1.99 (m, 2H), 2.88 (bs, 2H), 3.18 (dd, 1H), 3.28-3.19 (m, 1H), 4.04-3.98 (m, 3H), 6.48 (dd, 1H), 7.43 (d, 1H), 7.52 (d, 1H), 7.60 (dd, 1H), 7.96 (d, 1H), 8.01 (s, 1H)

logP (HCOOH): 3.18

MS (ESI): 358 ([M-COOC(CH₃)₃+2H]⁺)

### tert-Butyl-4-[4-(5-{2-[(cyclopropylmethoxy)carbonyl]phenyl}-4,5-dihydro-1,2-oxazol-3-yl)-1,3-thiazol-2-yl]piperidin-1-carboxylat (X-a-10)

Zu einer Lösung von 2-(3-{2-[1-(tert-Butoxycarbonyl)piperidin-4-yl]-1,3-thiazol-4-yl}-4,5-dihydro-1,2-oxazol-5-yl)benzoesäure (300 mg) in Dichlormethan (10 mL) wird bei Raumtemperatur Cyclopropylmethanol (47 mg), 4-Dimethylaminopyridin (8 mg) und 1-Ethyl-3-(3'-dimethylaminopropyl)carbodiimide (132 mg) gegeben. Das Gemisch wird für 3 Stunden bei Raumtemperatur gerührt und dann mit Wasser versetzt. Die wässrige Phase wird abgetrennt und mit Ethylacetat extrahiert. Die vereinigten organischen Phasen werden über Natriumsulfat getrocknet und unter vermindertem Druck eingeengt. Der Rückstand wird chromatographisch gereinigt. Man erhält *tert-*Butyl-4-[4-(5-{2-[(cyclopropylmethoxy)carbonyl]phenyl}-4,5-dihydro-1,2-oxazol-3-yl)-1,3-thiazol-2-yl]piperidin-1-carboxylat (270 mg).

¹H NMR (DMSO-d₆): δₚₚₘ: 0.40-0.37 (m, 2H), 0.61-0.56 (m, 2H), 1.30-1.22 (m, 1H), 1.40 (s, 9H), 1.59-1.48 (m, 2H), 2.05-1.98 (m, 2H), 2.88 (bs, 2H), 3.26-3.18 (m, 2H), 4.08-3.96 (m, 3H), 4.15 (dd, 2H), 6.31 (dd, 1H), 7.48 (dd, 1H), 7.56 (d, 1H), 7.65 (dd, 1H), 7.96 (d, 1H), 8.00 (s, 1H)

logP (HCOOH): 4.99

MS (ESI): 412 ([M-COOC(CH₃)₃+2H]⁺)

### Verfahren G und H

### Cyclopropylmethyl-2-{3-[2-(1-{[3,5-bis(difluormethyl)-1H-pyrazol-1-yl]acetyl}piperidin-4-yl)-1,3-thiazol-4-yl]4,5-dihydro-1,2-oxazol-5-yl}benzoat (I-13)

*tert*-Butyl-4-[4-(5-{2-[(cyclopropylmethoxy)carbonyl]phenyl}-4,5-dihydro-1,2-oxazol-3-yl)-1,3-thiazol-2-yl]piperidin-1-carboxylat (270 mg) wurde analog zu I-31 (Verfahren G und H) umgesetzt. Man erhielt Cyclopropylmethyl-2-{3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}benzoat (136 mg).

### Methyl-2-{3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}benzoat (I-43) und Methyl-2-{3-[2-(1-{[3,5-bis(difluormethyl)-1H-pyrazol-1-yl]acetyl}piperidin-4-yl)-5-chlor-1,3-thiazol-4-yl]-4,5-dihydro-1,2-oxazol-5-yl}benzoat (I-46)

### 4-(4-Formyl-1,3-thiazol-2-yl)piperidiniumchlorid

Zu *tert*-Butyl-4-(4-formyl-1,3-thiazol-2-yl)piperidin-1-carboxylat (10 g) wurde bei 0 °C eine 4-molare Lösung von Chlorwasserstoff in 1,4-Dioxan (100 mL) getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-(4-Formyl-1,3-thiazol-2-yl)piperidiniumchlorid (9 g).

¹H NMR (DMSO-d₆): δₚₚₘ : 2.05-1.94 (m, 2H), 2.25-2.17 (m, 2H), 3.09-2.97 (m, 2H), 3.37-3.30 (m, 2H), 3.48-3.40 (m, 1H), 8.68 (s, 1H), 9.15 (bs, 1H), 9.32 (bs, 1H), 9.90 (s, 1H)

### 2-(1-{[3,5-Bis(difluormethyl)-1H-pyrazol-1-yl]acetyl}piperidin-4-yl)-1,3-thiazol-4-carbaldehyd (VI-2)

Zu einer Lösung von [3,5-Bis-(difluormethyl)-1H-pyrazol-1-yl]essigsäure (3.64 g) in Dichlormethan (20 mL) werden bei 0 °C Oxalylchlorid (3.5 mL) und ein Tropfen N,N-Dimethylformamid gegeben. Das Reaktionsgemisch wird bei Raumtemperatur für 10 Minuten gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-(4-Formyl-1,3-thiazol-2-yl)piperidiniumchlorid (3.11 g) und Triethylamin (5.6 mL) in Dichlormethan (20 mL) gegeben. Die Reaktionsmischung wird für 3 Stunden bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-(1-{[3,5-Bis(difluormethyl)-1H-pyrazol-1-yl]acetyl}piperidin-4-yl)-1,3-thiazol-4-carbaldehyd (4.52 g).

¹H NMR (DMSO-d₆): δₚₚₘ : 1.65-1.53 (m, 1H), 1.89-1.77 (m, 1H), 2.18-2.08 (m, 2H), 2.90-2.80 (m, 1H), 3.48-3.22 (m, 2H), 4.00-3.93 (m, 1H), 4.38-4.31 (m, 1H), 5.34 (d, 1H), 5.39 (d, 1H), 6.90 (s, 1H), 7.02 (t, 1H), 7.18 (t, 1H), 8.65 (s, 1H), 9.90 (s, 1H)

logP (HCOOH): 1.93

MS (ESI): 405 ([M+H]⁺)

### Verfahren D und E

### 2-(1-{[3,5-Bis(difluormethyl)-1H-pyrazol-1-y1]acetyl}piperidin-4-yl)-1,3-thiazol-4-carbaldehydoxim

Zu einer Lösung von 2-(1-{[3,5-Bis(difluormethyl)-1H-pyrazol-1-yl]acetyl}piperidin-4-yl)-1,3-thiazol-4-carbaldehyd (487 mg) in Ethanol (5 mL) wurde Hydroxylamin (50% in Wasser, 44 µL) bei Raumtemperatur getropft. Die Reaktionsmischung wurde bei 60 °C für 2 Stunden gerührt, dann wurde das Lösungsmittel unter vermindertem Druck entfernt. Man erhielt 2-(1-{[3,5-Bis(difluormethyl)-1H-pyrazol-1-yl]acetyl}piperidin-4-yl)-1,3-thiazol-4-carbaldehydoxim (454 mg).

logP (HCOOH): 1.93 und 1.98 (2 Isomere)

MS (ESI): 420 ([M+H]⁺)

### Methyl-2-{3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}benzoat (I-43) und Methyl-2-{3-[2-(1-{[3,5-bis(difluormethyl)-1H-pyrazol-1-yl]acetyl}piperidin-4-yl)-5-chlor-1,3-thiazol-4-yl]-4,5-dihydro-1,2-oxazol-5-yl}benzoat (I-46)

Zu einer Lösung von 2-(1-{[3,5-Bis(difluormethyl)-1H-pyrazol-1-yl]acetyl}piperidin-4-yl)-1,3-thiazol-4-carbaldehydoxim (1.00 g) in DMF (4 mL) wurde bei 50 °C N-Chlorsuccinimid (382 mg) gegeben und für 30 Minuten gerührt. Zum Reaktionsgemisch wurde bei Raumtemperatur Triethylamin (1 mL) und Methyl-2-vinylbenzoat (0.50 g) gegeben. Nach Rühren für 2 Stunden bei 50 °C wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt Methyl-2-{3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}benzoat (287 mg) und Methyl-2-{3-[2-(1-{[3,5-bis(difluormethyl)-1H-pyrazol-1-yl]acetyl}piperidin-4-yl)-5-chlor-1,3-thiazol-4-yl]-4,5-dihydro-1,2-oxazol-5-yl}benzoat (193mg).

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-N-cyclopropylbenzamid (I-27)

### 2-{3-[2-(1-{(3,5-Bis(difluormethyl)-1H-pyrazol-1-yl]acetyl}piperidin-4-yl)-1,3-thiazol-4-yl]-4,5-dihydro-1,2-oxazol-5-yl}benzoesäure

Zu einer Lösung von Methyl-2-{3-[2-(1-{[3,5-bis(difluomethyl)-1H-pyrazol-1-yl]acetyl}piperidin-4-yl)-1,3-thiazol-4-yl]-4,5-dihydro-1,2-oxazol-5-yl}benzoat (208 mg) in Tetrahydrofuran (1 mL) und Wasser (0.2 mL) wird bei Raumtemperatur Lithiumhydroxidmonohydrat (23 mg) gegeben. Das Gemisch wird 2 h bei Raumtemperatur gerührt und dann mit eiskalter 1N HCl-Lösung versetzt. Die wässrige Phase wird mit Ethylacetat extrahiert und dann die vereinigten organischen Phasen mit Natriumsulfat getrocknet. Der Feststoff wird abfiltriert und das Lösungsmittel abdestilliert. Man erhält 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzoesäure (158 mg).

¹H NMR (DMSO-d₆): δₚₚₘ : 1.61-1.50 (m, 1H), 1.59-1.73 (m, 1H), 2.15-2.02 (m, 2H), 2.87-2.78 (m, 1H), 3.48-3.15 (m, 3H), 4.05-3.92 (m, 2H), 4.38-4.30 (m, 1H), 5.34 (d, 1H), 5.42 (d, 1H), 6.38 (dd, 1H), 6.89 (s, 1H), 7.02 (t, 1H), 7.17 (t, 1H), 7.43 (dd, 1H), 7.54 (d, 1H), 7.60 (dd, 1H), 7.96 (d, 1H), 8.02 (s, 1H)

logP (HCOOH): 2.63

MS (ESI): 566 ([M+H]⁺)

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-N-cyclopropylbenzamid (I-27)

Zu einer Lösung von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzoesäure (105 mg) in Dichlormethan (10 mL) wird bei Raumtemperatur Cyclopropanamin (11 mg), 4-Dimethylaminopyridin (2 mg) und 1-Ethyl-3-(3'-dimethylaminopropyl)carbodiimide (37 mg) gegeben. Das Gemisch wird für 3 Stunden bei Raumtemperatur gerührt und dann mit Wasser versetzt. Die wässrige Phase wird abgetrennt und mit Ethylacetat extrahiert. Die vereinigten organischen Phasen werden über Natriumsulfat getrocknet und unter vermindertem Druck eingeengt. Der Rückstand wird chromatographisch gereinigt. Man erhält 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-N-cyclopropylbenzamid (78 mg).

### 2-[3,5-Bis(difluormethyl)-1H-pyrazol-1-yl]-1-[4-(4-{5-[2-(hydroxymethyl)phenyl]-4,5-dihydro-1,2-oxazol-3-yl}-1,3-thiazol-2-yl)piperidin-1-yl]ethanon (I-45)

Zu einer Lösung von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzaldehyd (1.9 g) in Methanol (50 mL) wurde bei 0 °C Natriumborhydrid (0.16 g) gegeben und danach 2 Stunden bei Raumtemperatur gerührt. Anschließend wurde die Reaktionsmischung bei 0 °C mit 0.1-molarer Salzsäure und Ethylacetat versetzt. Die organischen Extrakte wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-[4-(4-{5-[2-(hydroxymethyl)phenyl]-4,5-dihydro-1,2-oxazol-3-yl}-1,3-thiazol-2-yl)piperidin-1-yl]ethanon (1.1 g).

### 2-[3,5-Bis(difluormethyl)-1H-pyrazol-1-yl]-1-[4-(4-{5-[2-(chlormethyl)phenyl]-4,5-dihydro-1,2-oxazol-3-yl}-1,3-thiazol-2-yl)piperidin-1-yl]ethanon (I-44)

**Zu einer Lösung von** 2-[3,5-Bis(difluormethyl)-1H-pyrazol-1-yl]-1-[4-(4-{5-[2-(hydroxymethyl)phenyl]-4,5-dihydro-1,2-oxazol-3-yl}-1,3-thiazol-2-yl)piperidin-1-yl]ethanon (0.55 g) in Dichlormethan (10 mL) wurde bei Raumtemperatur Thionylchlorid (0.24 g) und ein Tropfen DMF gegeben und die Mischung danach 2 Stunden refluxiert. Anschließend wurde die Reaktionsmischung unter vermindertem Druck eingeengt. Man erhielt ohne weitere Reinigung 2-[3,5-Bis(difluormethyl)-1H-pyrazol-1-yl]-1-[4-(4-{5-[2-(chlormethyl)phenyl]-4,5-dihydro-1,2-oxazol-3-yl}-1,3-thiazol-2-yl)piperidin-1-yl]ethanon (0.59 g).

### 2-[3,5-Bis(difluormethyl)-1H-pyrazol-1-yl]-1-{4-[4-(5-{2-[(2-methoxyethoxy)methyl]phenyl}-4,5-dihydro-1,2-oxazol-3-yl)-1,3-thiazol-2-yl]piperidin-1-yl}ethanon (I-11)

2-Methoxyethanol (1.5 mL) wurde bei Raumtemperatur Natriumhydrid (60%ig, 17 mg) versetzt und danach 2 Stunden lang bei Raumtemperatur gerührt. Zu dieser Mischung wurde eine Lösung von 2-[3,5-Bis(difluormethyl)-1H-pyrazol-1-yl]-1-[4-(4-{5-[2-(chlormethyl)phenyl]-4,5-dihydro-1,2-oxazol-3-yl}-1,3-thiazol-2-yl)piperidin-1-yl]ethanon (0.20 g) in 2-Methoxyethanol (1.5 g) getropft und die Mischung danach 16 Stunden bei Raumtemperatur gerührt.. Anschließend wurde die Reaktionsmischung mit Wasser und Dichlormethan versetzt. Die organischen Extrakte wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-{4-[4-(5-{2-[(2-methoxyethoxy)methyl]phenyl}-4,5-dihydro-1,2-oxazol-3-yl)-1,3-thiazol-2-yl]piperidin-1-yl}ethanon (0.11 g).

### 2-[3,5-Bis(difluormethyl)-1H-pyrazol-1-yl]-1-{4-[4-(5-{2-[(ethylsulfanyl)methyl]phenyl}-4,5-dihydro-1,2-oxazol-3-yl)-1,3-thiazol-2-yl]piperidin-1-yl}ethanon (I-12)

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzylimidothiocarbamat

Eine Mischung von 2-[3,5-Bis(difluormethyl)-1H-pyrazol-1-yl]-1-[4-(4-{5-[2-(chlormethyl)phenyl]-4,5-dihydro-1,2-oxazol-3-yl}-1,3-thiazol-2-yl)piperidin-1-yl]ethanon (0.20 g) und Thioharnstoff (29 mg) in Ethanol (5 mL) 1 Stunde lang refluxiert. Anschließend wurde die Reaktionsmischung unter vermindertem Druck eingeengt und der Rückstand mit 5 %iger Natriumhydrogencarbonatlösung und Essigester versetzt. Die organischen Extrakte wurden über Natriumsulfat getrocknet und unter vermindertem Druck eingeengt. Der Rückstand wurde in einer Mischung aus Cyclohexan und Essigester (1:2) über Kieselgel filtriert. Man erhielt 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzylimidothiocarbamat (0.15 g).

¹H-NMR : δₚₚₘ: 1.58 (m, 1H), 1.80 (m, 1H), 2.18-2.05 (m, 2H), 2.82 (m, 1H), 3.42-3.20 (m, 3H), 3.97 (m, 2H), 4.32 (m, 2H), 5.40 (dd, 2H), 6.06 (dd, 1H), 6.59 (bs, 1H), 6.91 (s, 1H), 7.03 (t, 1H), 7.18 (t, 1H), 7.45-7.25 (m, 4H), 8.03 (s, 1H)

logP (HCOOH): 1.68

### 2-[3,5-Bis(difluormethyl)-1H-pyrazol-1-yl]-1-{4-[4-(5-{2-[(ethylsulfanyl)methyl]phenyl}-4,5-dihydro-1,2-oxazol-3-yl)-1,3-thiazol-2-yl]piperidin-1-yl}ethanon (I-12)

Zu einer Lösung von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}benzylimidothiocarbamat (0.15 g) in Toluol (5 mL) wurde 50%ige Natronlauge (0.5 mL), Iodethan (42 mg) und ein Tropfen Tetra-n-butylammoniumbromid gegeben. Danach wurde die Mischung 2 Stunden lang bei Raumtemperatur kräftig gerührt. Anschließend wurde die Reaktionsmischung mit Wasser und Essigester versetzt. Die organischen Extrakte wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-{4-[4-(5-{2-[(ethylsulfanyl)methyl]phenyl}-4,5-dihydro-1,2-oxazol-3-yl)-1,3-thiazol-2-yl]piperidin-1-yl}ethanon (50 mg).

### 1-[4-(4-{5-[2-(Allyloxy)phenyl]-4,5-dihydro-1,2-oxazol-3-yl}-1,3-thiazol-2-yl)piperidin-1-yl]-2-[3,5-bis(dilluormethyl)-1H-pyrazol-1-yl]ethanon (I-14)

Zu einer Lösung von 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (81 mg) und Kaliumcarbonat (105 mg) in Aceton (5 mL) wird bei Raumtemperatur Allylbromid (73 mg) gegeben. Das Reaktionsgemisch wird für 5 h bei Rückfluss gerührt. Daraufhin wird das Gemisch mit Wasser versetzt und mit Essigester extrahiert. Die vereinigten organischen Phasen werden über Natriumsulfat getrocknet und eingeengt. Nach säulenchromatographischer Reinigung erhält man 1-[4-(4-{5-[2-(Allyloxy)phenyl]-4,5-dihydro-1,2-oxazol-3-yl}-1,3-thiazol-2-yl)piperidin-1-yl]-2-[3,5-bis(difluormethyl)-1H-pyrazol-1-yl]ethanon (48 mg).

### 2-[3,5-Bis(difluormethyl)-1H-pyrazol-1-yl]-1-{4-[4-(5-{2-[(3-methylbut-2-en-1-yl)oxy]phenyl}-4,5-dihydro-1,2-oxazol-3-yl)-1,3-thiazol-2-yl]piperidin-1-yl}ethanon (I-20) und 2-[3,5-Bis(difluormethyl)-1H-pyrazol-1-yl]-1-{4-[4-(5-{2-[(2-methylprop-2-en-1-yl)oxy]phenyl}-4,5-dihydro-1,2-oxazol-3-yl)-1,3-thiazol-2-yl]piperidin-1-yl}ethanon (I-18) wurden analog zu I-14 umgesetzt.

### 2-[3,5-Bis(difluormethyl)-1H-pyrazol-1-yl]-1-[4-(4-{5-[2-(2-methoxyethoxy)phenyl]-4,5-dihydro-1,2-oxazol-3-yl}-1,3-thiazol-2-yl)piperidin-1-yl]ethanon (I-19)

Zu einer Lösung von 2-[3,5-Bis(difluormethyl)-1H-pyrazol-1-yl]-1-(4-{4-[5-(2-hydroxyphenyl)-4,5-dihydro-1,2-oxazol-3-yl]-1,3-thiazol-2-yl}piperidin-1-yl)ethanon (81 mg) und Kaliumcarbonat (105 mg) in N,N-Dimethylformamid (5 mL) wird bei Raumtemperatur 1-Brom-2-methoxyethan (84 mg) und Kaliumiodid (2.5 mg) gegeben. Das Reaktionsgemisch wird für 3 h bei 80 °C gerührt. Daraufhin wird das Gemisch mit Wasser versetzt und mit Essigester extrahiert. Die vereinigten organischen Phasen werden über Natriumsulfat getrocknet und eingeengt. Nach säulenchromatographischer Rei**nigung erhält t man** 2-[3,5-Bis(difluormethyl)-1H-pyrazol-1-yl]-1-[4-(4-{5-[2-(2-methoxyethoxy)phenyl]-4,5-dihydro-1,2-oxazol-3-yl}-1,3-thiazol-2-yl)piperidin-1-yl]ethanon (41 mg).

### 2-[3,5-Bis(difluormethyl)-1H-pyrazol-1-yl]-1-[4-(4-{5-[3-(2-methoxyethoxy)phenyl]-4,5-dihydro-1,2-oxazol-3-yl}-1,3-thiazol-2-yl)piperidin-1-yl]ethanon (I-28)

### Verfahren D und E

### tert-Butyl-4-(4-{5-[3-(2-methoxyethoxy)phenyl]-4,5-dihydro-1,2-oxazol-3-yl}-1,3-thiazol-2-yl)piperidin-1-carboxylat (X-a-11)

*tert*-Butyl-4-{4-[(hydroxyimino)methyl]-1,3-thiazol-2-yl}piperidin-1-carboxylat (250 mg) und **1-(2-**Methoxyethoxy)-3-vinylbenzol wurden analog zu I-31 (Verfahren D und E) umgesetzt. Man erhielt tert-Butyl-4-(4-{5-[3-(2-methoxyethoxy)phenyl]-4,5-dihydro-1,2-oxazol-3-yl}-1,3-thiazol-2-yl)piperidin-1-carboxylat (170 mg)

¹H NMR (DMSO-d₆): δₚₚₘ : 8.00 (s, 1H), 7.29 (t, 1H), 7.10-6.80 (m, 3H), 5.68 (dd, 1H), 4.15-3.95 (m, 4H), 3.84 (dd, 1H), 3.70-3.60 (m, 2 H), 3.45-3.20 (m), 2.95-2.80 (m, 2H), 2.08-1.98 (m, 2H), 1.63-1.48 'm, 2H), 1.40 (s, 9H)

Der Reaktant 1-(2-Methoxyethoxy)-3-vinylbenzol wurde ausgehend von 3-(2-Methoxyethoxy)-benzaldehyd nach dem Fachmann geläufigen Vorschriften (Wittig Reaktion: Chem. Rev. 1989, 89, 863-927) synthetisiert.

### Verfahren G und H

### 2-[3,5-Bis(difluormethyl)-1H-pyrazol-1-yl]-1-[4-(4-{5-[3-(2-methoxyethoxy)phenyl]-4,5-dihydro-1,2-oxazol-3-yl}-1,3-thiazol-2-yl)piperidin-1-yl]ethanon (I-28)

tert-Butyl-4-(4-{5-[3-(2-methoxyethoxy)phenyl]-4,5-dihydro-1,2-oxazol-3-yl}-1,3-thiazol-2-yl)piperidin-1-carboxylat (279 mg) wurde analog zu I-31 (Verfarhren G und H) umgesetzt. Man erhielt 2-[3,5-Bis(difluormethyl)-1H-pyrazol-1-yl]-1-[4-(4-{5-[3-(2-methoxyethoxy)phenyl]-4,5-dihydro-1,2-oxazol-3-yl}-1,3-thiazol-2-yl)piperidin-1-yl]ethanon (180 mg).

### 2-[3,5-Bis(difluormethyl)-1H-pyrazol-1-yl]-1-(4-{4-[5-(2-chlor-6-hydroxyphenyl)-4,5-dihydro-1,2-oxazol-3-yl]-1,3-thiazol-2-yl}piperidin-1-yl)ethanon (I-54)

### Verfahren D und E

### tert-Butyl-4-{4-[5-(2-chlor-6-hydroxyphenyl)-4,5-dihydro-1,2-oxazol-3-yl]-1,3-thiazol-2-yl}piperidin-1-carboxylat (X-a-12)

Zu einer Lösung von *tert*-Butyl-4-{4-[(hydroxyimino)methyl]-1,3-thiazol-2-yl}piperidin-1-carboxylat (400 mg) in Ethylacetat (10 mL) wurde N-Chlorsuccinimid (206 mg) gegeben. Das Reaktionsgemisch wird für 30 min bei Rückfluss gerührt. Zu dem Reaktionsgemisch wurde 3-Chlor-2-vinylphenol (397 mg) und Kaliumhydrogencarbonat (257 mg) bei Raumtemperatur gegeben und anschließend ein Tropfen Wasser. Nach Rühren über Nacht bei Raumtemperatur wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(2-chlor-6-hydroxyphenyl)-4,5-dihydro-1,2-oxazol-3-yl]-1,3-thiazol-2-yl}piperidin-1-carboxylat (345 mg).

¹HNMR (DMSO-d₆): δₚₚₘ : 7.95 (s, 1H), 7.18 (t, 1H), 6.92 (d, 1H), 6.82 (d, 1H), 6.17 (dd, 1H), 4.1-3.95 (m), 3.30-3.15 (m), 3.05-2.75 (m, 2H), 2.08-2.0 (m, 2H), 1.62-1.50 (m, 2H), 1.40 (s, 9H)

Auch in diesem Fall wurde der Reaktant 3-Chlor-2-vinylphenol ausgehend von 2-Chlor-6-hydroxybenzaldehyd nach dem Fachmann geläufigen Synthesevorschriften (Wittig Reaktion: Chem. Rev. 1989, 89, 863-927) synthetisiert.

### Verfahren G und H

### 2-[3,5-Bis(difluormethyl)-1H-pyrazol-1-yl]-1-(4-{4-[5-(2-chlor-6-hydroxyphenyl)-4,5-dihydro-1,2-oxazol-3-yl]-1,3-thiazol-2-yl}piperidin-1-yl)ethanon (I-54)

Zu einer Lösung von *tert*-Butyl-4-{4-[5-(2-chlor-6-hydroxyphenyl)-4,5-dihydro-1,2-oxazol-3-yl]-1,3-thiazol-2-yl}piperidin-1-carboxylat (346 mg) in Dichlormethan wurde bei 0 °C eine 4-molare Lösung von Chlorwasserstoff (4.0 Äq.) in 1,4-Dioxan getropft. Das Reaktionsgemisch wurde bei 0 °C gerührt und dann langsam auf Raumtemperatur erwärmt. Nach Rühren über 5 Stunden wurden das Lösungsmittel und überschüssiger Chlorwasserstoff entfernt. Man erhielt 4-{4-[5-(2-Chlor-6-hydroxyphenyl)-4,5-dihydro-1,2-oxazol-3-yl]-1,3-thiazol-2-yl}piperidiniumchlorid (XII-9).

Zu einer Lösung von [3,5-Bis-(difluormethyl)-1H-pyrazol-1-yl]essigsäure (177 mg) in Dichlormethan (40 mL) werden bei 0 °C Oxalylchlorid (290 mg) und ein Tropfen N,N-Dimethylformamid gegeben. Das Reaktionsgemisch wird bei Raumtemperatur für 2 Stunden gerührt. Das Lösungsmittel und das überschüssige Reagenz werden unter vermindertem Druck entfernt. Der feste Rückstand wird erneut in Dichlormethan gelöst und bei 0 °C tropfenweise zu einer Lösung von 4-{4-[5-(2-Chlor-6-hydroxyphenyl)-4,5-dihydro-1,2-oxazol-3-yl]-1,3-thiazol-2-yl}piperidiniumchlorid und Triethylamin (5.0 Äq.) in Dichlormethan (25 mL) gegeben. Die Reaktionsmischung wird über Nacht bei Raumtemperatur gerührt. Daraufhin wird sie mit konzentrierter Natriumhydrogencarbonatlösung versetzt, die wässrige Phase abgetrennt und mit Essigsäureethylester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-(4-{4-[5-(2-chlor-6-hydroxyphenyl)-4,5-dihydro-1,2-oxazol-3-yl]-1,3-thiazol-2-yl}piperidin-1-yl)ethanon (110 mg).

¹H NMR (DMSO-d₆): δₚₚₘ : 10.28 (s, 1H), 7.98 (s, 1H), 7.30-6.80 (m, 6H), 6.17 (dd, 1H), 5.40 (dd, 2H), 4.36 (d, 1H), 3.97 (d, 1H), 3.71-3.55 (m, 2H), 3.32-3.24 (m, 1H), 2.84 (t, 1H), 2.16-2.08 (m, 2H), 1.85-1.75 (m, 1H), 1.62-1.53 (m, 1H).

Log P (HCOOH): 2.94

### 1-[4-(4-{5-[2-(Allyloxy)-6-chlorphenyl]-4,5-dihydro-1,2-oxazol-3-yl}-1,3-thiazol-2-yl)piperidin-1-yl]-2-[3,5-bis(difluormethyl)-1H-pyrazol-1-yl]ethanon (I-51)

Zu einer Lösung von 2-[3,5-Bis(difluormethyl)-1H-pyrazol-1-yl]-1-(4-{4-[5-(2-chlor-6-hydroxyphenyl)-4,5-dihydro-1,2-oxazol-3-yl]-1,3-thiazol-2-yl}piperidin-1-yl)ethanon (39 mg) und Kaliumcarbonat (47 mg) in Aceton (5 mL) wird bei Raumtemperatur Allylbromid (33 mg) gegeben. Das Reaktionsgemisch wird für 5 h bei Rückfluss gerührt. Daraufhin wird das Gemisch mit Wasser versetzt und mit Essigester extrahiert. Die vereinigten organischen Phasen werden über Natriumsulfat getrocknet und eingeengt. Nach säulenchromatographischer Reinigung erhält man 1-[4-(4-{5-[2-(Allyloxy)-6-chlorphenyl]-4,5-dihydro-1,2-oxazol-3-yl}-1,3-thiazol-2-yl)piperidin-1-yl]-2-[3,5-bis(difluormethyl)-1H-pyrazol-1-yl]ethanon (26 mg).

### 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon (I-49)

Zu einer Lösung von 2-[3,5-Bis(difluormethyl)-1H-pyrazol-1-yl]-1-(4-{4-[5-(2-chlor-6-hydroxyphenyl)-4,5-dihydro-1,2-oxazol-3-yl]-1,3-thiazol-2-yl}piperidin-1-yl)ethanon (33 mg) und Kaliumcarbonat (12 mg) in N,N-Dimethylformamid (5 mL) werden bei Raumtemperatur Kaliumiodid (5 mg) und 3-Bromprop-1-in (11 mg) gegeben. Das Reaktionsgemisch wird für 9 h bei 80 °C gerührt. Daraufhin wird das Gemisch mit verdünnter Salzsäure versetzt und mit Essigester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon (19 mg).

### 2-[3,5-Bis(difluormethyl)-1H-pyrazol-1-yl]-1-[4-(4-{5-[2-chlor-6-(2-methoxyethoxy)phenyl]-4,5-dihydro-1,2-oxazol-3-yl}-1,3-thiazol-2-yl)piperidin-1-yl]ethanon (I-50)

Zu einer Lösung von 2-[3,5-Bis(difluormethyl)-1H-pyrazol-1-yl]-1-(4-{4-[5-(2-chlor-6-hydroxyphenyl)-4,5-dihydro-1,2-oxazol-3-yl]-1,3-thiazol-2-yl}piperidin-1-yl)ethanon (39 mg) und Kaliumcarbonat (47 mg) in N,N-Dimethylformamid (5 mL) werd bei Raumtemperatur 1-Brom-2-methoxyethan (38 mg) und Kaliumiodid (1.1 mg) gegeben. Das Reaktionsgemisch wird für 3 h bei 80 °C gerührt. Daraufhin wird das Gemisch mit Wasser versetzt und mit Essigester extrahiert. Die vereinigten organischen Phasen werden über Natriumsulfat getrocknet und eingeengt. Nach säulenchromatographischer Reinigung erhält man 2-[3,5-Bis(difluormethyl)-1H-pyrazol-1-yl]-1-[4-(4-{5-[2-chlor-6-(2-methoxyethoxy)phenyl]-4,5-dihydro-1,2-oxazol-3-yl}-1,3-thiazol-2-yl)piperidin-1-yl]ethanon (17 mg).

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-fluorbenzaldehyd (I-17)

### 3-Fluor-2-vinylbenzaldehyd

Zu einer Lösung von 1-Brom-3-fluor-2-vinylbenzol (900 mg) in THF (40 mL) wurde bei -78 °C n-Butyllithium (3.4 ml 1.6 M/Hexan) zugetropft. Nach 60 Minuten wurde bei -78 °C N,N-Dimethyl-formamid (0.69 ml) zugetropft. Nach 60-minutigem Rühren bei -78 °C wurde die Reaktionsmischung mit Wasser versetzt und danach auf Raumtemperatur erwärmt. Daraufhin wurde das Gemisch mit Wasser versetzt und mit Essigester extrahiert. Die vereinigten organischen Phasen wurden über Natriumsulfat getrocknet und eingeengt. Nach säulenchromatographischer Reinigung erhält man 3-Fluor-2-vinylbenzaldehyd (160 mg).

Auch in diesem Fall wurde der Reaktant 1-Brom-3-fluor-2-vinylbenzol ausgehend von 2-Brom-6-fluorbenzaldehyd nach dem Fachmann geläufigen Synthesevorschriften (Wittig Reaktion: Chem. Rev. 1989, 89, 863-927) synthetisiert.

### Verfahren D und E

### tert-Butyl-4-{4-[5-(2-fluor-6-formylphenyl)-4,5-dihydro-1,2-oxazol-3-yl]-1,3-thiazol-2-yl}piperidin-1-carboxylat (X-a-13)

Zu einer Lösung von *tert*-Butyl-4-{4-[(hydroxyimino)methyl]-1,3-thiazol-2-yl}piperidin-1-carboxylat (166 mg) in Ethylacetat (10 mL) wurde N-Chlorsuccinimid (85.5 mg) gegeben. Das Reaktionsgemisch wurde für 30 min bei Rückfluss gerührt. Zu diesem Reaktionsgemisch wurde bei Raumtemperatur 3-Fluor-2-vinylbenzaldehyd (160 mg) und Kaliumhydrogencarbonat (106.7 mg) gegeben und anschließend ein Tropfen Wasser. Nach Rühren über Nacht bei Raumtemperatur wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(2-fluor-6-formylphenyl)-4,5-dihydro-1,2-oxazol-3-yl]-1,3-thiazol-2-yl}piperidin-1-carboxylat (110 mg)

¹H-NMR (DMSO-d₆): 10.26 (s, 1H), 8.03 (s, 1H), 7.76 (dd, 1 H), 7.68-7.64 (m, 1H), 7.62-7.57 (m, 1H), 6.55 (t, 1H), 4.08-3.90 (m, 3H), 3.53 (dd, 1H), 3.40-3.30 (m, 1H), 2.05-2.00 (m, 2H), 1.62-1.52 (m, 2H), 1.40 (s, 9H). logP (HCO2H): 3.62

### Verfahren G und H

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-fluorbenzaldehyd (I-17)

Zu einer Lösung *tert*-Butyl-4-{4-[5-(2-fluor-6-formylphenyl)-4,5-dihydro-1,2-oxazol-3-yl]-1,3-thiazol-2-yl}piperidin-1-carboxylat (112 mg) in Dichlormethan wurde bei 0 °C eine 4-molare Lösung von Chlorwasserstoff (4.0 Äq.) in 1,4-Dioxan getropft. Das Reaktionsgemisch wurde bei 0 °C gerührt und dann langsam auf Raumtemperatur erwärmt. Nach Rühren über 5 Stunden wurden das Lösungsmittel und überschüssiger Chlorwasserstoff entfernt. Man erhielt 4-{4-[5-(2-Fluor-6-formylphenyl)-4,5-dihydro-1,2-oxazol-3-yl]-1,3-thiazol-2-yl}piperidiniumchlorid (XII-10).

Zu einer Lösung von [3,5-Bis-(difluormethyl)-1H-pyrazol-1-yl]essigsäure (58 mg) in Dichlormethan (20 mL) wurden bei 0 °C Oxalylchlorid (94.5 mg) und ein Tropfen N,N-Dimethylformamid gegeben. Das Reaktionsgemisch wurde bei Raumtemperatur für 2 Stunden gerührt. Das Lösungsmittel und das überschüssige Reagenz wurden unter vermindertem Druck entfernt. Der feste Rückstand wurde erneut in Dichlormethan (5 mL) gelöst und bei 0 °C tropfenweise zu einer Lösung von 4-{4-[5-(2-Fluor-6-formylphenyl)-4,5-dihydro-1,2-oxazol-3-yl]-1,3-thiazol-2-yl}piperidiniumchlorid und Triethylamin (5.0 Äq.) in Dichlormethan (15 ml) gegeben. Die Reaktionsmischung wurde über Nacht bei Raumtemperatur gerührt. Daraufhin wurde sie mit konzentrierter Natriumhydrogencarbonatlösung versetzt, die wässrige Phase abgetrennt und mit Essigsäureethylester extrahiert. Die vereinigten organischen Phasen wurden über Natriumsulfat getrocknet und eingeengt. Nach säulenchromatographischer Reinigung erhielt man 2-{3-[2-(1-{[3,5-Bis(difluorinethyl)-1H-pyrazol-1-yl]acetyl}-piperidin-4-yl)-1,3-thiazol-4-yl]-4,5-dihydro-1,2-oxazol-5-yl}-3-fluorbenzaldehyd (55 mg).

### Beispiele

**Tabelle 1**

| | | | | | | | |
|---|---|---|---|---|---|---|---|
| 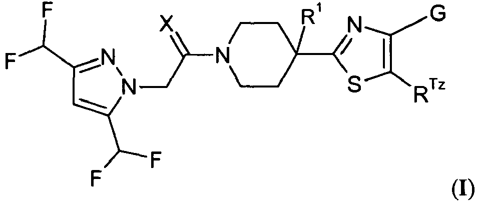 | | | | | | | |
| In Tabelle 1 kommt die Gruppe G insbesondere in den Spezifikationen G3 und G4 vor: | | | | | | | |
| G3 bedeutet 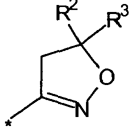 G4 bedeutet: 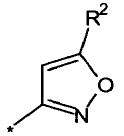 | | | | | | | |

| **Bsp** | **X** | **R¹** | **G** | **R²** | **R³** | **R^{Tz}** | **logp** |
|---|---|---|---|---|---|---|---|
| I-1 | O | H | G3 | Phenyl | H | H | 3,17^{[b]};3,11^{[c]} |
| I-2 | O | H | G3 | 2,6-Difluorphenyl | H | H | 3,14^{[b]} |
| I-3 | O | H | G4 | Phenyl | | H | 3,66^{[b]} |
| I-4 | O | Fluor | G3 | 2-Fluorphenyl | H | H | 3,49^{[b]} |
| I-5 | O | Fluor | G3 | Phenyl | H | H | 3,41^{[b]} |
| I-6 | O | Fluor | G3 | 2,6-Difluorphenyl | H | H | 3,43^{[b]};3,34^{[c]} |
| I-7 | O | H | G3 | 2-Acetylphenyl | H | H | 3,08^{[b]} |
| I-8 | O | H | G3 | 3-Acetylphenyl | H | H | 2,78^{[b]} |
| I-9 | O | H | G3 | 2-Hydroxyphenyl | H | H | 2,58^{[a]};2,6^{[c]} |
| I-10 | O | H | G3 | 2-Nitrophenyl | H | H | 3,19^{[b]} |
| I-11 | O | H | G3 | 2-[(2-Methoxyethoxy)methyl]phenyl | H | H | 3,21^{[b]} |
| I-12 | O | H | G3 | 2-[(Ethylsulfanyl)methyl]phenyl | H | H | 3,9^{[b]} |
| I-13 | O | H | G3 | 2-[(Cyclopropylmethoxy)carbonyl]phenyl | H | H | 3,92^{[c]};3,99^{[b]} |
| I-14 | O | H | G3 | 2-(Allyloxy)phenyl | H | H | 3,63^{[b]};3,65^{[c]} |
| I-15 | O | H | G3 | 3-(But-2-in-1-yloxy)phenyl | H | H | 3,33^{[c]};3,29^{[a]} |
| I-16 | O | H | G3 | 2-(Butoxymethyl)phenyl | H | H | 4,24^{[b]} |
| I-17 | O | H | G3 | 2-Fluor-6-formylphenyl | H | H | 2,82^{[c]};2,92^{[b]} |
| I-18 | O | H | G3 | 2-[(2-Methylprop-2-en-1-yl)oxy]phenyl | H | H | 3,96^{[b]};3,92^{[c]} |
| I-19 | O | H | G3 | 2-(2-Methoxyethoxy)phenyl | H | H | 3,18^{[c]};3,26^{[b]} |
| I-20 | O | H | G3 | 2-[(3-Methylbut-2-en-1-yl)oxy]phenyl | H | H | 4,19^{[b]};4,11^{[c]} |
| I-21 | O | H | G3 | 3-(Prop-2-in-1-yloxy)phenyl | H | H | 3,16^{[c]};3,24^{[a]} |
| I-22 | O | H | G3 | 4-(Prop-2-in-1-yloxy)phenyl | H | H | 3,14^{[a]};3,08^{[c]} |
| I-23 | O | H | G3 | 3-Formylphenyl | H | H | 2,78^{[b]} |
| I-24 | O | H | G3 | 2-(Cyclohexylmethoxy)phenyl | H | H | 4,9^{[a]};4,9^{[c]} |
| I-25 | O | H | G3 | 2-(Pent-2-in-1-yloxy)phenyl | H | H | 3,91^{[b]} |
| I-26 | O | H | G3 | 2-Formylphenyl | H | H | 2,98^{[b]} |
| I-27 | O1 | H | G3 | 2-(Cyclopropylcarbamoyl)phenyl | H | H | 2,64^{[b]} |
| I-28 | O | H | G3 | 3-(2-Methoxyethoxy)phenyl | H | H | 3,03^{[b]};3,04^{[c]} |
| I-29 | O | H | G3 | 2-(But-2-in-1-yloxy)-6-fluorphenyl | H | H | 3,46^{[a]};3,42^{[c]} |
| I-30 | O | H | G3 | 2-Fluor-6-(prop-2-in-1-yloxy)phenyl | H | H | 3,18^{[a]};3,15^{[c]} |
| I-31 | O | H | G4 | 2-Formylphenyl | | H | 13,25^{[b]} |
| I-32 | O | H | G3 | 2-[(Cyclohexylcarbonyl)oxy]phenyl | H | H | 4,27^{[a]};4,19^{[c]} |
| I-33 | O | H | G3 | 2-[(Cyclopropylcarbonyl)oxy]phenyl | H | H | 3,28^{[c]};3,34^{[a]} |
| I-34 | O | H | G3 | 3-(Pent-2-in-1-yloxy)phenyl | H | H | 3,74^{[c]};3,83^{[a]} |
| I-35 | O | H | G3 | 2-(But-2-in-1-yloxy)phenyl | H | H | 3,55^{[c]};3,65^{[a]} |
| I-36 | O | H | G3 | 2-[(3,3,3-Trifluorpropanoyl)oxy]phenyl | H | H | 9,43^{[a]};3,5^{[c]} |
| I-37 | O | H | G3 | 2-[(Methylsulfonyl)amino]phenyl | H | H | 2,51^{[b]} |
| I-38 | O | H | G3 | 2-Ethinylphenyl | H | H | 3,36^{[b]} |
| I-39 | O | H | G3 | 2-(Prop-2-in-1-yloxy)phenyl | H | H | 3,23^{[c]};3,28^{[a]} |
| I-40 | O | H | G3 | 4-[(Methylsulfonyl)amino]phenyl | H | H | 2,41^{[b]} |
| I-41 | O | H | G3 | 2-Aminophenyl | H | H | 2,71^{[c]};2,7^{[a]} |
| I-42 | O | H | G3 | 3-Hydroxyphenyl | H | H | 2,45^{[c]};2,43^{[a]} |
| I-43 | O | H | G3 | 2-(Methoxycarbonyl)phenyl | H | H | 3,3^{[b]} |
| I-44 | O | H | G3 | 2-(Chloromethyl)phenyl | H | H | 3,36^{[b]} |
| I-45 | O | H | G3 | 2-(Hydroxymethyl)phenyl | H | H | 2,45^{[b]} |
| I-46 | O | H | G3 | 2-(Methoxycarbonyl)phenyl | H | Chlor | 3,98^{[b]} |
| I-47 | O | H | G3 | 4-(Pent-2-in-1-yloxy)phenyl | H | H | 3,74^{[c]};3,76^{[a]} |
| I-48 | O | H | G3 | 4-(But-2-in-1-yloxy)phenyl | H | H | 3,42^{[c]};3,43^{[a]} |
| I-49 | O | H | G3 | 2-Chlor-6-(prop-2-in-1-yloxy)phenyl | H | H | 3,5^{[b]}3,34^{[c]} |
| I-50 | O | H | G3 | 2-Chlor-6-(2-methoxyethoxy)phenyl | H | H | 3,36^{[b]} |
| I-51 | O | H | G3 | 2-(Allyloxy)-6-chlorphenyl | H | H | 3,71^{[c]};3,82^{[b]} |
| I-52 | O | H | G3 | 2-[(2,2,2-Trifluorethoxy)methyl]phenyl | H | H | 3,6^{[c]};3,4^{[b]} |
| I-53 | O | H | G3 | 2-[(Ethylsulfonyl)methyl]phenyl | H | H | 2,76^{[b]} |
| I-54 | O | H | G3 | 2-Chlor-6-hydroxyphenyl | H | H | 2,94^{[b]} |

Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril.
^{[b]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
^{[c]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

| **Bsp** | **NMR-Daten** |
|---|---|
| I-1 | ¹H-NMR : δₚₚₘ : 1.63 (m, 1H), 1.79 (m, 1H), 2.18-2.05 (m, 2H), 2.88 (m, 1H), 3.42-3.20 (m, 3H), 3.87 (dd, 1H), 3.98 (m, 1H), 4.32 (m, 1H), 5.35 (bs, 2H), 5.72 (dd, 1H), 6.85 (s, 1H), 6.97 (t, 1H), 7.14 (t, 1H), 7.40-7.30 (m, 5H), 7.97 (s, 1H) |
| I-2 | ¹H-NMR : δₚₚₘ : 1.58 (m, 1H), 1.83 (m, 1H), 2.18-2.07 (m, 2H), 2.85 (m, 1H), 3.25 (m, 1H), 3.41 (m, 1H), 3.52 (dd, 1H), 3.90 (dd, 1H), 3.98 (m, 1H), 4.36 (m, 1H), 5.35 (d, 1H), 5.47 (d, 1H), 6.01 (dd, 1H), 6.90 (s, 1H), 7.02 (t, 1H), 7.18 (t, 1H), 7.19-7.12 (m, 2H), 7.50 (m, 1H), 8.03 (s, 1H) |
| I-3 | ¹H-NMR : δₚₚₘ : 1.66 (m, 1H), 1.85 (m, 1H), 2.22-2.10 (m, 2H), 2.88 (dd, 1H), 3.50-3.45 (m, 2H), 4.00 (d, 1H), 4.39 (d, 1H), 5.37 (d, 1H), 5.45 (d, 1H), 6.91 (s, 1H), 7.03 (t, 1H), 7.19 (t, 1H), 7.44 (s, 1H), 7.60-7.53 (m, 3H), 7.96 (dd, 2H), 8.23 (s, 1H) |
| I-4 | ¹H-NMR : δₚₚₘ : 2.45-2.05 (m, 4H), 3.10 (m, 1H), 3.44 (dd, 1H), 3.47 (m, 1H), 3.98-3.90 (m, 2H), 4.28 (m, 1H), 5.40 (d, 1H), 5.51 (d, 1H), 5.94 (dd, 1H), 6.91 (s, 1H), 7.03 (t, 1H), 7.18 (t, 1H), 7.32-7.20 (m, 2H), 7.48-7.38 (m, 2H), 8.24 (s, 1H) |
| I-5 | ¹H-NMR : δₚₚₘ : 2.45-2.05 (m, 4H), 3.11 (m, 1H), 3.38 (dd, 1H), 3.48 (m, 1H), 3.95-3.86 (m, 2H), 4.26 (m, 1H), 5.39 (d, 1H), 5.50 (d, 1H), 5.76 (dd, 1H), 6.91 (s, 1H), 7.03 (t, 1H), 7.18 (t, 1H), 7.42-7.33 (m, 5H), 8.22 (s, 1H) |
| I-6 | ¹H-NMR (CD₃CN) : δₚₚₘ : 2.50-2.10 (m, 4H), 3.18 (m, 1H), 3.55 (m, 1H), 3.59 (dd, 1H), 3.89-3.78 (m, 2H), 4.36 (m, 1H), 5.22 (d, 1H), 5.29 (d, 1H), 6.07 (dd, 1H), 6.79 (t, 1H), 6.83 (s, 1H), 6.91 (t, 1H), 7.07-6.99 (m, 2H), 7.42 (m, 1H), 7.88 (s, 1H) |

Die chemischen NMR-Verschiebungen in ppm wurden bei 400 MHz falls nicht anders angegeben im Lösungsmittel DMSO-d₆ mit Tetramethylsilan als internem Standard gemessen.

Folgende Abbkürzungen beschreiben die Signalaufspaltung:
b = breit, s = Singulett, d = Dublett, t = Triplett, q = Quadruplett, m = Multiplett

### NMR-Daten ausgewählter Beispiele

### NMR-Peak-Listenverfahren

Die 1H-NMR-Daten ausgewählter Beispiele sind in Form von 1H-NMR-Peaklisten notiert. Zu jedem Signalpeak sind der 5-Wert in ppm und die Signalintensität in Klammern aufgeführt.

| |
|---|
| **Bsp. I-8, Solvent: DMSO-d₆** |
| 8,0369 (5,64); 7,953 (3,33); 7,9506 (3,17); 7,9343 (1,15); 7,9312 (1,42); 7,6713 (1,04); 7,652 (1,46); 7,5832 (1,24); 7,5643 (1,87); 7,5446 (0,75); 7,307 (1); 7,1737 (2,29); 7,1587 (1,17); 7,0404 (1,14); 7,0226 (2,57); 6,9004 (2,47); 6,8868 (1,32); 5,8661 (0,82); 5,8458 (1); 5,8389 (0,98); 5,8188 (0,86); 5,4508 (0,47); 5,408 (1,81); 5,3671 (1,73); 5,3247 (0,46); 4,3643 (0,54); 4,33 (0,57); 4,0573 (0,63); 4,0395 (1,85); 4,0217 (1,88); 4,0039 (0,73); 3,984 (0,53); 3,9632 (1,19); 3,9508 (0,62); 3,9359 (1,24); 3,92 (1,23); 3,8927 (1,02); 3,4369 (1,22); 3,4167 (1,62); 3,4071 (0,58); 3,3936 (1,63); 3,3881 (1,26); 3,3735 (1,77); 3,3194 (308,6); 3,2418 (0,85); 2,891 (0,55); 2,8724 (0,42); 2,8441 (0,72); 2,8149 (0,43); 2,7325 (0,4); 2,6704 (0,34); 2,5949 (16); 2,5522 (0,37); 2,5404 (0,69); 2,5235 (1,54); 2,5102 (19,34); 2,5059 (35,33); 2,5015 (45,46); 2,4971 (31,98); 2,4929 (15,8); 2,3283 (0,36); 2,1377 (0,55); 2,1018 (1,05); 2,0693 (0,92); 1,9871 (8,04); 1,8117 (0,49); 1,7902 (0,44); 1,5915 (0,45); 1,5827 (0,5); 1,5611 (0,46); 1,5519 (0,44); 1,398 (2,53); 1,2365 (0,34); 1,193 (2,23); 1,1752 (4,38); 1,1575 (2,15); 0,0078 (0,39); -0,0002 (7,25); -0,0082 (0,34) |
| **Bsp. I-9, Solvent: DMSO-d₆** |
| 9,7066 (7,97); 8,0287 (0,34); 8,0022 (10,13); 7,3071 (1,98); 7,2167 (2,69); 7,1976 (2,79); 7,1737 (4,5); 7,1591 (2,52); 7,1535 (1,76); 7,1494 (1,48); 7,1338 (2,64); 7,1147 (1,75); 7,1106 (1,5); 7,0405 (2,26); 7,0231 (4,87); 6,8986 (5,15); 6,8873 (2,7); 6,8638 (3,65); 6,8438 (3,23); 6,8125 (1,98); 6,7939 (3,39); 6,7754 (1,61); 5,8556 (1,76); 5,8368 (2,06); 5,8279 (2); 5,8092 (1,77); 5,4492 (0,98); 5,4074 (3,65); 5,3673 (3,74); 5,3251 (0,97); 4,3592 (1,22); 4,3252 (1,24); 4,0571 (1,37); 4,0393 (3,86); 4,0215 (3,89); 4,0038 (1,48); 3,9781 (1,2); 3,9449 (1,26); 3,8484 (1,8); 3,8204 (2,02); 3,8055 (2,29); 3,7778 (1,96); 3,4036 (1,2); 3,3939 (1,1); 3,3846 (1,56); 3,3756 (2,26); 3,3661 (1,82); 3,3464 (2,39); 3,3065 (242,8); 3,2791 (3,62); 3,2656 (2,17); 3,2552 (2,76); 3,2364 (2,8); 2,8685 (0,84); 2,8404 (1,49); 2,8107 (0,85); 2,6692 (0,58); 2,6648 (0,47); 2,5392 (1,55); 2,5046 (63,17); 2,5003 (76,51); 2,4964 (53,38); 2,3316 (0,41); 2,327 (0,5); 2,1301 (1,13); 2,0982 (2,27); 2,0692 (1,31); 1,9869 (16); 1,8411 (0,47); 1,8172 (0,97); 1,811 (1,01); 1,7889 (0,92); 1,759 (0,37); 1,6137 (0,42); 1,6042 (0,48); 1,5829 (0,94); 1,5746 (0,98); 1,5538 (0,95); 1,5452 (0,87); 1,5238 (0,37); 1,3981 (0,73); 1,1928 (4,43); 1,175 (8,57); 1,1572 (4,18); -0,0002 (2,26) |
| **Bsp. I-10, Solvent: DMSO-d₆** |
| 9,9026 (0,53); 8,7786 (0,82); 8,6473 (0,5); 8,4336 (0,4); 8,1699 (2,2); 8,1671 (2,25); 8,1494 (2,4); 8,1466 (2,37); 8,0449 (8,69); 7,8303 (0,96); 7,8276 (0,99); 7,81 (2,12); 7,7925 (1,53); 7,7897 (1,47); 7,6818 (2,46); 7,665 (1,83); 7,6621 (1,84); 7,6401 (1,46); 7,6366 (1,32); 7,6191 (2,13); 7,6013 (1,16); 7,5979 (1,04); 7,305 (1,64); 7,1717 (3,75); 7,1581 (1,97); 7,0384 (1,89); 7,0221 (4,3); 6,8984 (4,07); 6,8862 (2,33); 6,2689 (1,29); 6,2533 (1,45); 6,2409 (1,46); 6,2251 (1,32); 5,7475 (1,13); 5,4471 (0,79); 5,4048 (2,82); 5,365 (2,84); 5,3233 (0,77); 4,3558 (0,94); 4,3252 (1,02); 4,3078 (0,59); 4,2898 (0,36); 4,1407 (1,39); 4,1124 (1,59); 4,0965 (1,79); 4,0684 (1,57); 4,0573 (1,34); 4,0395 (3,7); 4,0217 (3,75); 4,0039 (1,38); 3,9768 (0,91); 3,9436 (0,96); 3,4422 (1,76); 3,4264 (1,82); 3,4113 (0,58); 3,3982 (2,02); 3,3825 (2,38); 3,3737 (1,46); 3,3641 (0,94); 3,3541 (0,78); 3,3445 (1,03); 3,3352 (0,97); 3,3023 (178,37); 3,2798 (2,37); 3,2613 (1,61); 3,2312 (0,85); 3,0381 (0,37); 2,8638 (0,7); 2,8507 (0,64); 2,8327 (1,16); 2,8038 (0,66); 2,6696 (0,41); 2,6649 (0,32); 2,5396 (0,72); 2,5092 (23,41); 2,5049 (43,1); 2,5005 (56,19); 2,4961 (40,14); 2,4918 (20,08); 2,3319 (0,33); 2,3275 (0,41); 2,1465 (0,37); 2,1243 (0,96); 2,0891 (1,7); 2,0702 (0,87); 2,0549 (0,95); 1,9871 (16); 1,8344 (0,43); 1,8118 (0,79); 1,8063 (0,8); 1,7814 (0,74); 1,6064 (0,38); 1,5981 (0,43); 1,5768 (0,77); 1,5683 (0,78); 1,5476 (0,73); 1,5382 (0,67); 1,3977 (1,14); 1,3202 (0,35); 1,3026 (0,69); 1,2848 (0,36); 1,2363 (0,38); 1,193 (4,46); 1,1752 (8,83); 1,1574 (4,38); 0,0596 (1,49); -0,0002 (3,18) |
| **Bsp. I-11, Solvent: CD₃CN** |
| 7,6716 (4,88); 7,4701 (1,37); 7,4579 (1,37); 7,4558 (1,26); 7,3757 (0,71); 7,366 (1,87); 7,3544 (2); 7,3193 (1,14); 7,3079 (1,33); 7,2958 (0,49); 6,9878 (1,11); 6,898 (2,29); 6,8776 (1,16); 6,8311 (2,32); 6,8083 (1,15); 6,7866 (2,38); 6,6957 (1,19); 5,9944 (1,02); 5,98 (1,14); 5,9761 (1,13); 5,9617 (1,04); 5,2629 (0,75); 5,2346 (2,39); 5,203 (2,4); 5,1748 (0,78); 4,6614 (1,85); 4,6422 (2,48); 4,534 (2,57); 4,5148 (1,92); 4,4734 (0,56); 4,4508 (0,59); 4,0646 (0,52); 4,0527 (0,52); 3,9375 (1,16); 3,919 (1,47); 3,9088 (1,74); 3,8903 (1,81); 3,633 (0,33); 3,6312 (0,34); 3,622 (0,93); 3,6155 (1,02); 3,6127 (1,16); 3,6068 (1,68); 3,6003 (1,56); 3,593 (1,26); 3,5821 (0,5); 3,575 (0,41); 3,5279 (2,27); 3,5209 (2,9); 3,5178 (1,41); 3,5142 (1,25); 3,5114 (1,13); 3,3636 (0,49); 3,3509 (0,52); 3,3445 (1); 3,3381 (0,53); 3,3254 (0,53); 3,3069 (0,46); 3,3023 (0,6); 3,2958 (16); 3,283 (1,39); 3,2669 (1,45); 3,2594 (0,49); 3,2527 (1,3); 3,2383 (1,21); 3,224 (1,19); 2,8775 (0,38); 2,873 (0,42); 2,8557 (0,73); 2,8523 (0,75); 2,8351 (0,45); 2,8306 (0,41); 2,201 (69,88); 2,1761 (1,1); 2,1451 (0,6); 2,1232 (0,61); 1,9733 (2,23); 1,9665 (1,21); 1,9583 (0,58); 1,9504 (8,08); 1,9463 (14,95); 1,9422 (21,76); 1,9381 (14,82); 1,934 (7,55); 1,8641 (0,53); 1,8576 (0,54); 1,8431 (0,5); 1,8364 (0,52); 1,6993 (0,54); 1,6927 (0,57); 1,6786 (0,53); 1,672 (0,51); 1,4358 (0,96); 1,2157 (0,58); 1,2039 (1,15); 1,192 (0,58); 1,1138 (0,43); -0,0002 (3,37) |
| **Bsp. I-12, Solvent: DMSO-d₆** |
| 8,0249 (9,55); 8,018 (0,5); 7,3706 (1,21); 7,3647 (1,69); 7,348 (2,04); 7,3268 (1,05); 7,3187 (1,26); 7,3109 (2,51); 7,3054 (3,71); 7,3007 (3,98); 7,2948 (1,66); 7,2903 (3,15); 7,2842 (4,3); 7,2772 (1,28); 7,2741 (1,16); 7,2685 (1,2); 7,2553 (0,42); 7,1752 (3,67); 7,1646 (1,82); 7,042 (1,78); 7,0286 (4,12); 6,9069 (3,51); 6,8928 (2); 6,0983 (1,32); 6,0778 (1,61); 6,0706 (1,59); 6,0501 (1,38); 5,4543 (0,81); 5,4117 (2,73); 5,37 (2,71); 5,3271 (0,8); 4,3626 (0,8); 4,3301 (0,86); 4,0165 (1,37); 3,9887 (2,07); 3,9734 (2,27); 3,9457 (2,23); 3,9192 (1,95); 3,8865 (3,67); 3,8314 (3,75); 3,7985 (1,95); 3,5567 (0,35); 3,5284 (0,34); 3,5202 (0,37); 3,5117 (0,33); 3,4896 (0,71); 3,4738 (0,77); 3,4573 (1,27); 3,4434 (1,57); 3,4376 (1,71); 3,3605 (1918,14); 3,3154 (7,54); 3,2944 (5,31); 3,2716 (3,59); 3,2513 (2,77); 3,2419 (1,37); 3,236 (1,44); 3,1808 (0,62); 3,153 (0,39); 3,1441 (0,39); 3,1275 (0,39); 3,1088 (0,34); 3,0997 (0,34); 3,0366 (0,38); 2,8643 (0,67); 2,8337 (1,11); 2,8064 (0,66); 2,6818 (0,5); 2,6773 (0,95); 2,6728 (1,31); 2,6682 (0,99); 2,6636 (0,52); 2,5428 (0,86); 2,5258 (3,63); 2,5127 (71,69); 2,5082 (143,99); 2,5036 (190,58); 2,499 (138,34); 2,4945 (68,05); 2,4728 (8,16); 2,4544 (2,6); 2,3396 (0,51); 2,3349 (0,97); 2,3304 (1,33); 2,3257 (0,98); 2,3213 (0,52); 2,1309 (0,76); 2,0983 (1,52); 2,0724 (2,06); 2,0645 (0,89); 1,9887 (1,18); 1,8357 (0,37); 1,814 (0,68); 1,8056 (0,73); 1,7838 (0,68); 1,7757 (0,65); 1,7534 (0,32); 1,6126 (0,35); 1,6031 (0,37); 1,5818 (0,68); 1,5728 (0,74); 1,5511 (0,72); 1,5426 (0,67); 1,5216 (0,33); 1,4083 (0,58); 1,4018 (1,38); 1,3975 (3,65); 1,2468 (0,49); 1,2348 (1,13); 1,2098 (7,77); 1,1915 (16); 1,1815 (0,75); 1,173 (7,39); 1,1632 (0,52); 1,1571 (0,51); -0,0002 (2,47) |
| **Bsp. 1-13, Solvent: DMSO-d6** |
| 9,6057 (0,54); 8,2467 (0,32); 8,0242 (16); 7,9807 (4,9); 7,9789 (5,09); 7,9613 (5,33); 7,9594 (5,41); 7,6688 (1,95); 7,651 (4,61); 7,6335 (3,5); 7,6311 (3,38); 7,5754 (6,21); 7,5559 (3,99); 7,4997 (3,02); 7,4806 (4,85); 7,462 (2,37); 7,4596 (2,24); 7,4309 (0,6); 7,3103 (3,18); 7,2985 (1,58); 7,2187 (0,36); 7,1926 (0,81); 7,1769 (6,82); 7,1667 (4,6); 7,1588 (1,71); 7,0569 (1,42); 7,0438 (3,68); 7,0306 (7,77); 6,9685 (1,07); 6,9211 (1,09); 6,9073 (8,56); 6,895 (4,7); 6,8611 (0,87); 6,3432 (2,94); 6,3268 (3,57); 6,3227 (2,96); 6,3155 (3,57); 6,3069 (2,32); 6,2992 (3,1); 5,7558 (9,53); 5,4535 (1,72); 5,4109 (6,13); 5,3724 (6,29); 5,3547 (1,01); 5,3299 (1,91); 5,3073 (2,85); 5,0692 (0,44); 4,3568 (1,99); 4,3242 (2,46); 4,2663 (0,93); 4,2552 (1,08); 4,2435 (0,97); 4,2242 (0,45); 4,2144 (0,37); 4,1905 (0,9); 4,1795 (1,43); 4,1621 (8,14); 4,1571 (8,22); 4,1436 (8,48); 4,1391 (7,89); 4,1101 (1,18); 4,0779 (2,79); 4,0499 (3,03); 4,0339 (3,39); 4,006 (3); 3,9757 (1,91); 3,9412 (2,17); 3,9051 (0,41); 3,7197 (0,55); 3,71 (0,93); 3,7025 (1,42); 3,6921 (1,76); 3,6756 (2,52); 3,6662 (2); 3,6626 (1,99); 3,6539 (1,21); 3,3958 (1,33); 3,3856 (1,23); 3,3755 (1,92); 3,3669 (3,52); 3,3372 (183,1); 3,2889 (2,29); 3,2593 (5,73); 3,2434 (4,25); 3,2277 (2,14); 3,2156 (3,76); 3,1994 (3,23); 3,0397 (0,96); 2,8529 (1,59); 2,8294 (2,58); 2,7993 (1,59); 2,5085 (31,73); 2,5042 (40,36); 2,5006 (31,19); 2,1191 (1,74); 2,0847 (3,76); 2,0484 (2,3); 1,9901 (0,75); 1,9116 (0,53); 1,8388 (0,7); 1,8288 (0,81); 1,8077 (1,59); 1,8 (1,7); 1,7771 (1,66); 1,7691 (1,56); 1,7472 (0,89); 1,6014 (0,74); 1,5924 (0,93); 1,5708 (1,64); 1,5626 (1,79); 1,5404 (1,69); 1,532 (1,7); 1,5103 (0,79); 1,5016 (0,72); 1,4187 (0,45); 1,396 (0,45); 1,3124 (0,58); 1,3056 (0,97); 1,2937 (1,9); 1,2858 (1,89); 1,2822 (1,76); 1,2741 (2,96); 1,2624 (2,15); 1,2554 (2,32); 1,2434 (1,7); 1,235 (2,16); 1,1935 (0,39); 1,1757 (0,59); 1,1579 (0,48); 1,145 (0,41); 0,6187 (1,63); 0,6077 (6,47); 0,6032 (7,52); 0,5875 (6,99); 0,5833 (7,15); 0,5745 (2,7); 0,555 (0,38); 0,4071 (2,63); 0,3943 (8,38); 0,385 (8,14); 0,3822 (8,4); 0,371 (2,61); -0,0002 (6,9) |
| **Bsp. I-14, Solvent: DMSO-d₆** |
| 8,0236 (0,7); 8,0022 (16); 7,3308 (0,39); 7,31 (4,8); 7,3047 (5,83); 7,294 (5,45); 7,2907 (5,94); 7,2867 (3,62); 7,2762 (2,96); 7,272 (1,65); 7,2604 (0,35); 7,1711 (6,22); 7,1574 (3,08); 7,098 (0,36); 7,0613 (4,54); 7,0385 (5,81); 7,0214 (7,05); 6,9759 (2,57); 6,9556 (4,33); 6,9388 (2,08); 6,8984 (6); 6,8855 (3,41); 6,0801 (0,82); 6,0676 (1,68); 6,0539 (1,23); 6,041 (1,97); 6,0369 (1,1); 6,0285 (1,1); 6,0243 (2,09); 6,0112 (1,46); 5,9978 (1,99); 5,9853 (0,98); 5,9175 (2,25); 5,8995 (2,61); 5,8897 (2,54); 5,8715 (2,27); 5,7458 (1,02); 5,4481 (1,46); 5,4408 (1,86); 5,4364 (3,92); 5,4321 (3,92); 5,4278 (1,69); 5,406 (4,76); 5,3977 (2,07); 5,3931 (3,63); 5,3888 (3,51); 5,3846 (1,53); 5,3636 (4,56); 5,3206 (1,24); 5,249 (1,58); 5,2453 (3,51); 5,2413 (3,35); 5,2224 (1,44); 5,2188 (3,21); 5,2148 (3,09); 4,6659 (0,44); 4,6531 (0,55); 4,6319 (5,25); 4,6273 (5,61); 4,6235 (4,66); 4,6195 (5,22); 4,6149 (4,69); 4,5934 (0,44); 4,5807 (0,37); 4,3587 (1,45); 4,3249 (1,48); 4,0571 (1,18); 4,0393 (3,19); 4,0215 (3,25); 4,0036 (1,32); 3,9797 (1,39); 3,943 (1,53); 3,8883 (2,58); 3,8602 (2,89); 3,8454 (3,27); 3,8174 (2,78); 3,6096 (0,34); 3,5899 (0,32); 3,581 (0,32); 3,5439 (0,41); 3,4592 (0,77); 3,4119 (2,02); 3,4044 (2,54); 3,384 (3,45); 3,3751 (4,96); 3,3656 (4,99); 3,3168 (1799,9); 3,2952 (11,71); 3,2702 (5,7); 3,2455 (3,61); 3,2275 (3,65); 2,8652 (1); 2,8349 (1,7); 2,8071 (0,97); 2,6745 (0,73); 2,6699 (0,91); 2,6653 (0,7); 2,54 (1,38); 2,5097 (53,59); 2,5054 (99,23); 2,5009 (128,95); 2,4965 (89,43); 2,4921 (43,1); 2,4526 (0,36); 2,3323 (0,63); 2,3277 (0,83); 2,3232 (0,59); 2,1295 (1,28); 2,0945 (2,65); 2,069 (2,03); 2,0582 (1,46); 1,9868 (13,72); 1,9081 (0,4); 1,8418 (0,51); 1,8321 (0,56); 1,8033 (1,16); 1,7809 (1,08); 1,7503 (0,44); 1,6001 (0,58); 1,5701 (1,17); 1,5485 (1,11); 1,5401 (1,07); 1,5208 (0,44); 1,2365 (0,93); 1,1929 (3,85); 1,1751 (7,58); 1,1573 (3,78); -0,0002 (1,21) |
| **Bsp. I-15, Solvent: DMSO-d₆** |
| 19,3402 (0,52); 18,635 (0,51); 16,0748 (0,5); 15,6162 (0,51); 10,962 (0,5); 8,7762 (1,87); 8,0216 (12,7); 7,338 (1,94); 7,3172 (3,86); 7,3056 (2,78); 7,2972 (2,3); 7,1726 (5,32); 7,158 (2,83); 7,1274 (0,51); 7,0389 (2,79); 7,0219 (6,63); 6,9886 (2,92); 6,9713 (5,97); 6,9679 (5,9); 6,9426 (2,25); 6,9374 (1,8); 6,9239 (2,13); 6,9204 (2,16); 6,916 (1,65); 6,8985 (5,7); 6,886 (3,09); 5,7459 (3,25); 5,7257 (1,7); 5,7056 (2,21); 5,6986 (2,02); 5,6795 (1,74); 5,4503 (1,08); 5,4065 (3,97); 5,3662 (4,29); 5,3405 (0,56); 5,3239 (1,22); 4,9643 (0,56); 4,8644 (0,94); 4,7451 (2,83); 4,7392 (6,89); 4,7334 (6,77); 4,3597 (1,41); 4,3248 (1,57); 4,2932 (0,57); 4,254 (0,53); 4,0647 (0,54); 4,0492 (0,56); 4,0238 (0,77); 4,0151 (0,8); 3,9841 (1,58); 3,9491 (1,86); 3,9194 (0,74); 3,9007 (1,97); 3,873 (2,14); 3,8577 (2,62); 3,8301 (2,25); 3,8081 (0,75); 3,7756 (0,67); 3,7622 (0,75); 3,7313 (0,88); 3,681 (0,88); 3,6589 (0,97); 3,6381 (1,03); 3,6044 (1,02); 3,6014 (1,13); 3,5919 (1,14); 3,5702 (1,18); 3,5265 (1,44); 3,5016 (1,8); 3,488 (1,94); 3,4506 (2,67); 3,4442 (2,95); 3,3911 (8,54); 3,3836 (9); 3,3714 (11,9); 3,312 (9031,04); 3,2888 (42,26); 3,2562 (4,61); 3,2375 (2,49); 3,2002 (0,97); 2,8742 (1,14); 2,8423 (1,67); 2,8136 (1,06); 2,7557 (0,71); 2,7114 (0,6); 2,689 (0,92); 2,6739 (5,79); 2,6696 (7,64); 2,6649 (5,69); 2,66 (2,96); 2,6526 (1,24); 2,6439 (1,02); 2,6164 (2,23); 2,5395 (10,71); 2,5226 (33,05); 2,5094 (449,88); 2,505 (830,26); 2,5005 (1078,36); 2,4961 (734,7); 2,4916 (343,72); 2,4181 (0,66); 2,3318 (5,5); 2,3272 (7,16); 2,3226 (5,11); 2,318 (2,55); 2,1526 (0,69); 2,1332 (1,28); 2,1035 (2,53); 2,0689 (2,9); 1,8059 (8,69); 1,8001 (16); 1,7943 (7,63); 1,7485 (0,63); 1,6193 (0,53); 1,583 (1,21); 1,5655 (0,81); 1,5568 (1,11); 1,3515 (0,62); 1,2977 (0,67); 1,2593 (1,19); 1,2361 (3,44); 1,1523 (0,5); 1,1082 (0,83); 1,0908 (1,05); 0,8911 (0,5); 0,8613 (1,13); 0,0079 (2,63); -0,0002 (60,11); -0,0085 (2,24); -2,4058 (0,5) |
| **Bsp. I-16, Solvent: DMSO-d₆** |
| 8,0088 (10,55); 7,3947 (1,35); 7,3898 (1,87); 7,3842 (1,49); 7,3778 (2,38); 7,3724 (3,88); 7,3657 (3,51); 7,3594 (1,85); 7,3461 (2,79); 7,3421 (1,98); 7,3275 (3,4); 7,3219 (2,58); 7,3096 (2,42); 7,3051 (2,79); 7,2924 (0,86); 7,2872 (0,72); 7,1726 (3,67); 7,1632 (1,95); 7,0442 (0,73); 7,0394 (1,84); 7,0272 (4,12); 6,9623 (0,49); 6,9066 (3,51); 6,8914 (2,05); 5,9844 (1,31); 5,9645 (1,63); 5,9566 (1,58); 5,9367 (1,39); 5,6319 (0,74); 5,4476 (0,7); 5,405 (2,7); 5,3699 (2,69); 5,3272 (0,73); 5,2701 (0,39); 4,5849 (1,61); 4,5554 (3,94); 4,5218 (4,01); 4,4922 (1,69); 4,3564 (0,76); 4,3237 (0,85); 4,1907 (0,6); 4,0563 (0,83); 4,0385 (2,51); 4,0207 (2,6); 4,0029 (0,91); 3,9773 (0,73); 3,9441 (0,8); 3,9205 (1,41); 3,8926 (1,52); 3,8773 (1,73); 3,8495 (1,41); 3,5021 (0,56); 3,4856 (1,17); 3,4788 (1,82); 3,4626 (4,53); 3,4471 (4,64); 3,4403 (1,69); 3,4311 (2,29); 3,424 (2,09); 3,4047 (3,38); 3,3648 (663,23); 3,2947 (3,3); 3,2745 (2,64); 3,2675 (1,8); 3,2514 (2,31); 3,2316 (2,44); 3,1937 (0,37); 3,1863 (0,35); 2,8676 (0,6); 2,8366 (1,04); 2,8103 (0,62); 2,6779 (0,33); 2,6733 (0,45); 2,6687 (0,35); 2,5265 (1,15); 2,5133 (25,22); 2,5088 (51,37); 2,5042 (68,85); 2,4996 (50,01); 2,495 (24,56); 2,3354 (0,37); 2,3309 (0,49); 2,3263 (0,36); 2,1286 (0,71); 2,0973 (1,47); 2,0725 (0,98); 2,0611 (0,9); 2,0228 (1,11); 2,0072 (0,51); 1,9889 (11,5); 1,9102 (1,26); 1,8302 (0,37); 1,8086 (0,67); 1,8007 (0,71); 1,7787 (0,69); 1,7706 (0,64); 1,7491 (0,34); 1,6148 (0,38); 1,6044 (0,43); 1,5847 (0,75); 1,5742 (0,87); 1,5686 (0,94); 1,5648 (0,89); 1,5508 (2,22); 1,5482 (2,2); 1,5434 (1,71); 1,5344 (2,8); 1,5304 (2,52); 1,5181 (1,67); 1,513 (3,06); 1,4965 (1,43); 1,4786 (0,59); 1,462 (0,56); 1,4549 (0,41); 1,4407 (0,4); 1,3831 (0,68); 1,3645 (2,01); 1,3454 (3,11); 1,3266 (3,26); 1,3086 (2,13); 1,2903 (0,88); 1,2813 (0,59); 1,235 (2,24); 1,2102 (0,51); 1,2011 (0,58); 1,1929 (3,68); 1,1751 (6,89); 1,1692 (0,96); 1,1573 (3,46); 1,1119 (0,33); 0,8923 (7,76); 0,8739 (16); 0,8554 (7,42); 0,8433 (1,11); 0,8357(1); -0,0002 (4,51) |
| **Bsp. I-17, Solvent: DMSO-d₆** |
| 10,2605 (6,56); 9,8996 (1,37); 8,6648 (1,24); 8,048 (14,68); 7,7701 (2,71); 7,7681 (2,95); 7,7574 (3,7); 7,7553 (3,67); 7,6819 (1,07); 7,6733 (1,16); 7,6685 (1,93); 7,6601 (1,93); 7,6554 (1,23); 7,647 (1,1); 7,6113 (1,52); 7,6095 (1,53); 7,5978 (1,16); 7,5957 (1,23); 7,5931 (1,73); 7,5911 (1,61); 7,5794 (1,09); 7,5774 (1,07); 7,3707 (0,39); 7,282 (0,85); 7,2731 (1,85); 7,1934 (0,5); 7,1845 (4,41); 7,1399 (0,46); 7,1277 (2,02); 7,096 (2,12); 7,0495 (1,09); 7,0373 (5,43); 6,9593 (0,54); 6,947 (2,39); 6,9132 (4,67); 6,5802 (1,51); 6,5631 (1,94); 6,5438 (1,51); 5,7615 (16); 5,4563 (1,4); 5,4278 (3,17); 5,3792 (3,33); 5,3508 (1,46); 5,0918 (0,47); 4,3659 (1,01); 4,3437 (1,03); 4,0459 (1,09); 4,0341 (3,28); 4,0222 (3,3); 4,0104 (1,11); 3,979 (1,53); 3,959 (1,77); 3,9561 (1,57); 3,9502 (1,29); 3,9471 (1,24); 3,9273 (1,05); 3,5858 (1,32); 3,568 (1,64); 3,5574 (1,16); 3,5395 (1,13); 3,4304 (0,6); 3,4237 (0,94); 3,417 (0,75); 3,4108 (1,14); 3,4046 (1,86); 3,3984 (1,29); 3,392 (1,08); 3,3854 (1,5); 3,3488 (364,36); 3,3255 (4,74); 3,2936 (0,88); 3,2736 (1,38); 3,2551 (0,76); 3,0367 (0,34); 2,8638 (0,74); 2,8483 (1,28); 2,8264 (0,74); 2,6179 (0,66); 2,6149 (0,95); 2,6119 (0,65); 2,5426 (0,66); 2,5242 (1,66); 2,5211 (2,12); 2,518 (2,39); 2,5092 (54,14); 2,5062 (120,28); 2,5031 (167,97); 2,5001 (118,69); 2,497 (50,51); 2,3903 (0,58); 2,3873 (0,88); 2,3842 (0,6); 2,1466 (0,86); 2,1274 (1,02); 2,1072 (0,92); 2,0877 (0,93); 2,0768 (1,48); 1,99 (14,99); 1,9096 (0,4); 1,8457 (0,37); 1,8257 (0,8); 1,8052 (0,76); 1,6076 (0,34); 1,5888 (0,75); 1,5688 (0,77); 1,234 (0,57); 1,1862 (4,33); 1,1744 (9,3); 1,1626 (4,08); 1,0551 (0,32); 0,0053 (1,57); -0,0002 (50,67); -0,0057 (1,48) |
| **Bsp. I-18, Solvent: DMSO-d₆** |
| 8,012 (15,01); 7,3025 (4,08); 7,2994 (2,15); 7,2909 (4,11); 7,2897 (4,25); 7,2889 (4,36); 7,2869 (2,41); 7,2792 (2,16); 7,2763 (1,24); 7,2679 (1,71); 7,1793 (4,09); 7,1258 (1,87); 7,0908 (1,96); 7,0511 (2,91); 7,0499 (3,08); 7,0452 (0,57); 7,0353 (7,48); 6,9669 (1,77); 6,9656 (1,74); 6,9545 (3,16); 6,9532 (3,28); 6,945 (2,37); 6,9422 (1,81); 6,9408 (1,62); 6,9111 (4,11); 5,9209 (1,7); 5,9091 (1,96); 5,9025 (1,84); 5,8905 (1,71); 5,7615 (16); 5,4504 (1,42); 5,422 (3,14); 5,3707 (3,11); 5,3424 (1,42); 5,0906 (3,19); 5,0894 (3,21); 4,9454 (3,14); 4,5462 (0,76); 4,5248 (3,65); 4,5148 (3,71); 4,4932 (0,75); 4,3533 (0,93); 4,3316 (0,95); 4,0341 (0,38); 4,0223 (0,37); 3,9698 (0,85); 3,9473 (0,92); 3,8827 (1,89); 3,8642 (2,31); 3,8544 (2,45); 3,8359 (1,99); 3,5679 (1,49); 3,3996 (0,42); 3,3932 (0,79); 3,3869 (0,55); 3,3804 (0,9); 3,3737 (1,81); 3,3692 (1,06); 3,3674 (1,15); 3,3488 (538,95); 3,3251 (3,75); 3,287 (2,31); 3,2751 (2,72); 3,2586 (3,24); 3,2467 (2,29); 3,238 (0,74); 3,2337 (0,58); 2,8506 (0,55); 2,8466 (0,66); 2,826 (1,16); 2,8083 (0,68); 2,618 (0,39); 2,6149 (0,53); 2,6119 (0,39); 2,5242 (0,7); 2,5211 (0,9); 2,518 (0,85); 2,5092 (28); 2,5062 (63,2); 2,5031 (87,71); 2,5001 (61,35); 2,497 (26,88); 2,3904 (0,38); 2,3873 (0,52); 2,3842 (0,36); 2,1173 (0,79); 2,0956 (0,92); 2,0767 (1,37); 2,0573 (0,87); 1,99 (1,75); 1,8163 (0,4); 1,8021 (0,77); 1,7964 (0,99); 1,7845 (15,03); 1,762 (0,35); 1,563 (0,72); 1,5563 (0,76); 1,5427 (0,73); 1,5361 (0,69); 1,2335 (0,41); 1,1862 (0,49); 1,1744 (0,98); 1,1625 (0,49); 0,0052 (0,56); -0,0002 (19,67); -0,0058 (0,48) |
| **Bsp. I-19, Solvent: DMSO-d₆** |
| 7,9976 (2,12); 7,9829 (16); 7,3208 (2,15); 7,3167 (3,02); 7,3041 (7,32); 7,2999 (8,68); 7,2813 (7,15); 7,2609 (0,49); 7,2412 (0,34); 7,2375 (0,35); 7,1713 (7,18); 7,1576 (3,49); 7,0691 (4,58); 7,0489 (3,89); 7,038 (3,87); 7,0214 (7,92); 6,9888 (0,49); 6,9721 (3,03); 6,9553 (4,72); 6,9362 (2,77); 6,9152 (0,92); 6,8984 (7,15); 6,8856 (3,94); 5,9563 (0,33); 5,9291 (0,33); 5,8518 (2,45); 5,833 (2,84); 5,824 (2,78); 5,8049 (2,47); 5,7461 (10,38); 5,4488 (1,5); 5,4059 (5,41); 5,3629 (5,28); 5,3201 (1,47); 4,8695 (2,28); 4,3592 (1,77); 4,325 (1,83); 4,1728 (0,6); 4,156 (5,47); 4,1449 (8,21); 4,1328 (5,55); 4,1068 (0,33); 4,0571 (0,4); 4,0392 (0,95); 4,0212 (0,98); 4,0037 (0,56); 3,9788 (1,72); 3,9471 (1,77); 3,9125 (0,51); 3,8847 (0,51); 3,869 (0,61); 3,8499 (2,63); 3,8422 (0,77); 3,8218 (3,05); 3,8071 (3,52); 3,779 (2,93); 3,7293 (0,32); 3,6912 (0,34); 3,6615 (0,56); 3,6334 (5,73); 3,6212 (6,37); 3,6112 (4,36); 3,5913 (0,63); 3,5808 (0,53); 3,5098 (0,57); 3,4853 (0,69); 3,4427 (1,07); 3,4066 (2,9); 3,3781 (4,95); 3,3687 (5,02); 3,3123 (2048,24); 3,2908 (15,02); 3,2726 (6,61); 3,233 (53,23); 3,1991 (0,47); 3,1883 (0,43); 2,8704 (1,23); 2,8373 (2,06); 2,8084 (1,11); 2,6742 (1); 2,6696 (1,27); 2,665 (0,94); 2,5394 (1,88); 2,5093 (73,81); 2,505 (136,17); 2,5006 (176,7); 2,4962 (123,71); 2,4919 (60,26); 2,3319 (0,88); 2,3273 (1,19); 2,3226 (0,94); 2,1909 (4,67); 2,1267 (1,5); 2,0943 (3,12); 2,0691 (2,56); 2,0592 (1,75); 1,9868 (3,35); 1,908 (0,6); 1,8417 (0,6); 1,8319 (0,66); 1,8093 (1,34); 1,8028 (1,41); 1,78 (1,28); 1,7503 (0,56); 1,6118 (0,66); 1,605 (0,69); 1,5798 (1,35); 1,5731 (1,39); 1,5494 (1,28); 1,5429 (1,23); 1,5213 (0,52); 1,5111 (0,54); 1,2366 (0,82); 1,1927 (0,99); 1,1749 (1,87); 1,1571 (1); -0,0002 (3,93) |
| **Bsp. I-20, Solvent: DMSO-d₆** |
| 8,0084 (0,89); 7,9873 (10,75); 7,3268 (0,42); 7,313 (1,28); 7,3089 (1,93); 7,3049 (2,44); 7,2901 (6,41); 7,2714 (5,75); 7,2543 (0,4); 7,1715 (4,74); 7,1572 (2,24); 7,1135 (0,39); 7,0914 (0,41); 7,0734 (2,9); 7,0537 (2,65); 7,0382 (2,36); 7,0212 (5,18); 6,9542 (1,78); 6,9355 (3,12); 6,9169 (1,58); 6,898 (4,6); 6,8852 (2,5); 5,8386 (1,55); 5,8204 (1,85); 5,8104 (1,72); 5,792 (1,62); 5,7466 (1,92); 5,4479 (0,96); 5,4061 (3,68); 5,3796 (2,33); 5,3761 (2,08); 5,3639 (4,29); 5,3227 (0,87); 4,5753 (4,18); 4,5595 (3,72); 4,5259 (0,51); 4,3602 (1,11); 4,3273 (1,11); 4,0568 (0,71); 4,0392 (2,12); 4,0213 (2,17); 4,0036 (0,88); 3,98 (1,02); 3,9458 (1,07); 3,8391 (1,71); 3,8104 (1,9); 3,7961 (2,2); 3,7678 (1,82); 3,5678 (0,92); 3,4054 (1,21); 3,3968 (1,19); 3,3868 (1,62); 3,3774 (2,32); 3,3675 (1,99); 3,3076 (966,41); 3,2843 (14,1); 3,2747 (5,11); 3,2497 (2,72); 3,2315 (2,71); 2,8903 (1,83); 2,8675 (0,75); 2,8405 (1,29); 2,8103 (0,68); 2,7313 (1,4); 2,6739 (0,63); 2,6693 (0,8); 2,665 (0,62); 2,5392 (1,1); 2,509 (47,63); 2,5047 (88,76); 2,5003 (115,69); 2,4959 (80,61); 2,4915 (38,79); 2,3314 (0,58); 2,3271 (0,78); 2,3221 (0,57); 2,1247 (0,99); 2,1161 (1,57); 2,0939 (1,98); 2,0692 (1,2); 2,0615 (1,12); 1,9868 (9,1); 1,8411 (0,43); 1,833 (0,48); 1,8037 (0,88); 1,7812 (1,19); 1,7462 (0,85); 1,6883 (16); 1,6711 (13,36); 1,6147 (0,87); 1,5814 (1,04); 1,5507 (0,88); 1,5431 (0,83); 1,5216 (0,43); 1,512 (0,39); 1,3983 (2,96); 1,3523 (0,58); 1,2986 (0,34); 1,2587 (0,51); 1,2359 (1,43); 1,1927 (2,52); 1,175 (5,09); 1,1571 (2,64); 1,1421 (2,46); 1,1205 (0,39); 0,0079 (1,11); -0,0002 (24,37); -0,0085 (1,09) |
| **Bsp. I-21, Solvent: DMSO-d₆** |
| 19,9888 (0,96); 8,7773 (2,8); 8,0193 (16); 7,3512 (2,89); 7,3311 (6,43); 7,3106 (4,08); 7,3061 (3,56); 7,1727 (6,69); 7,1581 (3,79); 7,0396 (3,35); 7,0221 (8,31); 7,0073 (4,13); 6,9899 (9,48); 6,9708 (3,6); 6,9522 (2,67); 6,8982 (7,66); 6,8863 (4,58); 5,7254 (2,15); 5,7048 (2,75); 5,6978 (2,67); 5,6775 (2,23); 5,4502 (1,34); 5,4072 (5,03); 5,3667 (5,84); 5,325 (1,55); 4,8067 (14,14); 4,8007 (14,52); 4,7457 (0,98); 4,3573 (1,76); 4,3279 (1,93); 3,9827 (1,72); 3,9459 (1,87); 3,9011 (2,58); 3,8738 (2,92); 3,8579 (3,19); 3,8308 (2,68); 3,543 (3,63); 3,5371 (7,54); 3,5309 (3,71); 3,3966 (6,6); 3,3763 (7,98); 3,3535 (11,12); 3,3033 (8172,92); 3,0367 (1,37); 2,8727 (1,94); 2,8404 (2,75); 2,8131 (2,05); 2,6735 (9,82); 2,6688 (15,1); 2,6643 (9,66); 2,6595 (5,58); 2,5389 (17,62); 2,522 (52,17); 2,5087 (690,63); 2,5043 (1289,14); 2,4998 (1683,73); 2,4954 (1157,27); 2,491 (547,17); 2,3358 (4,09); 2,331 (8,04); 2,3265 (11); 2,322 (7,85); 2,1376 (1,7); 2,1029 (3,11); 2,069 (7,23); 1,9865 (1,42); 1,8197 (1,54); 1,7898 (1,46); 1,5909 (1,65); 1,5608 (1,44); 1,3984 (1,61); 1,3517 (1,09); 1,2979 (1,04); 1,2587 (1,67); 1,236 (5,02); 1,1748 (0,98); 0,8542 (0,88); 0,1462 (0,95); 0,0079 (9,36); -0,0002 (181,58); -0,0085 (5,8); -0,1492 (0,7) |
| **Bsp. I-22, Solvent: DMSO-d₆** |
| 9,9022 (0,44); 8,7772 (0,5); 8,6463 (0,42); 8,013 (16); 7,7728 (0,64); 7,7581 (0,33); 7,7407 (0,5); 7,5929 (0,52); 7,3484 (9,21); 7,3313 (4,39); 7,3267 (10,28); 7,3088 (3,3); 7,2042 (0,38); 7,1754 (7,08); 7,1595 (3,59); 7,0421 (3,74); 7,0234 (8,65); 7,0126 (11,26); 7,0076 (4,29); 6,9955 (3,95); 6,9907 (9,69); 6,9573 (0,62); 6,9001 (7,6); 6,8876 (4,1); 5,7464 (13,29); 5,6954 (2,45); 5,6739 (3,26); 5,6688 (2,96); 5,6472 (2,43); 5,4524 (1,52); 5,4104 (5,36); 5,3688 (5,44); 5,3262 (1,43); 4,8008 (14,95); 4,7949 (14,86); 4,7407 (0,94); 4,3647 (1,74); 4,3317 (1,8); 4,0571 (0,8); 4,0392 (2,27); 4,0214 (2,32); 4,0037 (1,21); 3,985 (1,69); 3,9517 (1,8); 3,853 (2,59); 3,826 (2,95); 3,81 (3,38); 3,7831 (2,88); 3,5499 (3,63); 3,544 (7,26); 3,5382 (3,57); 3,4766 (0,33); 3,4424 (0,49); 3,4165 (1,72); 3,407 (1,58); 3,3895 (5,58); 3,3785 (2,68); 3,3685 (4,75); 3,3595 (2,82); 3,3469 (4,7); 3,3252 (7,94); 3,3047 (416,16); 3,281 (7,73); 3,2426 (1,41); 3,0372 (0,33); 2,8752 (1,34); 2,8491 (2,27); 2,817 (1,23); 2,6737 (0,74); 2,6693 (1,21); 2,6648 (0,7); 2,539 (1,68); 2,5087 (48,88); 2,5045 (86,78); 2,5 (110,03); 2,4957 (76,72); 2,3313 (0,55); 2,3268 (0,72); 2,3222 (0,49); 2,1375 (1,59); 2,1049 (3,18); 2,0691 (2,49); 1,9868 (9,24); 1,908 (0,66); 1,8444 (0,72); 1,8229 (1,38); 1,8154 (1,43); 1,792 (1,34); 1,7626 (0,56); 1,6213 (0,6); 1,6144 (0,71); 1,5938 (1,36); 1,5841 (1,46); 1,563 (1,36); 1,5542 (1,28); 1,534 (0,6); 1,2366 (0,62); 1,1927 (2,59); 1,1749 (5,02); 1,1571 (2,49); -0,0002 (5,18) |
| **Bsp. I-23, Solvent: DMSO-d₆** |
| 10,0348 (11,5); 9,9019 (0,99); 8,6468 (0,91); 8,0407 (12,2); 7,9296 (4,81); 7,9028 (1,89); 7,8996 (2,76); 7,896 (1,64); 7,884 (2,18); 7,8807 (3,27); 7,8772 (1,89); 7,748 (2,07); 7,7287 (3,1); 7,6642 (3); 7,6453 (4,38); 7,6263 (1,82); 7,3069 (2,13); 7,1736 (4,82); 7,1587 (2,52); 7,0403 (2,41); 7,0227 (5,6); 6,9 (5,04); 6,8868 (2,92); 5,8986 (1,71); 5,8791 (2,02); 5,8715 (1,99); 5,8517 (1,78); 5,4502 (0,99); 5,408 (3,84); 5,3905 (0,39); 5,3673 (3,62); 5,3257 (0,94); 4,3619 (1,13); 4,3302 (1,22); 4,0571 (1,18); 4,0393 (3,62); 4,0215 (3,67); 4,0038 (1,43); 3,9836 (2,98); 3,956 (3,14); 3,9405 (3,28); 3,913 (2,22); 3,4478 (2,55); 3,4282 (2,8); 3,4158 (1,2); 3,4046 (2,91); 3,3965 (1,55); 3,3854 (3,77); 3,3778 (1,74); 3,3681 (1,5); 3,3583 (2,17); 3,3118 (463,38); 3,2415 (1,71); 3,1651 (0,35); 2,8729 (0,84); 2,8451 (1,48); 2,8132 (0,86); 2,6744 (0,56); 2,6698 (0,75); 2,6651 (0,54); 2,5399 (1,48); 2,5229 (3,26); 2,5096 (43,45); 2,5053 (80,56); 2,5008 (105,01); 2,4964 (74,11); 2,4921 (36,83); 2,3322 (0,66); 2,3276 (0,82); 2,3231 (0,6); 2,1344 (1,13); 2,1019 (2,17); 2,0695 (2,7); 1,987 (16); 1,8408 (0,54); 1,8113 (1); 1,7883 (0,92); 1,7607 (0,41); 1,7493 (0,34); 1,6199 (0,42); 1,6115 (0,54); 1,5916 (0,94); 1,582 (1,03); 1,5595 (0,93); 1,5512 (0,88); 1,5303 (0,4); 1,5208 (0,37); 1,1929 (4,42); 1,1751 (8,81); 1,1573 (4,38); -0,0002 (3,62) |
| **Bsp. I-24, Solvent: DMSO-d₆** |
| 7,9871 (15,6); 7,3167 (1,71); 7,3126 (2,43); 7,3058 (3,38); 7,2929 (8,88); 7,2741 (7,96); 7,1725 (6,27); 7,1576 (3,14); 7,0391 (3,35); 7,0217 (9,55); 7,0049 (4,02); 6,9437 (2,71); 6,9251 (4,79); 6,8992 (6,78); 6,8856 (3,65); 5,8162 (2,25); 5,7963 (2,72); 5,7877 (2,66); 5,7676 (2,35); 5,4527 (1,31); 5,4103 (4,62); 5,3648 (4,6); 5,3221 (1,31); 4,3603 (1,48); 4,3274 (1,53); 4,0577 (1,28); 4,04 (3,76); 4,0222 (3,87); 4,0044 (1,53); 3,9836 (1,42); 3,9495 (1,52); 3,8159 (2,63); 3,7978 (6,32); 3,7859 (7,44); 3,7735 (4,14); 3,7589 (0,74); 3,7447 (2,63); 3,4189 (0,72); 3,4098 (1,27); 3,3928 (4,03); 3,3813 (2,86); 3,3729 (4,36); 3,3622 (1,64); 3,3505 (4,06); 3,3424 (2,18); 3,33 (5,88); 3,3135 (263,23); 3,2723 (2,56); 3,2422 (1,28); 2,875 (1,04); 2,846 (1,83); 2,8169 (1,04); 2,5099 (12,34); 2,5057 (22,74); 2,5012 (29,39); 2,4969 (20,73); 2,4927 (10,24); 2,1255 (1,36); 2,0925 (2,77); 2,0697 (1,29); 2,0584 (1,57); 1,9873 (16); 1,8358 (0,72); 1,8063 (1,55); 1,7849 (2,84); 1,7543 (2,27); 1,7232 (1,96); 1,6931 (1,74); 1,6095 (4,67); 1,5851 (3,35); 1,5554 (2,26); 1,5252 (0,62); 1,3974 (9,05); 1,1933 (4,55); 1,1755 (8,83); 1,1577 (4,64); 1,1322 (1,11); 1,1016 (2,23); 1,0756 (5,25); 1,0597 (4,86); 1,0278 (2,56); 1,0205 (2,72); 0,9908 (1,63); 0,9689 (0,46); 0,9607 (0,46); -0,0002 (0,72) |
| **Bsp. I-25, Solvent: DMSO-d₆** |
| 18,8254 (0,66); 18,584 (0,69); 16,784 (0,68); 14,5953 (0,66); 13,1838 (0,64); 12,04 (0,63); 8,0258 (0,91); 8,007 (10,22); 7,3381 (1,45); 7,3338 (1,18); 7,3145 (5,76); 7,3028 (2,82); 7,2953 (5,13); 7,1697 (4,63); 7,1561 (2,21); 7,1342 (2,74); 7,1143 (2,48); 7,0359 (2,08); 7,0204 (4,96); 7,0039 (2,08); 6,9865 (2,96); 6,9669 (1,89); 6,8968 (4,55); 6,8846 (2,48); 5,8754 (1,85); 5,8574 (1,62); 5,8472 (1,87); 5,8309 (1,76); 5,4464 (1,25); 5,4029 (3,77); 5,3627 (3,42); 5,3494 (0,86); 5,3178 (1,35); 5,0603 (0,66); 4,8497 (0,85); 4,8244 (7,6); 4,8186 (4,48); 4,5358 (0,68); 4,4958 (0,67); 4,4923 (0,77); 4,4719 (0,65); 4,3876 (0,7); 4,3572 (1,55); 4,3262 (1,69); 4,2932 (0,75); 4,2478 (0,68); 4,1998 (0,66); 4,1293 (0,86); 4,1155 (0,69); 4,0578 (0,98); 4,0393 (1,27); 4,0211 (1,5); 3,9724 (1,72); 3,9609 (1,59); 3,9432 (2,01); 3,8852 (2,18); 3,8568 (2,79); 3,8418 (2,82); 3,8355 (1,21); 3,8138 (2,72); 3,8021 (1,23); 3,7875 (1,24); 3,7714 (1,34); 3,7587 (1,21); 3,7525 (1,26); 3,742 (1,25); 3,7382 (1,11); 3,724 (1,34); 3,7016 (1,31); 3,6484 (1,54); 3,6357 (1,73); 3,5352 (2,74); 3,4997 (3,05); 3,491 (3,38); 3,3112 (8889,96); 3,2453 (6,66); 3,2272 (4,55); 3,1928 (1,43); 3,1604 (0,94); 3,1517 (0,71); 3,1382 (0,84); 2,9122 (0,75); 2,8934 (0,67); 2,8612 (1,1); 2,8509 (1,04); 2,8326 (1,86); 2,8017 (1,35); 2,7871 (0,76); 2,7498 (1,06); 2,7227 (1,1); 2,6948 (2,09); 2,6738 (7,06); 2,6694 (8,98); 2,6648 (6,83); 2,643 (1,9); 2,5394 (19,3); 2,5091 (540,65); 2,5048 (961,58); 2,5003 (1221,22); 2,496 (846,06); 2,4916 (407,46); 2,356 (0,67); 2,3316 (5,95); 2,3271 (8,06); 2,3226 (5,63); 2,2293 (1,48); 2,2095 (4,15); 2,2045 (2,56); 2,1911 (3,93); 2,178 (1,22); 2,172 (1,42); 2,1328 (1,02); 2,1278 (1,22); 2,0901 (2,25); 2,069 (6,74); 2,0555 (1,26); 1,9866 (3,01); 1,8043 (1,08); 1,7803 (0,92); 1,5694 (1,02); 1,5662 (0,79); 1,5526 (1,06); 1,3985 (14,05); 1,2973 (0,72); 1,259 (1,04); 1,2367 (2,77); 1,1927 (1,09); 1,1749 (2,08); 1,1572 (1,15); 1,0542 (8,06); 1,0356 (16); 1,0168 (7,48); 0,9469 (0,73); 0,89 (0,75); 0,1461 (0,63); 0,0379 (0,8); 0,008 (6,36); -0,0002 (113,74); -0,0085 (4,98); -0,0292 (0,66); -2,0597 (0,67) |
| **Bsp. I-27, Solvent: DMSO-d₆** |
| 8,4981 (3,72); 8,4875 (3,58); 8,0129 (16); 7,4941 (1,02); 7,4744 (3,45); 7,4711 (3,81); 7,4585 (10,06); 7,4554 (9,77); 7,4388 (5,13); 7,4208 (6); 7,3851 (3,4); 7,3799 (2,95); 7,3689 (2,71); 7,3656 (2,94); 7,3604 (1,95); 7,3508 (1,33); 7,3451 (1,27); 7,3055 (2,93); 7,1721 (6,57); 7,1578 (3,56); 7,039 (3,29); 7,0219 (7,47); 6,8975 (6,96); 6,886 (3,78); 6,002 (2,73); 5,9834 (3,3); 5,9746 (3,2); 5,9557 (2,71); 5,7468 (7,45); 5,4471 (1,31); 5,4042 (5,11); 5,366 (5,11); 5,3238 (1,29); 4,3531 (1,67); 4,3222 (1,77); 4,0389 (0,69); 4,021 (0,68); 3,9765 (1,68); 3,9408 (4,14); 3,9128 (3,2); 3,8967 (3,34); 3,8695 (2,77); 3,7691 (0,34); 3,7614 (0,32); 3,6672 (0,39); 3,6627 (0,33); 3,6586 (0,36); 3,6261 (0,34); 3,6154 (0,36); 3,5911 (0,42); 3,5384 (0,54); 3,5283 (0,57); 3,5165 (0,61); 3,5024 (0,64); 3,4819 (0,77); 3,405 (2,62); 3,3969 (2,62); 3,377 (4,86); 3,3672 (4,57); 3,3061 (1839,02); 3,2826 (19,62); 3,257 (5,23); 3,2318 (4,35); 3,213 (3,12); 3,1892 (0,36); 2,8688 (1,52); 2,859 (1,85); 2,8485 (3,18); 2,8406 (4,71); 2,8307 (3,61); 2,822 (2,42); 2,8122 (2,55); 2,6736 (1,25); 2,6693 (1,66); 2,6646 (1,26); 2,6474 (0,36); 2,6296 (0,41); 2,6127 (0,49); 2,5391 (2,86); 2,5087 (94,6); 2,5045 (172,38); 2,5001 (221,82); 2,4957 (155,71); 2,4914 (76,1); 2,4284 (0,34); 2,4204 (0,33); 2,3269 (1,41); 2,3222 (1,02); 2,1264 (1,41); 2,0946 (2,99); 2,0695 (1,74); 2,0607 (1,74); 1,9867 (2,17); 1,8377 (0,63); 1,8099 (1,31); 1,7842 (1,25); 1,7573 (0,61); 1,6026 (0,73); 1,58 (1,31); 1,5735 (1,33); 1,5506 (1,34); 1,5411 (1,24); 1,5203 (0,57); 1,2354 (0,95); 1,1926 (0,65); 1,1749 (1,1); 1,1573 (0,61); 0,8903 (0,39); 0,8804 (0,54); 0,7214 (1,61); 0,7075 (4,67); 0,7033 (6,63); 0,6913 (5,83); 0,6851 (5,31); 0,674 (2,23); 0,6533 (0,45); 0,6338 (0,7); 0,6234 (0,45); 0,5933 (2,69); 0,5829 (7,2); 0,5754 (6,42); 0,5664 (4,87); 0,5552 (1,83); 0,0664 (0,33); 0,0079 (0,82); - 0,0002 (13,42) |
| **Bsp. I-28, Solvent: DMSO-d₆** |
| 9,9013 (0,53); 8,6464 (0,53); 8,0179 (15,28); 7,3196 (2,72); 7,3066 (3,18); 7,299 (5,35); 7,2797 (2,76); 7,1734 (6,02); 7,1587 (2,95); 7,0402 (3,1); 7,0227 (6,79); 6,9616 (3,89); 6,9443 (8,41); 6,9407 (8,6); 6,919 (3,44); 6,913 (2,72); 6,8993 (8,65); 6,8904 (3,05); 6,8869 (3,66); 5,7459 (1,42); 5,7185 (2,22); 5,6983 (2,65); 5,6917 (2,59); 5,6714 (2,26); 5,4494 (1,23); 5,4075 (4,65); 5,367 (4,47); 5,3239 (1,16); 4,3609 (1,49); 4,3283 (1,5); 4,1321 (0,32); 4,105 (5,25); 4,0938 (5,94); 4,0896 (5); 4,0819 (5,91); 4,057 (1,44); 4,0392 (3,89); 4,0214 (3,85); 4,0036 (1,51); 3,9823 (1,43); 3,9484 (1,53); 3,8922 (2,43); 3,8649 (2,72); 3,8492 (3,13); 3,8221 (2,69); 3,6592 (6,77); 3,6515 (5,61); 3,6476 (6,96); 3,6443 (5,51); 3,6362 (6); 3,5682 (0,37); 3,4945 (0,54); 3,4891 (0,54); 3,4738 (0,81); 3,454 (0,72); 3,421 (1,55); 3,4132 (2,22); 3,3957 (5,44); 3,3848 (4,28); 3,3756 (6,39); 3,3529 (8,44); 3,3143 (1657,1); 3,2997 (69,45); 3,2708 (3,69); 3,2406 (1,59); 3,1928 (0,32); 3,1214 (0,34); 2,872 (0,94); 2,844 (1,7); 2,8143 (1); 2,6743 (0,71); 2,6698 (0,93); 2,6653 (0,73); 2,5657 (1,67); 2,5395 (1,44); 2,5095 (56,57); 2,5052 (103,94); 2,5007 (134,27); 2,4964 (93,13); 2,492 (44,99); 2,4479 (0,38); 2,3322 (0,71); 2,3276 (0,85); 2,3227 (0,68); 2,1347 (1,33); 2,1026 (2,58); 2,069 (2,13); 1,9868 (16); 1,9081 (0,57); 1,8414 (0,58); 1,8114 (1,2); 1,7888 (1,09); 1,7601 (0,49); 1,6225 (0,56); 1,6109 (0,66); 1,5902 (1,12); 1,5809 (1,22); 1,5612 (1,08); 1,552 (1,02); 1,53 (0,49); 1,5202 (0,42); 1,3983 (0,86); 1,2375 (0,41); 1,1928 (4,44); 1,175 (8,82); 1,1572 (4,31); -0,0002 (1,96) |
| **Bsp. I-29, Solvent: DMSO-d₆** |
| 7,9726 (11,82); 7,4193 (1,12); 7,3983 (2,39); 7,3812 (2,34); 7,3608 (1,18); 7,3089 (2,02); 7,1757 (4,66); 7,1592 (2,23); 7,0424 (2,27); 7,0231 (5,17); 6,9821 (3,52); 6,961 (3,13); 6,8999 (4,73); 6,8875 (3,85); 6,8642 (2,22); 6,8429 (1,67); 6,0641 (1,51); 6,0423 (1,98); 6,0345 (1,89); 6,0117 (1,64); 5,4562 (0,97); 5,413 (3,42); 5,3697 (3,39); 5,3271 (1); 4,8073 (0,37); 4,7737 (5,62); 4,768 (5,6); 4,3713 (1,13); 4,3692 (1,17); 4,3406 (1,14); 3,9911 (1,17); 3,9584 (1,19); 3,7947 (1,21); 3,7659 (1,47); 3,7544 (1,85); 3,7222 (1,59); 3,6983 (0,36); 3,6678 (0,43); 3,6525 (0,39); 3,5964 (0,45); 3,5693 (0,5); 3,5395 (2,27); 3,5178 (2,32); 3,4968 (1,94); 3,4754 (2,07); 3,4274 (2,13); 3,398 (3,64); 3,3882 (3,45); 3,3797 (3,8); 3,3127 (2744,28); 3,2894 (25,2); 3,257 (2,1); 2,8782 (0,78); 2,8521 (1,41); 2,8219 (0,8); 2,6739 (1,19); 2,6696 (1,62); 2,665 (1,2); 2,6259 (0,33); 2,6072 (0,34); 2,5855 (0,5); 2,5395 (2,35); 2,5093 (97,19); 2,505 (179,71); 2,5006 (232,83); 2,4962 (162,42); 2,4919 (78,86); 2,332 (1,16); 2,3271 (1,52); 2,3222 (1,05); 2,1515 (0,98); 2,1201 (1,96); 2,0786 (1,21); 2,0689 (6,74); 1,8494 (0,51); 1,8217 (0,93); 1,7992 (0,89); 1,759 (8,2); 1,7533 (16); 1,7477 (7,73); 1,6309 (0,41); 1,6235 (0,49); 1,5999 (0,95); 1,592 (0,98); 1,5718 (0,86); 1,5395 (0,39); 1,3983 (0,42); 1,2369 (0,61); -0,0002 (14,45) |
| **Bsp. I-30, Solvent: DMSO-d₆** |
| 7,9681 (16); 7,433 (1,5); 7,416 (1,98); 7,4119 (3,1); 7,3952 (3,13); 7,391 (1,89); 7,3739 (1,51); 7,3085 (2,56); 7,175 (5,8); 7,1585 (2,74); 7,0418 (2,83); 7,0226 (6,92); 7,0121 (4,36); 6,9908 (3,84); 6,9093 (3,01); 6,9008 (5,87); 6,8865 (5,33); 6,8644 (1,87); 6,074 (1,94); 6,0516 (2,36); 6,0436 (2,19); 6,0213 (1,96); 5,4545 (1,14); 5,411 (4,19); 5,369 (4,16); 5,3271 (1,09); 4,887 (0,53); 4,8816 (0,59); 4,8472 (6,66); 4,8417 (10,99); 4,8361 (6,08); 4,802 (0,48); 4,7962 (0,58); 4,3673 (1,31); 4,3338 (1,42); 3,9904 (1,39); 3,9536 (1,47); 3,8311 (0,43); 3,8095 (1,58); 3,7769 (1,77); 3,7681 (2,13); 3,7375 (1,93); 3,5278 (3,35); 3,5207 (4,59); 3,5148 (8,43); 3,5088 (5); 3,4848 (3,01); 3,4631 (3,39); 3,3942 (7,5); 3,3307 (5101,71); 3,3077 (39,28); 3,2505 (2,33); 3,214 (0,93); 3,1513 (0,33); 2,8803 (0,83); 2,8505 (1,57); 2,8219 (0,84); 2,6753 (1,19); 2,6708 (1,61); 2,6661 (1,17); 2,6613 (0,59); 2,5407 (2,04); 2,5107 (94,07); 2,5063 (176,46); 2,5017 (231,85); 2,4973 (160,85); 2,4929 (77,91); 2,3331 (1,24); 2,3283 (1,57); 2,1548 (1,15); 2,1171 (2,28); 2,0796 (1,44); 2,0684 (11,58); 1,8497 (0,53); 1,8211 (1,07); 1,7995 (1,06); 1,6243 (0,49); 1,5944 (1,06); 1,5722 (1,05); 1,5414 (0,42); 1,2375 (0,37); -0,0002 (12,57) |
| **Bsp. I-31, Solvent: DMSO-d₆** |
| 10,2929 (9,8); 8,7763 (4,66); 8,2983 (15,34); 8,0298 (3,08); 8,0268 (3,07); 8,0104 (3,61); 8,0074 (3,55); 7,9472 (2,56); 7,9445 (2,64); 7,9278 (4,4); 7,925 (4,28); 7,8912 (2,38); 7,8878 (2,33); 7,8727 (3,9); 7,8692 (3,55); 7,8536 (1,98); 7,8502 (1,68); 7,7879 (2,19); 7,7685 (3,22); 7,7506 (1,42); 7,4325 (16); 7,3636 (0,36); 7,3559 (0,57); 7,3182 (2,51); 7,3032 (1,2); 7,284 (0,51); 7,1849 (5,51); 7,1696 (3,14); 7,1643 (3,27); 7,157 (1,77); 7,1516 (1,2); 7,1117 (0,6); 7,0519 (2,69); 7,0363 (1,62); 7,0284 (6,38); 7,021 (3,52); 6,9056 (6,47); 6,8924 (4); 6,8853 (1,94); 5,7459 (7,44); 5,4676 (1,16); 5,4256 (4,12); 5,3959 (2,55); 5,3844 (4,4); 5,3658 (1,99); 5,3538 (2,02); 5,3425 (1,57); 5,3066 (1,18); 5,1928 (0,67); 4,5401 (0,32); 4,486 (0,36); 4,3976 (1,37); 4,3587 (1,65); 4,3162 (0,88); 4,2906 (0,34); 4,2601 (0,36); 4,2062 (0,33); 4,1853 (0,33); 4,1801 (0,4); 4,149 (0,45); 4,1315 (1,07); 4,114 (0,98); 4,0994 (0,42); 4,0962 (0,51); 4,0567 (0,9); 4,0391 (2,37); 4,0214 (3,01); 4,0035 (1,62); 3,9809 (1,98); 3,9407 (0,89); 3,8995 (0,43); 3,8305 (0,46); 3,8229 (0,47); 3,8148 (0,51); 3,8009 (0,56); 3,7917 (0,49); 3,7729 (0,5); 3,739 (0,58); 3,7245 (0,59); 3,7066 (0,58); 3,6805 (0,67); 3,6669 (0,75); 3,6348 (0,77); 3,5909 (0,8); 3,5762 (0,89); 3,544 (1,01); 3,5038 (1,37); 3,4839 (2,13); 3,456 (3,53); 3,4474 (3,04); 3,4271 (3,51); 3,4141 (3,55); 3,3145 (4026,52); 3,2023 (0,7); 3,1638 (0,36); 3,037 (2,37); 2,9084 (0,93); 2,8737 (1,7); 2,8499 (3,05); 2,8096 (0,56); 2,6992 (0,37); 2,6746 (1,93); 2,6698 (2,51); 2,6651 (1,88); 2,6417 (0,45); 2,6177 (0,56); 2,6052 (0,65); 2,5399 (3,63); 2,5096 (137,51); 2,5053 (254,27); 2,5008 (329,93); 2,4964 (227,27); 2,492 (107,64); 2,332 (1,47); 2,3275 (2,04); 2,3231 (1,54); 2,1956 (1,21); 2,1611 (2,32); 2,1257 (1,67); 2,102 (1,16); 2,0848 (14,59); 2,0692 (1,6); 2,0476 (0,5); 2,0429 (0,41); 2,0149 (0,32); 1,9868 (8,85); 1,8984 (0,58); 1,8707 (1,17); 1,8491 (1,21); 1,8178 (0,85); 1,7906 (0,67); 1,7861 (0,59); 1,7788 (0,61); 1,7388 (0,38); 1,6831 (0,78); 1,651 (1,48); 1,6215 (1,46); 1,589 (0,85); 1,5803 (0,66); 1,5602 (0,51); 1,5561 (0,55); 1,5531 (0,5); 1,5286 (0,47); 1,525 (0,44); 1,3986 (1,68); 1,2402 (1,4); 1,2221 (2,07); 1,2043 (0,95); 1,1928 (2,53); 1,175 (4,9); 1,1572 (2,49); 1,1471 (0,43); 0,9453 (0,33); -0,0002 (13,04); -0,0086 (0,54) |
| **Bsp. I-32, Solvent: DMSO-d₆** |
| 8,0564 (0,67); 8,0421 (16); 7,4458 (3,11); 7,442 (3,43); 7,4268 (3,82); 7,4227 (4,75); 7,4155 (1,9); 7,4005 (3,86); 7,3961 (3,09); 7,3811 (2,97); 7,3767 (2,33); 7,3042 (5,74); 7,2881 (4,13); 7,2853 (4,14); 7,2692 (1,72); 7,2665 (1,62); 7,202 (0,34); 7,171 (6,68); 7,1569 (3,32); 7,1431 (4,64); 7,1406 (4,46); 7,1231 (4,07); 7,1206 (3,69); 7,0377 (3,28); 7,0209 (7,38); 6,8985 (6,33); 6,885 (3,66); 5,7504 (2,42); 5,7304 (2,9); 5,7216 (2,69); 5,7019 (2,48); 5,4518 (1,33); 5,4089 (4,58); 5,3638 (4,56); 5,3213 (1,3); 4,3853 (0,42); 4,3618 (1,57); 4,3593 (1,58); 4,3289 (1,54); 4,0569 (0,94); 4,0391 (2,62); 4,0214 (2,58); 4,0034 (1,17); 3,9821 (1,51); 3,9509 (1,7); 3,8783 (2,45); 3,8498 (2,86); 3,8355 (3,33); 3,8219 (0,49); 3,8068 (2,77); 3,7302 (0,35); 3,7053 (0,35); 3,6846 (0,39); 3,633 (0,44); 3,6077 (0,5); 3,58 (0,49); 3,5626 (0,57); 3,4258 (2,02); 3,4165 (2,62); 3,3882 (4,64); 3,3779 (4,5); 3,3103 (1658,59); 3,262 (5,58); 3,2419 (4,22); 3,0574 (14,26); 2,8746 (0,98); 2,8433 (1,78); 2,8131 (0,98); 2,6738 (1,19); 2,6694 (1,65); 2,6647 (1,19); 2,6604 (0,69); 2,5863 (1,14); 2,5775 (1,86); 2,5687 (1,53); 2,5587 (2,41); 2,5497 (3,93); 2,5397 (3,62); 2,5092 (90,95); 2,5048 (169,36); 2,5003 (221,44); 2,4959 (153,89); 2,4915 (73,81); 2,3316 (1,05); 2,3268 (1,46); 2,3226 (1,08); 2,1566 (0,5); 2,1534 (0,47); 2,1288 (1,46); 2,0952 (2,79); 2,069 (4,5); 2,0094 (1,48); 1,9867 (12,07); 1,937 (1,48); 1,9072 (1,58); 1,8514 (0,38); 1,8366 (0,59); 1,8075 (1,63); 1,7742 (1,62); 1,691 (2,87); 1,6777 (2,37); 1,6661 (2,03); 1,6382 (1,11); 1,6104 (2,08); 1,6039 (2,28); 1,583 (2,59); 1,553 (1,62); 1,5441 (1,47); 1,5219 (0,68); 1,5096 (1,04); 1,5009 (1,3); 1,4939 (1); 1,4719 (2,77); 1,4428 (2,64); 1,422 (1,04); 1,4121 (1,32); 1,3984 (4,82); 1,3599 (0,49); 1,3522 (0,4); 1,3237 (0,97); 1,3161 (0,74); 1,2936 (1,53); 1,2855 (1,57); 1,2775 (1,47); 1,2539 (2,13); 1,221 (3,2); 1,1927 (6,06); 1,1749 (7,28); 1,1571 (3,48); 1,1511 (0,66); 1,0561 (0,62); 0,8896 (0,33); 0,008 (1,07); -0,0002 (23,89); -0,0084 (1,03) |
| **Bsp. I-33, Solvent: DMSO-d₆** |
| 8,0449 (0,85); 8,0307 (16); 7,4467 (3,19); 7,4426 (3,33); 7,4274 (3,8); 7,4236 (4,09); 7,4145 (1,89); 7,4098 (1,62); 7,3954 (3,73); 7,3906 (2,89); 7,3757 (2,96); 7,3713 (2,2); 7,3109 (3,24); 7,3078 (3,83); 7,2921 (3,92); 7,2892 (3,94); 7,2733 (1,65); 7,2338 (0,34); 7,1715 (10,39); 7,1572 (3,81); 7,1538 (4,36); 7,1511 (3,68); 7,0383 (3,11); 7,0214 (7,15); 6,8995 (6,18); 6,8855 (3,42); 5,7594 (2,31); 5,74 (2,86); 5,7311 (2,73); 5,7112 (2,3); 5,4498 (1,37); 5,4085 (4,59); 5,3638 (4,67); 5,3202 (1,27); 4,7805 (0,32); 4,7391 (0,32); 4,3623 (1,62); 4,3289 (1,63); 4,26 (0,32); 4,2317 (0,37); 4,2084 (0,37); 4,192 (0,35); 4,186 (0,41); 4,1654 (0,39); 4,1349 (0,4); 4,1203 (0,4); 4,0568 (1,39); 4,0392 (3,53); 4,0213 (3,67); 4,0034 (1,64); 3,9822 (1,64); 3,9482 (1,7); 3,8942 (2,81); 3,8653 (3,21); 3,8509 (3,36); 3,8224 (2,98); 3,7494 (0,57); 3,6821 (0,69); 3,6683 (0,77); 3,6417 (0,8); 3,6015 (1,59); 3,5876 (1,16); 3,5664 (1,08); 3,5378 (1,18); 3,5131 (1,33); 3,4823 (1,59); 3,4764 (1,78); 3,3833 (8,42); 3,3191 (4565,63); 3,2922 (16,26); 3,272 (8,14); 3,2484 (4,61); 3,2289 (3,64); 3,1704 (0,37); 3,0574 (3,22); 2,8707 (1,03); 2,8433 (1,69); 2,8113 (1,01); 2,6746 (1,55); 2,6701 (2,23); 2,6655 (1,58); 2,6609 (0,87); 2,54 (3,1); 2,5098 (135,46); 2,5054 (250,35); 2,501 (325,5); 2,4965 (224,28); 2,4921 (106,69); 2,3322 (1,5); 2,3275 (2,16); 2,3234 (1,54); 2,1319 (1,58); 2,0953 (2,73); 2,0687 (6,45); 1,9868 (14,8); 1,9083 (0,38); 1,8733 (0,76); 1,8565 (1,63); 1,8414 (2,92); 1,8356 (1,89); 1,8307 (2,09); 1,823 (1,8); 1,8106 (1,92); 1,7703 (1,41); 1,7605 (1,37); 1,7529 (0,81); 1,7439 (0,83); 1,601 (0,65); 1,5799 (1,23); 1,5733 (1,29); 1,5522 (1,15); 1,5199 (0,57); 1,3984 (5,98); 1,3499 (0,37); 1,2364 (1,21); 1,1928 (4,43); 1,175 (8,37); 1,1572 (4,29); 1,1241 (0,46); 1,107 (0,42); 1,0735 (1,13); 1,0654 (1,2); 1,0527 (2,77); 1,0472 (3,27); 1,0319 (5,75); 1,021 (4,22); 1,0149 (4,83); 1,0087 (4,54); 1,0042 (2,9); 0,998 (2,4); 0,9888 (2,88); 0,9834 (2,07); 0,9757 (2,28); 0,964 (2,97); 0,95 (1,67); 0,9445 (1,47); 0,9312 (0,76); 0,8904 (0,66); 0,8346 (0,33); 0,7914 (0,38); 0,7855 (0,37); 0,768 (0,32); 0,008 (1,2); -0,0002 (26,72); -0,0085 (1,09) |
| **Bsp. I-34. Solvent: DMSO-d₆** |
| 8,7767 (1,01); 8,0195 (10,27); 7,3364 (2,01); 7,3283 (0,48); 7,3162 (4,18); 7,306 (2,23); 7,2959 (2,57); 7,1727 (4,18); 7,1582 (2,25); 7,0395 (2,09); 7,0222 (4,95); 6,9869 (3,67); 6,9805 (3,48); 6,9752 (3,68); 6,9439 (1,88); 6,9421 (1,89); 6,9378 (1,57); 6,9234 (1,72); 6,9214 (1,68); 6,9173 (1,56); 6,9152 (1,42); 6,8992 (4,59); 6,8863 (2,54); 5,7461 (6,78); 5,7267 (1,38); 5,7067 (1,64); 5,6996 (1,6); 5,6795 (1,4); 5,4492 (0,78); 5,4067 (3,05); 5,3663 (3,24); 5,3236 (0,83); 4,7544 (4,46); 4,7491 (8,07); 4,7438 (4,45); 4,3597 (0,92); 4,3273 (1); 3,9814 (0,9); 3,9469 (0,99); 3,9041 (1,5); 3,8767 (1,71); 3,861 (1,94); 3,8337 (1,62); 3,4219 (0,62); 3,4121 (0,92); 3,4018 (0,85); 3,3904 (2,75); 3,3835 (1,97); 3,3702 (2,79); 3,3542 (2,09); 3,3471 (3,57); 3,3113 (921,47); 3,2763 (3,03); 3,2402 (1,32); 2,8904 (0,52); 2,8715 (0,76); 2,8421 (1,27); 2,8138 (0,71); 2,7311 (0,35); 2,6741 (0,56); 2,6696 (0,75); 2,665 (0,56); 2,6541 (0,51); 2,5697 (0,39); 2,5396 (0,86); 2,5227 (2,51); 2,5094 (40,17); 2,505 (76,36); 2,5005 (101,04); 2,4961 (70,54); 2,4917 (34,3); 2,3362 (0,35); 2,3319 (0,57); 2,3273 (0,77); 2,3226 (0,56); 2,22 (0,79); 2,2148 (1,38); 2,2094 (0,92); 2,2013 (1,96); 2,196 (3,73); 2,1907 (2,01); 2,1825 (2,06); 2,1773 (3,84); 2,172 (1,98); 2,164 (0,93); 2,1586 (1,52); 2,1533 (0,96); 2,1354 (1,27); 2,0999 (1,92); 2,069 (1,62); 1,9868 (1); 1,8389 (0,44); 1,8096 (0,86); 1,7859 (0,8); 1,7782 (0,76); 1,7567 (0,38); 1,6199 (0,38); 1,6092 (0,46); 1,588 (0,8); 1,5795 (0,88); 1,5576 (0,82); 1,5498 (0,8); 1,5292 (0,39); 1,439 (0,7); 1,4171 (0,63); 1,3982 (9,94); 1,3845 (0,84); 1,3525 (0,44); 1,2989 (0,35); 1,2588 (0,52); 1,2359 (0,97); 1,1928 (0,36); 1,175 (0,61); 1,1572 (0,34); 1,0618 (0,37); 1,0419 (7,86); 1,0232 (16); 1,0045 (7,35); 0,9857 (0,4); -0,0002 (5,44) |
| **Bsp. I-35, Solvent: DMSO-d₆** |
| 8,0082 (11,59); 7,3952 (0,36); 7,3372 (1,11); 7,3331 (1,49); 7,3138 (6,53); 7,3043 (2,48); 7,2949 (6,01); 7,2628 (0,73); 7,2251 (0,37); 7,1709 (4,28); 7,157 (2,11); 7,1289 (3,52); 7,1089 (2,94); 7,09 (0,84); 7,0377 (2,14); 7,021 (4,87); 7,0024 (1,97); 6,985 (3,29); 6,9666 (1,54); 6,9549 (0,45); 6,8983 (4,09); 6,8851 (2,38); 5,9134 (0,58); 5,9074 (0,64); 5,8763 (1,66); 5,8583 (2,03); 5,8485 (2,25); 5,8304 (1,74); 5,7452 (3,89); 5,4469 (0,79); 5,4047 (3,02); 5,3634 (2,99); 5,321 (0,78); 4,8129 (6,5); 4,807 (6,58); 4,3596 (0,94); 4,3268 (0,95); 3,9783 (0,88); 3,9439 (0,93); 3,8847 (1,63); 3,8568 (1,89); 3,8418 (2,12); 3,814 (1,78); 3,4127 (0,72); 3,4036 (1,12); 3,3947 (1,14); 3,3846 (1,56); 3,3752 (2,37); 3,3656 (2,18); 3,3185 (1355,83); 3,2952 (14,91); 3,2803 (4,54); 3,2621 (4,23); 3,2372 (2,87); 3,2192 (2,45); 2,8632 (0,68); 2,8339 (1,16); 2,8055 (0,69); 2,6793 (0,39); 2,6747 (0,72); 2,6701 (0,98); 2,6657 (0,73); 2,6608 (0,38); 2,5401 (1,07); 2,5233 (3,57); 2,5099 (54,86); 2,5055 (103,8); 2,501 (136,66); 2,4966 (94,58); 2,4922 (45,14); 2,3369 (0,42); 2,3324 (0,74); 2,3277 (0,99); 2,3232 (0,73); 2,3185 (0,39); 2,1279 (0,99); 2,094 (1,88); 2,0688 (2,48); 2,0602 (1,07); 1,9869 (0,33); 1,8234 (7,69); 1,8177 (16); 1,812 (8,61); 1,7817 (0,84); 1,7746 (0,79); 1,7526 (0,37); 1,6799 (0,38); 1,6512 (0,33); 1,6118 (0,38); 1,6008 (0,41); 1,5806 (0,76); 1,5708 (0,8); 1,5502 (0,75); 1,541 (0,71); 1,5201 (0,34); 1,3524 (0,58); 1,2985 (0,97); 1,259 (1,38); 1,2362 (1,58); 0,0079 (0,45); -0,0002 (10,51); -0,0085 (0,43) |
| **Bsp. I-36, Solvent: DMSO-d₆** |
| 8,0517 (0,55); 8,0421 (9,39); 7,487 (1,77); 7,483 (2,13); 7,468 (2,33); 7,4639 (2,89); 7,4565 (1,15); 7,4416 (2,4); 7,437 (1,9); 7,422 (1,97); 7,4176 (1,52); 7,3704 (1,79); 7,3678 (1,9); 7,3517 (2,43); 7,3489 (2,47); 7,3327 (1,03); 7,3302 (0,94); 7,3034 (1,8); 7,2377 (2,9); 7,2349 (2,76); 7,2177 (2,46); 7,215 (2,29); 7,17 (4,06); 7,157 (2,01); 7,0368 (2,04); 7,021 (4,53); 6,8989 (4,2); 6,8851 (2,3); 5,8325 (1,5); 5,8127 (1,73); 5,8042 (1,7); 5,7843 (1,5); 5,4469 (0,77); 5,4051 (3,05); 5,3656 (3); 5,3234 (0,79); 4,3588 (0,94); 4,3279 (1); 4,0806 (1,41); 4,0539 (4,47); 4,0391 (4,17); 4,0269 (4,73); 4,0214 (4,41); 4,0033 (1,92); 4,0002 (1,89); 3,9825 (0,99); 3,9466 (1,04); 3,9162 (1,61); 3,8879 (1,82); 3,873 (1,94); 3,8448 (1,68); 3,4712 (0,35); 3,413 (1,18); 3,4024 (1,14); 3,3936 (1,6); 3,3845 (2,21); 3,3755 (1,94); 3,355 (2,63); 3,3075 (760,65); 3,261 (3,18); 3,2408 (2,56); 3,2176 (1,78); 3,1978 (1,63); 2,8708 (0,64); 2,8411 (1,16); 2,8112 (0,64); 2,6738 (0,6); 2,6691 (0,78); 2,6648 (0,58); 2,5392 (1,14); 2,509 (44,2); 2,5047 (81,04); 2,5003 (104,48); 2,4959 (71,67); 2,4915 (33,77); 2,3317 (0,47); 2,3269 (0,69); 2,3225 (0,49); 2,1308 (0,83); 2,0988 (1,7); 2,0691 (1,76); 1,9867 (16); 1,84 (0,33); 1,8353 (0,35); 1,8234 (0,38); 1,8038 (0,77); 1,7819 (0,73); 1,6165 (0,51); 1,5845 (0,78); 1,5529 (0,73); 1,5438 (0,7); 1,5234 (0,35); 1,3983 (4,85); 1,2592 (0,37); 1,236 (0,78); 1,1927 (4,49); 1,1749 (8,81); 1,1571 (4,35); 0,0079 (1,6); -0,0002 (33,58); -0,0085 (1,31) |
| **Bsp. I-37, Solvent: DMSO-d₆** |
| 9,286 (0,77); 8,0342 (0,41); 8,0227 (6,59); 7,4384 (1,2); 7,4218 (1,56); 7,4179 (1,38); 7,4022 (0,48); 7,3983 (0,44); 7,378 (1,63); 7,3675 (2,98); 7,3618 (4,85); 7,359 (4,72); 7,3489 (1,31); 7,3418 (1,25); 7,3362 (1); 7,326 (0,42); 7,3199 (0,42); 7,3059 (1,22); 7,1727 (2,75); 7,1577 (1,34); 7,0393 (1,33); 7,0217 (3,07); 6,8983 (2,75); 6,8858 (1,55); 6,1213 (1); 6,1009 (1,24); 6,0939 (1,18); 6,0731 (1,03); 5,7462 (4,79); 5,4499 (0,57); 5,4078 (2,02); 5,3661 (2,03); 5,3244 (0,54); 4,3621 (0,65); 4,3289 (0,68); 4,057 (0,43); 4,0391 (1,24); 4,0214 (1,28); 4,0035 (0,54); 3,98 (0,7); 3,9542 (1,5); 3,9265 (1,25); 3,9104 (1,33); 3,8829 (1,06); 3,4406 (0,38); 3,4149 (0,94); 3,4044 (0,87); 3,3859 (1,6); 3,3769 (1,44); 3,3562 (2,16); 3,3105 (820,01); 3,2875 (8,8); 3,2661 (2,52); 3,2457 (1,52); 3,2383 (0,81); 3,0528 (0,75); 3,0357 (16); 2,8685 (0,47); 2,8395 (0,8); 2,8139 (0,48); 2,6741 (0,63); 2,6694 (0,85); 2,6651 (0,6); 2,5396 (1,07); 2,5225 (3,59); 2,5092 (46,39); 2,5049 (85,81); 2,5004 (111,31); 2,4961 (77,41); 2,4918 (37,22); 2,3316 (0,51); 2,3272 (0,72); 2,3226 (0,53); 2,1304 (0,57); 2,101 (1,15); 2,069 (1,34); 1,9867 (5,13); 1,8115 (0,51); 1,7875 (0,47); 1,5885 (0,52); 1,5789 (0,52); 1,5569 (0,5); 1,549 (0,47); 1,1927 (1,42); 1,1749 (2,77); 1,1571 (1,36); -0,0002 (3,8) |
| **Bsp. I-38, Solvent: DMSO-d₆** |
| 8,045 (16); 7,7484 (0,42); 7,5602 (3,52); 7,5418 (4,61); 7,478 (0,38); 7,4714 (0,79); 7,4685 (0,79); 7,4518 (3,13); 7,4487 (3,22); 7,4363 (8); 7,4329 (10,02); 7,4188 (1,4); 7,3968 (0,35); 7,3804 (2,83); 7,3742 (2,42); 7,3615 (2,72); 7,3587 (2,27); 7,355 (2,18); 7,346 (1,48); 7,3395 (1,46); 7,3056 (2,68); 7,1723 (6,13); 7,158 (3,08); 7,039 (3,04); 7,0219 (7,05); 6,9004 (6,15); 6,8861 (3,37); 6,0236 (2,56); 6,0054 (3); 5,9958 (2,9); 5,9775 (2,56); 5,7443 (11,99); 5,4496 (1,18); 5,4071 (4,43); 5,3648 (4,59); 5,3223 (1,19); 4,542 (13,91); 4,522 (0,34); 4,3622 (1,38); 4,3285 (1,5); 4,0575 (0,54); 4,0398 (1,48); 4,0219 (1,67); 4,0127 (2,68); 4,0042 (0,87); 3,9848 (4); 3,9696 (4,08); 3,9418 (3,99); 3,8728 (0,33); 3,5684 (1,48); 3,4573 (0,73); 3,4092 (2,36); 3,3992 (2,48); 3,3798 (5,27); 3,3333 (1502,56); 3,3141 (12,84); 3,3103 (13,57); 3,2958 (5,87); 3,2647 (2,23); 3,2351 (1,18); 2,8911 (0,39); 2,8659 (0,95); 2,8361 (1,67); 2,8077 (0,91); 2,676 (0,65); 2,6715 (0,81); 2,667 (0,59); 2,5415 (1,24); 2,5246 (3,49); 2,5113 (47,16); 2,507 (88,52); 2,5025 (115,82); 2,4981 (80,76); 2,4937 (38,83); 2,3339 (0,6); 2,3291 (0,78); 2,3245 (0,57); 2,1301 (1,22); 2,0971 (2,53); 2,0688 (1,68); 2,0381 (0,44); 1,9873 (6,06); 1,8376 (0,53); 1,817 (1,07); 1,8084 (1,14); 1,7853 (1,06); 1,7774 (1,02); 1,7566 (0,44); 1,6124 (0,49); 1,6027 (0,57); 1,5815 (1,08); 1,573 (1,18); 1,5512 (1,13); 1,5423 (1,05); 1,522 (0,49); 1,2365 (3,92); 1,1933 (1,69); 1,1755 (3,37); 1,1657 (1,98); 1,1577 (1,87); 0,8542 (0,44); -0,0002 (3,1) |
| **Bsp. I-39, Solvent: DMSO-d₆** |
| 8,7771 (2,11); 8,0267 (0,4); 8,0235 (0,36); 8,006 (16); 7,9817 (1,67); 7,5997 (0,93); 7,3469 (1,88); 7,3426 (2,19); 7,3238 (9,44); 7,3048 (11,46); 7,171 (7,57); 7,1571 (4,87); 7,1515 (4,97); 7,1318 (3,93); 7,0377 (3,99); 7,0213 (11,21); 7,0032 (4,63); 6,9848 (2,64); 6,9642 (0,53); 6,8979 (7,65); 6,8855 (4,6); 5,8875 (2,21); 5,8695 (2,62); 5,8598 (2,63); 5,8418 (2,35); 5,7464 (6,68); 5,4479 (1,38); 5,4053 (5,11); 5,3641 (5,49); 5,3214 (1,43); 4,8769 (12,77); 4,871 (12,92); 4,7991 (1,38); 4,3569 (1,65); 4,3255 (1,77); 4,1206 (0,91); 4,1143 (0,93); 4,039 (0,77); 4,0213 (0,89); 4,0032 (0,45); 3,9751 (1,7); 3,946 (1,94); 3,919 (0,49); 3,8967 (2,45); 3,8688 (2,85); 3,8537 (3,11); 3,8259 (2,56); 3,5666 (3,33); 3,5607 (7,23); 3,5548 (3,28); 3,4323 (0,43); 3,4124 (1,05); 3,4032 (1,62); 3,3932 (1,55); 3,3835 (2,24); 3,3747 (3,41); 3,365 (3,02); 3,3084 (1959,21); 3,2859 (20,62); 3,2716 (9,69); 3,2536 (7,04); 3,2286 (5,39); 3,2105 (4,11); 3,155 (0,95); 3,1487 (1,16); 3,1424 (0,94); 3,1116 (0,56); 3,0552 (0,43); 3,0369 (0,75); 2,9398 (0,48); 2,9243 (0,36); 2,9089 (0,36); 2,8899 (0,59); 2,8646 (1,51); 2,8491 (1,15); 2,8376 (2,33); 2,8047 (1,39); 2,7322 (0,36); 2,695 (0,48); 2,6737 (1,99); 2,6692 (5,8); 2,5654 (0,83); 2,5394 (4,15); 2,5224 (9,75); 2,5091 (122,25); 2,5047 (229,43); 2,5002 (302,8); 2,4958 (216,89); 2,4914 (108,87); 2,4039 (0,74); 2,3316 (1,84); 2,327 (2,36); 2,3225 (1,8); 2,2882 (0,41); 2,2805 (0,44); 2,2698 (0,37); 2,2532 (0,4); 2,2371 (0,36); 2,2001 (0,39); 2,1792 (0,43); 2,1286 (1,81); 2,0942 (3,42); 2,0691 (3,06); 2,0221 (0,37); 2,0091 (0,41); 1,9867 (3,55); 1,9078 (0,34); 1,8345 (0,85); 1,8054 (1,56); 1,7828 (1,53); 1,7525 (0,72); 1,6103 (0,74); 1,5994 (0,81); 1,5803 (1,56); 1,5702 (1,54); 1,5491 (1,56); 1,5415 (1,36); 1,5207 (0,71); 1,4701 (1,14); 1,4062 (2,26); 1,3982 (0,81); 1,3522 (0,8); 1,337 (0,35); 1,2983 (1,38); 1,2844 (0,38); 1,259 (2,18); 1,2363 (3,16); 1,1927 (1,18); 1,1749 (2,11); 1,1571 (1,17); 0,8706 (0,35); 0,8542 (0,53); 0,8374 (0,36); 0,0079 (1,18); -0,0002 (23,73); -0,0084 (1,06) |
| **Bsp. I-40, Solvent: DMSO-d₆** |
| 9,7795 (2,15); 8,0164 (4,15); 7,3719 (1,96); 7,3505 (2,62); 7,3073 (0,7); 7,2321 (2,84); 7,2106 (2,14); 7,1739 (1,6); 7,159 (0,81); 7,0407 (0,8); 7,023 (1,82); 6,8997 (1,61); 6,8871 (0,92); 5,7081 (0,57); 5,687 (0,73); 5,6815 (0,7); 5,6602 (0,59); 5,4515 (0,34); 5,4089 (1,22); 5,3673 (1,2); 4,3633 (0,38); 4,3298 (0,4); 4,0569 (1,23); 4,0391 (3,66); 4,0213 (3,7); 4,0035 (1,32); 3,9825 (0,39); 3,9505 (0,41); 3,8743 (0,6); 3,8471 (0,69); 3,8313 (0,79); 3,8043 (0,67); 3,5676 (0,45); 3,4145 (0,47); 3,387 (1,51); 3,3765 (0,78); 3,3661 (1,4); 3,3576 (1,06); 3,344 (1,94); 3,3089 (349,24); 3,242 (0,49); 2,9856 (11,18); 2,8443 (0,49); 2,674 (0,35); 2,6693 (0,43); 2,6647 (0,34); 2,5393 (0,77); 2,5223 (2,25); 2,509 (25,66); 2,5047 (46,92); 2,5003 (60,77); 2,4959 (42,78); 2,4915 (20,9); 2,3316 (0,33); 2,327 (0,43); 2,1462 (0,33); 2,1378 (0,36); 2,1018 (0,72); 2,0692 (0,49); 1,9867 (16); 1,8234 (0,32); 1,8106 (0,33); 1,5813 (0,33); 1,1927 (4,46); 1,1749 (8,83); 1,1571 (4,3); 0,0079 (0,33); -0,0002 (6,07) |
| **Bsp. I-41, Solvent: DMSO-d₆** |
| 8,7994 (0,47); 8,4174 (0,9); 8,2839 (0,36); 8,2697 (0,35); 8,0449 (0,54); 8,0402 (10,68); 7,2726 (1,64); 7,2052 (0,34); 7,1838 (3,6); 7,1299 (1,86); 7,1248 (0,46); 7,0951 (1,84); 7,0875 (2,13); 7,0852 (2,25); 7,0747 (2,17); 7,0724 (2,24); 7,0499 (0,42); 7,0393 (4,58); 7,0249 (1,31); 7,0223 (1,23); 7,0116 (2,05); 7,0103 (1,98); 6,9995 (1,42); 6,9969 (1,33); 6,9488 (2,1); 6,9259 (0,47); 6,9138 (4,9); 6,6961 (2,67); 6,6945 (2,7); 6,6828 (2,55); 6,6811 (2,46); 6,5725 (1,38); 6,5709 (1,35); 6,5601 (2,51); 6,5586 (2,39); 6,5478 (1,31); 6,5461 (1,24); 5,7809 (1,45); 5,7643 (16); 5,7492 (1,47); 5,4542 (1,24); 5,4257 (2,63); 5,4082 (0,39); 5,3775 (3,26); 5,349 (1,46); 5,0277 (5,17); 4,357 (0,98); 4,3351 (1,06); 3,9735 (0,97); 3,9504 (1,06); 3,8413 (1,49); 3,8232 (1,73); 3,8129 (1,83); 3,7948 (1,55); 3,4055 (0,41); 3,3991 (0,75); 3,3928 (0,53); 3,3862 (0,85); 3,3799 (1,46); 3,3737 (0,93); 3,3672 (0,66); 3,3607 (0,98); 3,3462 (82,73); 3,3226 (1,35); 3,2793 (0,79); 3,2593 (1,37); 3,2524 (2,16); 3,2375 (2,58); 3,23 (0,42); 3,2239 (1,86); 3,2091 (1,7); 3,1955 (1,72); 2,8489 (0,87); 2,8292 (1,24); 2,8103 (0,71); 2,6183 (0,45); 2,6153 (0,62); 2,6122 (0,45); 2,565 (0,84); 2,5427 (0,34); 2,5243 (1,2); 2,5212 (1,52); 2,5181 (1,48); 2,5093 (32,91); 2,5063 (73,12); 2,5032 (101,55); 2,5002 (73,05); 2,4972 (32,78); 2,3902 (0,43); 2,3871 (0,59); 2,3841 (0,42); 2,1241 (0,84); 2,1025 (1,06); 2,1 (1,06); 2,0869 (1,05); 2,0652 (1,02); 1,8294 (0,37); 1,8156 (0,74); 1,8095 (0,84); 1,795 (0,81); 1,7891 (0,8); 1,7753 (0,41); 1,7681 (0,38); 1,766 (0,39); 1,5923 (0,32); 1,5855 (0,38); 1,5717 (0,78); 1,5654 (0,86); 1,5513 (0,86); 1,545 (0,85); 1,5314 (0,44); 1,5244 (0,39); 1,4077 (0,38); 1,3509 (0,49); 1,2987 (1,71); 1,258 (2,56); 1,234 (1,6); 0,8535 (0,36); -0,0001 (1,81) |
| **Bsp. I-42, Solvent: DMSO-d₆** |
| 9,4493 (7,94); 8,7772 (0,82); 8,4303 (0,53); 8,0283 (0,8); 8,0179 (7,95); 7,308 (1,64); 7,2965 (0,35); 7,209 (0,44); 7,1937 (1,66); 7,1745 (6,63); 7,1597 (2,59); 7,1549 (2,47); 7,0415 (1,86); 7,0235 (4,22); 7,0145 (0,94); 6,9974 (0,37); 6,9001 (4,83); 6,8877 (2,35); 6,8786 (0,55); 6,7924 (2,09); 6,7697 (3,43); 6,7634 (3,24); 6,7228 (1,76); 6,721 (1,78); 6,7169 (1,49); 6,7027 (1,59); 6,7006 (1,53); 6,6969 (1,39); 5,6685 (1,23); 5,649 (1,52); 5,6413 (1,44); 5,622 (1,29); 5,5927 (0,65); 5,4513 (0,76); 5,409 (2,91); 5,3689 (3,36); 5,3259 (0,87); 4,3621 (0,97); 4,329 (1,06); 4,0571 (1,25); 4,0393 (3,76); 4,0216 (3,82); 4,0037 (1,47); 3,9832 (0,95); 3,9469 (1,09); 3,8812 (1,39); 3,8537 (1,5); 3,8382 (1,7); 3,8108 (1,45); 3,4222 (0,58); 3,4129 (0,84); 3,4038 (0,77); 3,3938 (1,08); 3,3846 (1,54); 3,3758 (1,09); 3,3643 (0,97); 3,3557 (1,28); 3,3355 (3,2); 3,3073 (237,48); 3,2837 (4,75); 3,2733 (3,42); 3,2409 (1,06); 2,874 (0,79); 2,8447 (1,37); 2,8145 (0,77); 2,6694 (0,4); 2,5656 (0,51); 2,5393 (0,89); 2,5087 (24,65); 2,5047 (42,64); 2,5003 (53,05); 2,4961 (36,83); 2,3314 (0,34); 2,3271 (0,4); 2,3225 (0,32); 2,1358 (1,03); 2,1026 (2,05); 2,069 (1,35); 1,9868 (16); 1,8419 (0,45); 1,823 (0,87); 1,8133 (0,91); 1,7906 (0,85); 1,7625 (0,39); 1,6224 (0,38); 1,6116 (0,45); 1,5906 (0,87); 1,5821 (0,91); 1,5603 (0,89); 1,5521 (0,84); 1,5307 (0,43); 1,3979 (0,61); 1,1928 (4,39); 1,175 (8,6); 1,1572 (4,23); -0,0002 (0,79) |
| **Bsp. I-43, Solvent: DMSO-d₆** |
| 8,7778 (0,33); 8,0198 (5,88); 7,9699 (1,46); 7,967 (1,51); 7,9504 (1,6); 7,9475 (1,57); 7,668 (0,58); 7,6648 (0,6); 7,6487 (1,35); 7,6301 (1,08); 7,6269 (1,02); 7,573 (1,76); 7,5557 (1,09); 7,4867 (0,94); 7,4835 (0,88); 7,4677 (1,45); 7,465 (1,36); 7,4491 (0,72); 7,4458 (0,65); 7,3042 (1,08); 7,1709 (2,45); 7,1579 (1,29); 7,0377 (1,24); 7,0219 (2,86); 6,8984 (2,52); 6,8861 (1,46); 6,3307 (0,91); 6,3144 (1,04); 6,3028 (1,02); 6,2866 (0,9); 5,7471 (1,24); 5,4458 (0,47); 5,403 (1,82); 5,3634 (1,83); 5,3207 (0,47); 4,3556 (0,57); 4,3222 (0,64); 4,066 (1,02); 4,0384 (1,29); 4,022 (1,63); 3,9941 (1,12); 3,9744 (0,58); 3,9402 (0,65); 3,896 (0,51); 3,882 (16); 3,8764 (2,11); 3,4031 (0,42); 3,3947 (0,62); 3,3847 (0,54); 3,3754 (0,76); 3,3659 (1,16); 3,3561 (0,96); 3,3096 (206,94); 3,2574 (0,88); 3,2373 (1,34); 3,2211 (1,51); 3,1932 (1,1); 3,1771 (1,05); 2,8592 (0,39); 2,8301 (0,71); 2,8006 (0,42); 2,5099 (10,14); 2,5056 (18,43); 2,5011 (23,66); 2,4968 (16,39); 2,4925 (7,85); 2,1203 (0,51); 2,0857 (1,04); 2,0701 (0,82); 2,0479 (0,61); 1,9873 (1,86); 1,8084 (0,45); 1,7991 (0,46); 1,7779 (0,45); 1,7692 (0,4); 1,573 (0,44); 1,5639 (0,48); 1,5416 (0,45); 1,5338 (0,43); 1,3978 (0,45); 1,1931 (0,5); 1,1754 (0,99); 1,1576 (0,49); -0,0002 (0,69) |
| **Bsp. I-44, Solvent: DMSO-d₆** |
| 8,0475 (5,06); 7,495 (1,24); 7,4826 (1,6); 7,4102 (1,83); 7,4041 (3,54); 7,3971 (0,34); 7,3692 (0,83); 7,3634 (0,77); 7,3604 (0,54); 7,3566 (0,75); 7,3501 (0,61); 7,342 (0,43); 7,2712 (0,78); 7,1826 (1,81); 7,1267 (0,87); 7,094 (0,87); 7,0363 (2,12); 6,9459 (0,98); 6,9113 (2,22); 6,1015 (0,88); 6,0881 (1); 6,083 (0,96); 6,0696 (0,87); 5,7625 (0,41); 5,453 (0,7); 5,4246 (1,58); 5,3741 (1,55); 5,3457 (0,71); 4,9788 (1,52); 4,9595 (2,11); 4,8775 (2,19); 4,8582 (1,56); 4,3593 (0,5); 4,3374 (0,52); 4,024 (0,84); 4,0055 (0,96); 3,9953 (1,04); 3,9768 (1,33); 3,9536 (0,52); 3,5692 (16); 3,405 (0,43); 3,3921 (0,47); 3,386 (0,78); 3,3797 (0,47); 3,3665 (0,43); 3,3179 (0,96); 3,3045 (0,98); 3,2891 (1,01); 3,2846 (0,4); 3,2758 (1,06); 3,2601 (0,69); 3,2413 (0,38); 2,9439 (4,47); 2,8484 (0,35); 2,8279 (0,64); 2,8105 (0,35); 2,7838 (3,59); 2,6147 (0,39); 2,5647 (2,32); 2,524 (0,48); 2,5209 (0,66); 2,5178 (0,79); 2,5088 (22,38); 2,506 (47,3); 2,503 (64,84); 2,5 (47,32); 2,4972 (22,18); 2,3872 (0,4); 2,1253 (0,44); 2,1059 (0,53); 2,0882 (0,49); 2,0771 (0,56); 2,0671 (0,48); 1,958 (3,87); 1,9096 (0,52); 1,8112 (0,39); 1,8049 (0,43); 1,7908 (0,41); 1,7849 (0,38); 1,5726 (0,4); 1,5662 (0,42); 1,5519 (0,43); 1,5458 (0,4); -0,0002 (7,43) |
| **Bsp. I-45, Solvent: DMSO-d₆** |
| 9,8994 (0,63); 8,6648 (0,61); 8,0229 (16); 7,4357 (1,96); 7,4324 (2,4); 7,4236 (1,91); 7,4213 (2,43); 7,3478 (1,87); 7,3445 (1,38); 7,3368 (3,12); 7,3327 (3,24); 7,3236 (0,36); 7,3188 (0,75); 7,3155 (1,2); 7,3066 (3,51); 7,3034 (2,83); 7,2992 (3,06); 7,2958 (5,35); 7,2914 (2,5); 7,2872 (2,31); 7,2844 (1,96); 7,2752 (0,76); 7,272 (0,84); 7,2686 (1,88); 7,18 (4,51); 7,1262 (2,14); 7,0915 (2,15); 7,0358 (5,68); 6,9455 (2,53); 6,9112 (4,84); 5,9981 (1,98); 5,985 (2,2); 5,9797 (2,14); 5,9666 (1,95); 5,4504 (1,65); 5,422 (3,66); 5,3725 (3,49); 5,3442 (1,59); 5,2615 (2,95); 5,2525 (6,4); 5,2434 (3,09); 4,5916 (5,58); 4,5828 (6,19); 4,3573 (1,04); 4,3356 (1,09); 4,0458 (0,36); 4,0339 (1,18); 4,022 (1,16); 4,0102 (0,41); 3,9728 (1,02); 3,9502 (1,11); 3,9242 (2,07); 3,9057 (2,46); 3,8955 (2,62); 3,8771 (2,16); 3,4048 (0,53); 3,399 (0,98); 3,3921 (0,76); 3,3858 (1,32); 3,3796 (2,18); 3,3733 (1,71); 3,3708 (1,53); 3,3455 (1366,81); 3,3281 (1,18); 3,3219 (7,12); 3,2787 (0,87); 3,2676 (2,65); 3,2545 (3,25); 3,2389 (3,08); 3,2259 (2,39); 2,8468 (0,77); 2,8292 (1,33); 2,8093 (0,77); 2,6345 (0,44); 2,6205 (0,48); 2,6175 (1,06); 2,6144 (1,48); 2,6114 (1,06); 2,6083 (0,44); 2,5645 (7,75); 2,5421 (0,9); 2,5237 (2,39); 2,5207 (3,2); 2,5175 (3,58); 2,5088 (77,52); 2,5057 (170,79); 2,5027 (231,96); 2,4996 (165,03); 2,4966 (71,78); 2,3929 (0,47); 2,3899 (1,05); 2,3869 (1,45); 2,3838 (1,01); 2,3806 (0,47); 2,1874 (0,42); 2,1221 (0,94); 2,1026 (1,13); 2,0865 (1,06); 2,0767 (2,1); 2,0628 (1,01); 1,9898 (5,36); 1,9087 (0,57); 1,8306 (0,39); 1,8242 (0,4); 1,811 (0,84); 1,8039 (0,89); 1,7902 (0,88); 1,7837 (0,81); 1,7698 (0,36); 1,5906 (0,36); 1,5839 (0,47); 1,5703 (0,89); 1,5637 (1,01); 1,5495 (0,96); 1,5436 (0,89); 1,5297 (0,41); 1,397 (2,51); 1,3003 (0,45); 1,2339 (0,42); 1,186 (1,58); 1,1742 (3,29); 1,1623 (1,47); -0,0002 (4,77) |
| **Bsp. I-46, Solvent: DMSO-d₆** |
| 8,0198 (0,55); 7,9687 (1,48); 7,9658 (1,51); 7,9492 (1,63); 7,9462 (1,59); 7,6807 (0,58); 7,6774 (0,6); 7,6615 (1,34); 7,6427 (1,06); 7,6394 (0,96); 7,5663 (1,7); 7,5478 (1,16); 7,4963 (0,94); 7,4933 (0,87); 7,4773 (1,47); 7,4746 (1,37); 7,4586 (0,74); 7,4555 (0,65); 7,2868 (1,01); 7,1535 (2,44); 7,1487 (1,38); 7,0205 (1,47); 7,0125 (2,57); 6,8914 (2,33); 6,8765 (1,25); 6,3284 (0,9); 6,3115 (1,04); 6,3002 (1,01); 6,2834 (0,89); 5,7472 (1,05); 5,4238 (0,46); 5,3815 (1,75); 5,3629 (0,38); 5,3439 (1,73); 5,3017 (0,44); 4,3222 (0,6); 4,2884 (0,56); 4,1332 (0,95); 4,1048 (1,14); 4,0886 (1,26); 4,0603 (1,09); 4,0395 (0,55); 4,0216 (0,6); 3,946 (0,59); 3,9122 (0,64); 3,8815 (2,97); 3,8756 (16); 3,364 (0,67); 3,3066 (262,77); 3,2665 (1,62); 3,2497 (1,65); 3,2364 (0,45); 3,2219 (1,75); 3,2053 (1,2); 3,1923 (0,49); 2,8275 (0,43); 2,7989 (0,68); 2,7687 (0,36); 2,5396 (0,5); 2,5094 (17,55); 2,5052 (31,81); 2,5007 (40,84); 2,4963 (28,27); 2,492 (13,58); 2,0699 (0,96); 2,0428 (1,01); 2,0081 (0,55); 1,9871 (2,09); 1,7583 (0,42); 1,7513 (0,45); 1,726 (0,4); 1,5359 (0,46); 1,5279 (0,46); 1,5054 (0,43); 1,4963 (0,38); 1,398 (0,61); 1,1971 (0,4); 1,1931 (0,61); 1,1789 (0,84); 1,1753 (1,24); 1,1575 (0,61); -0,0002 (1,5) |
| **Bsp. I-47, Solvent: DMSO-d₆** |
| 8,0201 (8,85); 7,3382 (4,89); 7,3163 (5,8); 7,1785 (3,4); 7,1663 (1,65); 7,0454 (1,74); 7,0304 (3,94); 6,997 (0,61); 6,99 (6,2); 6,9849 (1,95); 6,9681 (5,65); 6,9068 (3,57); 6,8946 (1,86); 5,6895 (1,29); 5,6676 (1,57); 5,6625 (1,56); 5,6404 (1,34); 5,4585 (0,77); 5,4161 (2,59); 5,3737 (2,58); 5,3312 (0,82); 4,7544 (3,69); 4,7492 (7,22); 4,7439 (3,96); 4,3653 (0,77); 4,333 (0,82); 3,9834 (0,7); 3,9527 (0,77); 3,85 (1,3); 3,8228 (1,57); 3,8068 (1,82); 3,78 (1,47); 3,4279 (0,53); 3,416 (0,87); 3,3975 (0,94); 3,3891 (2,92); 3,3789 (1,33); 3,3676 (2,99); 3,3445 (134,57); 3,3385 (175); 3,3322 (300,5); 3,3307 (334,71); 3,2696 (1,14); 3,2393 (0,64); 2,8687 (0,53); 2,8378 (0,96); 2,8109 (0,55); 2,6752 (0,67); 2,6705 (0,79); 2,6661 (0,62); 2,5411 (0,49); 2,5243 (1,49); 2,506 (90,88); 2,5019 (122,12); 2,3333 (0,65); 2,3287 (0,84); 2,2509 (0,5); 2,2454 (1,08); 2,2402 (0,63); 2,2319 (1,53); 2,2267 (3,22); 2,2215 (1,51); 2,2133 (1,6); 2,2081 (3,44); 2,2028 (1,58); 2,1945 (0,68); 2,1893 (1,18); 2,1843 (0,56); 2,1395 (0,65); 2,1052 (1,39); 2,073 (2,87); 1,9885 (0,63); 1,8206 (0,61); 1,8124 (0,65); 1,7915 (0,6); 1,7816 (0,6); 1,589 (0,61); 1,5782 (0,73); 1,5562 (0,62); 1,5494 (0,63); 1,2354 (0,59); 1,1745 (0,37); 1,0686 (7,63); 1,05 (16); 1,0312 (7,23); -0,0002 (6,53) |
| **Bsp. I-48, Solvent: DMSO-d₆** |
| 16,1708 (0,4); 14,1949 (0,4); 8,0206 (11,08); 7,338 (5,82); 7,3163 (6,87); 7,179 (4,05); 7,1667 (2,13); 7,0617 (0,39); 7,0457 (2,11); 7,0308 (4,58); 6,9876 (7,25); 6,9657 (6,39); 6,9072 (4,18); 6,8949 (2,24); 5,689 (1,51); 5,6685 (1,95); 5,6626 (1,9); 5,6414 (1,54); 5,4595 (1,02); 5,4162 (3,18); 5,3737 (3,01); 5,332 (0,97); 4,7412 (6,34); 4,7355 (6,31); 4,3661 (1,06); 4,3351 (1,05); 3,9863 (1,05); 3,9515 (1,06); 3,8497 (1,47); 3,823 (1,89); 3,8067 (2,08); 3,7799 (1,76); 3,7105 (0,49); 3,5114 (0,41); 3,4946 (0,43); 3,4615 (0,44); 3,4534 (0,43); 3,4442 (0,43); 3,4258 (0,72); 3,4162 (1,25); 3,3984 (1,64); 3,3899 (3,87); 3,3679 (4,4); 3,3449 (249,75); 3,3367 (240,68); 3,3323 (386,08); 3,3309 (361,35); 3,27 (1,58); 3,2389 (0,98); 3,2014 (0,41); 2,8672 (0,79); 2,8447 (1,24); 2,8112 (0,76); 2,6749 (0,79); 2,6706 (1,02); 2,5411 (0,71); 2,502 (150,05); 2,4522 (0,46); 2,3283 (1,08); 2,1327 (0,91); 2,1207 (0,73); 2,1032 (1,79); 2,0824 (0,86); 2,0731 (5,8); 1,8308 (7,32); 1,825 (16); 1,8192 (7,93); 1,7909 (0,87); 1,7561 (0,44); 1,6181 (0,52); 1,6114 (0,44); 1,5873 (0,95); 1,5788 (0,88); 1,5577 (0,82); 1,5486 (0,91); 1,5296 (0,4); 1,3503 (0,49); 1,235 (1,1); -0,0002 (10,3); -0,0086 (0,6) |
| **Bsp. I-49, Solvent: DMSO-d₆** |
| 11,229 (0,54); 7,966 (16); 7,4173 (2,95); 7,3966 (4,92); 7,3761 (4,16); 7,3179 (2,23); 7,1847 (5,22); 7,1669 (4,74); 7,1615 (5,88); 7,1453 (3,62); 7,1416 (4,91); 7,0517 (2,56); 7,0355 (6,07); 6,915 (4,71); 6,8997 (2,8); 6,2406 (2,1); 6,2163 (2,68); 6,2101 (2,45); 6,1853 (2,05); 5,7564 (15,94); 5,465 (1,16); 5,4225 (3,63); 5,3806 (3,49); 5,3384 (1,02); 4,8414 (0,53); 4,8347 (0,44); 4,8008 (4,39); 4,7923 (5,49); 4,7861 (4,32); 4,7524 (0,41); 4,7463 (0,7); 4,3743 (1,09); 4,3494 (0,99); 4,3439 (1,12); 4,3259 (0,33); 4,0615 (0,36); 4,0438 (0,77); 4,0262 (0,84); 3,9972 (1); 3,962 (1,2); 3,94 (0,32); 3,7713 (1,63); 3,7403 (1,93); 3,7292 (2,67); 3,6985 (2,21); 3,6491 (0,37); 3,6262 (0,36); 3,5878 (2,63); 3,5632 (2,64); 3,5457 (1,97); 3,5213 (1,92); 3,4969 (0,85); 3,4939 (0,8); 3,4682 (0,64); 3,4604 (0,9); 3,4441 (4,22); 3,4381 (8,78); 3,432 (4,65); 3,4194 (2,93); 3,4063 (5,77); 3,3984 (9,03); 3,3738 (6048,35); 3,3513 (14,05); 3,3368 (2,07); 3,3303 (1,97); 3,3266 (1,93); 3,3129 (2,2); 3,3005 (0,77); 3,2958 (0,91); 3,2834 (1,83); 3,2569 (0,7); 3,2521 (0,65); 3,2462 (0,81); 3,211 (0,37); 3,1698 (0,33); 3,0422 (0,5); 2,8868 (0,73); 2,8551 (1,62); 2,827 (0,71); 2,6837 (1,2); 2,6792 (1,71); 2,6745 (1,24); 2,5491 (0,92); 2,5323 (2,93); 2,5141 (178,87); 2,51 (245,29); 2,5065 (161,43); 2,3413 (1,23); 2,3367 (1,66); 2,3319 (1,19); 2,1629 (0,95); 2,1277 (1,77); 2,1053 (0,73); 2,0879 (1,15); 2,0777 (8,97); 2,064 (0,33); 1,9944 (3,02); 1,8555 (0,4); 1,8254 (0,94); 1,8012 (0,74); 1,7724 (0,36); 1,7665 (0,4); 1,6364 (0,44); 1,6299 (0,44); 1,6067 (0,88); 1,5972 (0,86); 1,5749 (0,87); 1,5455 (0,39); 1,5364 (0,32); 1,2404 (1,04); 1,1983 (0,95); 1,1806 (1,86); 1,1628 (0,91) |
| **Bsp. I-50, Solvent: DMSO-d₆** |
| 7,9628 (8,19); 7,3715 (1,63); 7,3509 (3,36); 7,3303 (2,22); 7,3138 (1,26); 7,1805 (2,8); 7,1668 (1,22); 7,0973 (2,07); 7,0951 (2,69); 7,0771 (3,29); 7,0747 (2,48); 7,059 (1,6); 7,0475 (1,37); 7,0307 (3,32); 6,906 (2,65); 6,8947 (1,57); 6,2186 (1,05); 6,1935 (1,5); 6,188 (1,16); 6,1631 (1,07); 5,4691 (0,61); 5,4256 (1,83); 5,3719 (1,71); 5,3299 (0,68); 4,3718 (0,59); 4,3368 (0,61); 4,0802 (1,56); 4,0687 (2,85); 4,0561 (2,58); 4,0379 (3,55); 4,02 (3,62); 4,002 (1,46); 3,9563 (0,55); 3,7289 (0,61); 3,6982 (0,69); 3,6875 (1,65); 3,6572 (1,48); 3,6468 (1,72); 3,6219 (1,72); 3,6049 (0,64); 3,5803 (0,62); 3,4316 (0,52); 3,4211 (0,54); 3,4035 (2,04); 3,394 (2,91); 3,3859 (2,57); 3,3741 (1,78); 3,3248 (939,63); 3,3013 (2,75); 3,2753 (0,86); 3,2465 (0,57); 3,2427 (0,52); 2,8975 (14,63); 2,8755 (0,52); 2,845 (0,8); 2,818 (0,48); 2,6892 (0,51); 2,6752 (0,85); 2,6703 (1,23); 2,6658 (0,89); 2,661 (0,49); 2,5406 (0,8); 2,5238 (2,07); 2,519 (2,95); 2,5095 (70,63); 2,5056 (125,01); 2,5014 (171,49); 2,4977 (112,07); 2,3327 (0,87); 2,3282 (1,15); 2,3235 (0,88); 2,1441 (0,52); 2,1087 (0,94); 2,0734 (1,28); 1,9883 (16); 1,8217 (0,51); 1,7923 (0,51); 1,5881 (0,51); 1,5784 (0,49); 1,553 (0,47); 1,3982 (0,39); 1,2354 (0,63); 1,1922 (4,44); 1,1744 (8,98); 1,1566 (4,44); 0,008 (1,76); -0,0002 (62,49); -0,0085 (1,59) |
| **Bsp. I-51, Solvent- DMSO-d₆** |
| 18,3412 (0,34); 16,7383 (0,35); 11,2347 (0,37); 7,9496 (16); 7,3773 (3,08); 7,3568 (6,3); 7,3362 (4,28); 7,316 (2,07); 7,1829 (5,12); 7,1688 (2,45); 7,1095 (3,66); 7,1073 (5,16); 7,0892 (7,26); 7,0871 (7,11); 7,0678 (3,07); 7,0497 (2,44); 7,0328 (6,11); 6,914 (4,66); 6,897 (2,75); 6,2663 (1,9); 6,2426 (2,59); 6,2352 (2,3); 6,2115 (2,14); 5,8335 (0,54); 5,8191 (1,11); 5,8067 (1,1); 5,7929 (1,49); 5,7761 (1,48); 5,7632 (1,26); 5,7525 (9,43); 5,7367 (0,81); 5,4692 (1,2); 5,4258 (3,44); 5,3759 (3,36); 5,3345 (1,1); 5,3208 (0,38); 5,2722 (2,98); 5,2681 (2,86); 5,2289 (2,45); 5,2249 (2,31); 5,2027 (0,33); 5,0209 (2,38); 5,0174 (2,36); 4,9948 (2,27); 4,991 (2,11); 4,5849 (0,71); 4,5724 (0,67); 4,5534 (2,13); 4,5398 (2,08); 4,5254 (2,05); 4,5114 (2,11); 4,4939 (0,74); 4,4799 (0,81); 4,3733 (1,01); 4,3387 (1,08); 4,0427 (0,45); 4,025 (0,51); 4,0042 (0,66); 3,9904 (0,91); 3,9604 (1,08); 3,7645 (1,44); 3,7334 (1,98); 3,7227 (2,79); 3,6918 (2,14); 3,6016 (0,34); 3,5818 (2,4); 3,5584 (2,31); 3,5402 (1,84); 3,5238 (0,68); 3,5166 (1,92); 3,4894 (0,61); 3,4847 (0,6); 3,4676 (0,7); 3,4573 (1,65); 3,451 (1,02); 3,4347 (2,84); 3,4252 (4,94); 3,4185 (6,74); 3,3951 (4801,39); 3,3751 (10,98); 3,3728 (10,61); 3,3624 (3,24); 3,3589 (3,53); 3,3523 (1,67); 3,3463 (1,79); 3,336 (1,28); 3,3269 (1,53); 3,3207 (1,35); 3,3148 (1,9); 3,3068 (0,92); 3,3036 (0,71); 3,2846 (1,77); 3,2573 (0,92); 3,2337 (0,34); 3,2137 (0,38); 3,1704 (0,36); 3,1667 (0,4); 2,8926 (0,66); 2,8576 (1,27); 2,8278 (0,72); 2,6837 (0,67); 2,6789 (0,93); 2,6745 (0,77); 2,5482 (0,41); 2,5324 (1,54); 2,5141 (95,3); 2,5099 (131,13); 2,5066 (86,18); 2,3416 (0,66); 2,3366 (0,9); 2,3277 (0,42); 2,1521 (0,97); 2,1182 (1,79); 2,0754 (6,83); 1,9929 (1,72); 1,8512 (0,48); 1,8218 (0,92); 1,8002 (0,9); 1,7666 (0,4); 1,6333 (0,5); 1,6214 (0,42); 1,6023 (0,87); 1,5911 (0,87); 1,5705 (0,88); 1,5304 (0,39); 1,2393 (0,73); 1,197 (0,5); 1,1792 (1,04); 1,1612 (0,48) |
| **Bsp. I-52, Solvent: CD₃CN** |
| 7,7021 (16); 7,4902 (4,15); 7,4886 (4,26); 7,4772 (5,81); 7,4758 (5,79); 7,4465 (4,28); 7,4446 (4,55); 7,434 (5,64); 7,4321 (6,12); 7,4177 (2,57); 7,4156 (2,55); 7,4053 (5,48); 7,4031 (4,76); 7,3926 (3,16); 7,3904 (2,61); 7,3628 (4,04); 7,3606 (4,01); 7,3504 (5,48); 7,3481 (5,25); 7,3379 (2,08); 7,3357 (1,97); 6,9894 (4,29); 6,8994 (8,98); 6,878 (4,59); 6,8321 (8,64); 6,8094 (4,5); 6,7868 (9,78); 6,6957 (4,81); 6,0769 (3,71); 6,0626 (4,14); 6,0584 (4,1); 6,0441 (3,79); 5,2659 (2,73); 5,2377 (8,2); 5,203 (8,19); 5,1747 (2,82); 4,8399 (6,89); 4,8202 (10,83); 4,7567 (11,1); 4,737 (7,03); 4,4764 (1,97); 4,454 (2,03); 3,9819 (3,93); 3,9633 (4,06); 3,9533 (4,5); 3,9347 (4,24); 3,9091 (1,9); 3,8858 (2,04); 3,3625 (0,82); 3,3561 (1,71); 3,3489 (5,54); 3,3434 (2); 3,3347 (5,88); 3,3307 (2,11); 3,3239 (1,36); 3,3202 (4,98); 3,3112 (1,1); 3,306 (4,54); 3,2997 (1,62); 3,2952 (1,73); 3,279 (2,1); 3,2756 (2,78); 3,2563 (1,64); 3,2518 (1,47); 2,867 (1,34); 2,8625 (1,49); 2,8452 (2,48); 2,8418 (2,54); 2,8244 (1,52); 2,82 (1,41); 2,1905 (1,72); 2,1691 (18,57); 2,1374 (1,79); 2,1164 (1,9); 1,9653 (7,38); 1,9572 (0,58); 1,953 (0,79); 1,9493 (7,87); 1,9452 (14,91); 1,9411 (21,83); 1,937 (14,88); 1,9328 (7,49); 1,878 (0,74); 1,8711 (0,82); 1,8577 (1,71); 1,851 (1,8); 1,8366 (1,66); 1,8302 (1,68); 1,8166 (0,7); 1,8096 (0,61); 1,7099 (0,79); 1,7028 (0,88); 1,6896 (1,79); 1,6828 (1,87); 1,6685 (1,76); 1,6618 (1,7); 1,6485 (0,77); 1,6414 (0,69); -0,0001 (5,97) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1H-NMR-Peaks die gewöhnlichen Lösungsmittelpeak

### Verwendungsbeispiele

### Beispiel A

### Phytophthora-Test (Tomate) / protektiv

| | |
|---|---|
| Lösungsmittel : | 24,5 Gewichtsteile Aceton |
| | 24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Phytophthora infestans*** inokuliert. Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von **10ppm** einen Wirkungsgrad von 70% oder mehr: I-1 (95%), 1-2 (99%), I-3 (100%), I-5 (93%), I-6 (94%), I-7 (97%), I-8 (97%), I-9 (96%), I-10 (97%), I-14 (98%), I-15 (95%), I-19 (96%), I-20 (95%), I-21 (98%), I-22 (98%), I-23 (97%), I-25 (95%), I-26 (97%), I-35 (88%), I-39 (99%), I-40 (95%), I-41 (94%), I-42 (94%).

### Beispiel B

### Plasmopara-Test (Rebe) / protektiv

| | |
|---|---|
| Lösungsmittel : | 24,5 Gewichtsteile Aceton |
| | 24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von **10ppm** einen Wirkungsgrad von 70% oder mehr: I-1 (100%), I-2 (100%), I-3 (100%), I-4 (100%), I-5 (97%), I-6 (100%), I-7 (100%), I-8 (99%), I-9 (99%), I-10 (100%), I-14 (100%), I-15 (93%), I-19 (96%), I-20 (98%), I-21 (100%), I-22 (97%), I-23 (99%), I-25 (74%), I-26 (100%), I-39 (99%), I-40 (91%), I-41 (100%), I-42 (97%).

### Beispiel C

### Peronospora Test (Rapssamen) / Saatgutbehandlung

Der Test wurde unter Gewächshausbedingungen durchgeführt.

Zur Herstellung einer Wirkstoffzubereitung löste man den Wirkstoff in N-methyl-2-pyrrolidon und verdünnte das Konzentrat mit Wasser auf die gewünschte Konzentration. Die mit dieser Lösung behandelten Rapssamen wurden in 6*6cm großen Töpfen ausgesät, die 4 cm hoch mit einer 1:1-Mischung aus dampfbehandeltem Ackerboden und Sand gefüllt waren. Dann wurden die Pflanzen bei 10°C angezogen.

Nach 14 Tagen wurden die Pflanzen mit einer wäßrigen Sporensuspension von ***Peronospora brassicae*** inokuliert. Anschließend wurden die Pflanzen 7 Tage im Gewächshaus bei ca. 15°C und 100% Luftfeuchtigkeit aufgestellt.

Die Auswertung erfolgte durch Beurteilung der infizierten Blattfläche pro Pflanze. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigten die nachfolgenden erfindungsgemäßen Verbindungen bei einer Konzentration an Wirkstoff von 50 g/dt einen Wirkungsgrad von 80% oder mehr: I-1 (100%), I-6 (100%), I-7 (100%).

## Patentansprüche

1. Verbindungen der Formel (I), 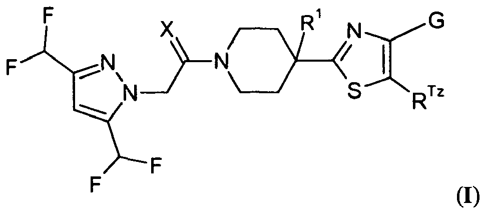 in welcher die Symbole folgende Bedeutungen haben:
X steht für Sauerstoff oder Schwefel,
G steht für G1 = 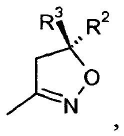 G2 = 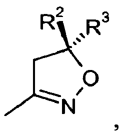 G3 = 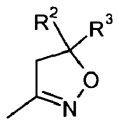 oder G4 = 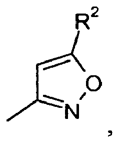
R¹ steht für Wasserstoff oder Halogen,
R² steht für C₁-C₆-Alkyl oder C₁-C₆-Halogenalkyl
oder
R² steht für ein C₃-C₈-Cycloalkyl, das bis zu zwei Substituenten enthalten kann, wobei die Substituenten unabhängig voneinander ausgewählt sind aus folgender Liste:
Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
oder
R² steht für ein C₅-C₈-Cycloalkenyl, das bis zu zwei Substituenten enthalten kann, wobei die Substituenten unabhängig voneinander ausgewählt sind aus folgender Liste:
Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
oder
R² steht für ein Phenyl, das bis zu zwei Substituenten enthalten kann, wobei die Substituenten unabhängig voneinander ausgewählt sind aus folgender Liste:
Amino, Halogen, Cyano, Hydroxy, SH, Nitro, C(=O)H, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Allrylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Allcinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -LQ,
oder
R² steht für gesättigtes oder teilweise oder vollständig ungesättigtes, Naphthyl oder Indenyl, das bis zu zwei Substituenten enthalten kann, wobei die Substituenten unabhängig voneinander ausgewählt sind aus folgender Liste:
Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
R² steht für einen 5- oder 6-gliedrigen Heteroarylrest, der bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -LQ,
Substituenten am Stickstoff C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
oder
R² und R³ bilden zusammen mit dem Kohlenstoffatom, an das sie gebunden sind, einen 5- bis 12-gliedriges, unsubstituiertes oder substituiertes, teilweise gesättigtes oder ungesättigtes, mono- oder bicyclisches Ringsystem, das bis zu drei weitere Heteroatome, ausgewählt aus N, O und S enthalten kann, wobei zwei Sauerstoffatome nicht benachbart sind und wobei mögliche Substituenten unabhängig voneinander ausgewählt sind aus Halogen, C₁-C₄-Alkyl, C₁-C₄-Halogenalkyl, C₁-C₄-Alkoxy, C₁-C₄-Halogenalkoxy, Oxo, Hydroxy, Benzyl oder Phenyl,
R³ steht für Wasserstoff, Cyano, C₁-C₃-Alkyl oder C₁-C₃-Halogenalkyl,
R^{Tz} steht für Halogen oder Wasserstoff
L steht für eine direkte Bindung, -CH₂-, -(C=O)-, Schwefel oder Sauerstoff,
Q steht für ein Phenyl, das bis zu zwei Substituenten enthalten kann, wobei die Substituenten unabhängig voneinander ausgewählt sind aus folgender Liste:
Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₂-C₆-Alkoxyalkyl, C₂-C₆-Alkylcarbonyl, C₂-C₆-Halogenalkylcarbonyl, C₂-C₆-Alkoxycarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Alkinyloxy, C₂-C₆-Alkoxyalkoxy, C₁-C₆-AWylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Tri(C₁-C₄-alkyl)silyl oder Phenyl,
oder
Q steht für ein 5- oder 6-gliedrigen Heteroarylrest, das bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkenyl, C₃-C₆-Cycloallcyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
Substituenten am Stickstoff: C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl, C₁-C₄-Alkylsulfonyl, C(=O)H, C(=O)Me, C(=O)OMe oder Phenyl,
sowie agrochemisch wirksame Salze, Metallkomplexe und N-Oxide davon.

2. Verbindungen der Formel **(I)** nach Anspruch 1,
in welcher die Symbole folgende Bedeutungen haben,
X steht für Sauerstoff,
R¹ steht für Wasserstoff oder Fluor,
G steht für G1, G3 und G4,
R² steht für ein C₃-C₈-Cycloalkyl, das bis zu zwei Substituenten enthalten kann, wobei die Substituenten unabhängig voneinander ausgewählt sind aus folgender Liste:
Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
R² steht außerdem für ein Phenyl, das bis zu zwei Substituenten enthalten kann, wobei die Substituenten unabhängig voneinander ausgewählt sind aus folgender Liste:
Amino, Halogen, Cyano, Hydroxy, SH, Nitro, C(=O)H, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino oder -LQ,
R² steht außerdem für einen 5- oder 6-gliedrigen Heteroarylrest, der bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, SH, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder -LQ,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogeneycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe, Benzyl oder Phenyl,
R³ steht für Wasserstoff, Cyano oder C₁-C₃-Alkyl,
L steht für eine direkte Bindung oder Sauerstoff,
Q steht für ein Phenyl, das bis zu zwei Substituenten enthalten kann, wobei die Substituenten unabhängig voneinander ausgewählt sind aus folgender Liste:
Halogen, Cyano, Hydroxy, Nitro, SH, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
Q steht außerdem für ein 5- oder 6-gliedrigen Heteroarylrest, das bis zu zwei Substituenten enthalten kann, wobei die Substituenten unabhängig voneinander ausgewählt sind aus folgender Liste:
Substituenten am Kohlenstoff
Halogen, Cyano, Hydroxy, Nitro, SH, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio oder Phenyl,
Substituenten am Stickstoff: C₁-C₆-Alkyl, C₂-C₆-Alkenyl, C₂-C₆-Alkinyl, C₃-C₆-Cycloalkyl oder Phenyl,
sowie agrochemisch wirksame Salze, Metallkomplexe und N-Oxide davon.

3. Verbindungen der Formel (**I**) nach einem der vorherigen Ansprüche, in welcher die Symbole folgende Bedeutungen haben,
G für G3 und/oder G4 und insbesondere für G3 steht,
sowie agrochemisch wirksame Salze, Metallkomplexe und N-Oxide davon.

4. Verbindungen der Formel (**I**) nach einem der vorherigen Ansprüche, in welcher die Symbole folgende Bedeutungen haben,
R² steht für ein Phenyl, das bis zu zwei Substituenten enthalten kann, wobei die Substituenten unabhängig voneinander ausgewählt sind aus folgender Liste:
Amino, Halogen, Cyano, Nitro, C₁-C₆-Alkyl, C₂-C₆-Alkenyl, C₂-C₆-Alkinyl, C₁-C₆-Halogenalkyl, C₂-C₆-Halogenalkenyl, C₂-C₆- Halogenalkinyl, C₃-C₈-Cycloalkyl, C₃-C₈-Halogencycloalkyl, C₂-C₆-Alkoxyalkyl, C₂-C₆-Alkylcarbonyl, C₂-C₆-Halogenalkylcarbonyl, C₂-C₆-Alkoxycarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Alkinyloxy, C₂-C₆-Alkoxyalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Phenyl,
sowie agrochemisch wirksame Salze, Metallkomplexe und N-Oxide davon.

5. Verbindungen der Formel (I) nach Anspruch 4, in welcher
R² für Phenyl, 2-Fluorphenyl, 2,6-Difluorphenyl, 2-Acetylphenyl, 3-Acetylphenyl, 2-Hydroxyphenyl, 2-Nitrophenyl, 2-[(2-Methoxyethoxy)methyl]phenyl, 2-[(Ethylsulfanyl)methyl]phenyl, 2-[(Cyclopropylmethoxy)carbonyl]phenyl, 2-(Allyloxy)phenyl, 3-(But-2-in-1-yloxy)phenyl, 2-(Butoxymethyl)phenyl, 2-Fluor-6-formylphenyl, 2-[(2-Methylprop-2-en-1-yl)oxy]phenyl, 2-(2-Methoxyethoxy)phenyl, 2-[(3-Methylbut-2-en-1-yl)oxy]phenyl, 3-(Prop-2-in-1-yloxy)phenyl, 4-(Prop-2-in-1-yloxy)phenyl, 3-Formylphenyl, 2-(Cyclohexylmethoxy)phenyl, 2-(Pent-2-in-1-yloxy)phenyl, 2-Formylphenyl, 2-(Cyclopropylcarbamoyl)phenyl, 2-(But-2-in-1-yloxy)-6-fluorphenyl, 2-Fluor-6-(prop-2-in-1-yloxy)pheny, 2-[(Cyclohexylcarbonyl)oxy]phenyl, 2-[(Cyclopropylcarbonyl)oxy]phenyl, 3-(Pent-2-in-1-yloxy)phenyl, 2-(But-2-in-1-yloxy)phenyl, 2-[(3,3,3-Trifluorpropanoyl)oxy]phenyl, 2-[(Methylsulfonyl)amino]phenyl, 2-Ethinylphenyl, 2-(Prop-2-in-1-yloxy)phenyl, 4-[(Methylsulfonyl)amino]phenyl, 2-Aminophenyl, 3-Hydroxyphenyl, 2-(Methoxycarbonyl)phenyl, 2-(Chloromethyl)phenyl, 4-(Pent-2-in-1-yloxy)phenyl, 4-(But-2-in-1-yloxy)phenyl, 2-Chlor-6-(prop-2-in-1-yloxy)phenyl, 2-Chlor-6-(2-methoxyethoxy)phenyl, 2-(Allyloxy)-6-chlorphenyl, 2-[(2,2,2-Trifluorethoxy)methyl]phenyl, 2-[(Ethylsulfonyl)methyl]phenyl oder 2-(Hydroxymethyl)phenyl steht,
sowie agrochemisch wirksame Salze, Metallkomplexe und N-Oxide davon

6. Verbindungen der Formel (I) nach einem der vorherigen Ansprüche, in welcher
R³ für Wasserstoff steht,
sowie agrochemisch wirksame Salze, Metallkomplexe und N-Oxide davon

7. Verbindungen der Formel (I) nach einem der vorherigen Ansprüche, in welcher
R^{Tz} für Wasserstoff steht,
sowie agrochemisch wirksame Salze, Metallkomplexe und N-Oxide davon.

8. Verfahren zur Bekämpfung von pflanzenpathogenen Schadpilzcn, **dadurch gekennzeichnet, dass** man Verbindungen der Formel (**I**) gemäß einem der Ansprüche 1 bis 7, auf die pflanzenpathogenen Schadpilze und/oder deren Lebensraum ausbringt.

9. Mittel zur Bekämpfung von pflanzenpathogenen Schadpilzen, **gekennzeichnet durch** einen Gehalt an mindestens einer Verbindung der Formel (**I**) gemäß einem der Ansprüche 1 bis 7, neben Streckmitteln und/oder oberflächenaktiven Stoffen.

10. Verwendung von Bis(difluormethyl)pyrazol-Derivaten der Formel (**I**), gemäß einem der Ansprüche 1 bis 7 zur Bekämpfung von pflanzenpathogenen Schadpilzen.

11. Verfahren zur Herstellung von Mitteln zur Bekämpfung von pflanzenpathogenen Schadpilzen, **dadurch gekennzeichnet, dass** man Bis(difluormethyl)pyrazol-Derivate der Formel (I) gemäß einem der Ansprüche 1 bis 7 mit Streckmitteln und/oder oberflächenaktiven Stoffen vermischt.

12. Verwendung von Verbindungen der Formel (**I**) gemäß einem der Ansprüchen 1 bis 7 zur Behandlung von transgenen Pflanzen.

13. Verfahren zum Herstellen der Verbindungen der Formel (I) gemäß einem der Ansprüche 1 bis 7 umfassend wenigstens einen der folgenden Schritte (E) bis (J):
Schritt (E): Zykloaddition einer Verbindung der allgemeinen Formel (VIII) mit einer Verbindung der allgemeinen Formel (IX) zu Verbindungen der Formel (X-a) oder (Ia) in Gegenwart einer Base und gegebenenfalls in Gegenwart eines Lösungsmittels gemäß dem nachfolgenden Reaktionsschema (Schema 5): 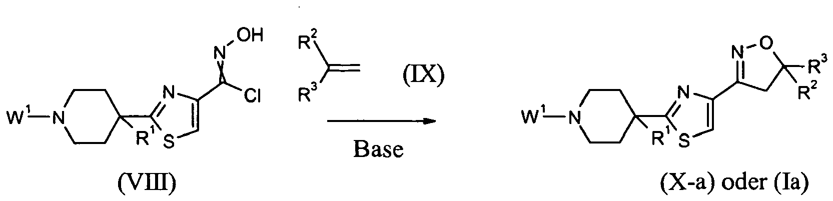 W¹ steht für Acetyl, C₁-C₄-Alkoxycarbonyl, Benzyl, Benzyloxycarbonyl oder [3,5-Bis(difluormethyl)-1H-pyrazol-1-yl]acetyl,
die Reste R¹, R², R³ haben die gleiche Bedeutung wie die in den vorherigen Ansprüchen für Formel (I) definierten Reste R¹, R², R³;
Schritt (F): Zykloaddition einer Verbindung der allgemeinen Formel (VIII) mit einer Verbindung der allgemeinen Formel (XI) zu Verbindungen der Formel (X-b) oder (Ia) in Gegenwart einer Base und gegebenenfalls in Gegenwart eines Lösungsmittels gemäß dem nachfolgenden Reaktionsschema (Schema 6): 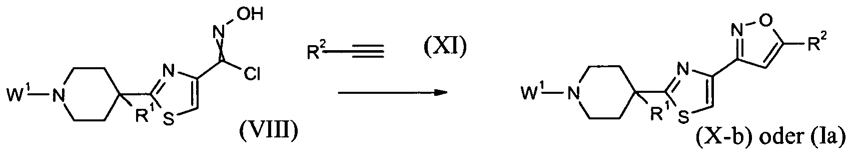 W¹ steht für Acetyl, C₁-C₄-Alkoxycarbonyl, Benzyl, Benzyloxycarbonyl oder [3,5-Bis(difluormethyl)-1H-pyrazol-1-yl]acetyl,
die Reste R¹, R² haben die gleiche Bedeutung wie die in den vorherigen Ansprüchen für Formel (I) definierten Reste R¹, R²;
Schritt (G): Entfernung der Schutzgruppe der Verbindungen der allgemeinen Formel (X) zum Erhalt von Verbindungen der allgemeinen Formel (XII) gemäß dem nachfolgenden Reaktionsschema (Schema 7): 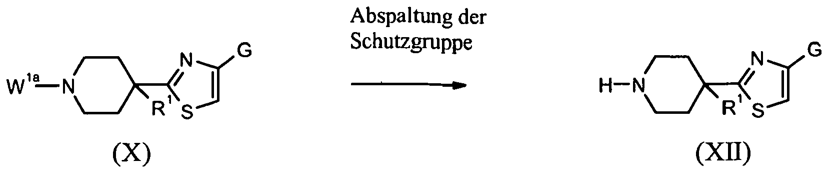 W^{1a} steht für Acetyl, C₁-C₄-Alkoxycarbonyl, Benzyl oder Benzyloxycarbonyl, die Reste R¹, G haben die gleiche Bedeutung wie die in den vorherigen Ansprüchen für Formel (I) definierten Reste R¹, G;
Schritt (H): Kupplungsreaktion der Verbindungen der allgemeinen Formel (XII) mit einem Substrat der allgemeinen Formel (XIII)) zum Erhalt von Verbindungen der allgemeinen Formel (Ia) gemäß dem nachfolgenden Reaktionsschema (Schema 8): 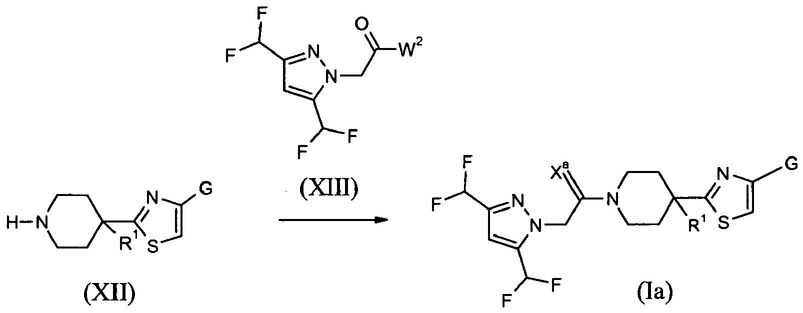 W² steht für Chlor oder OH,
X^{a} steht für Sauerstoff,
die Reste R¹, G haben die gleiche Bedeutung wie die in den vorherigen Ansprüchen für Formel (I) definierten Reste R¹, G;
Schritt (I): Schwefelung der Verbindungen gemäß allgemeiner Formel (I-a) zum Erhalt von Verbindungen der allgemeinen Formel (I-c) gemäß dem nachfolgenden Reaktionsschema (Schema 9): 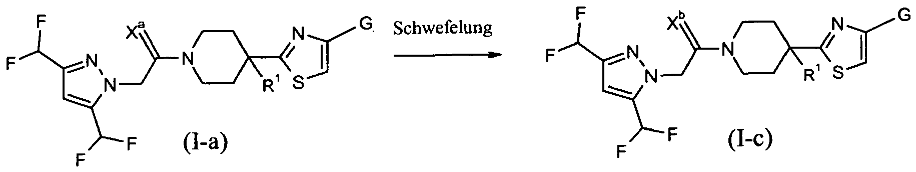 X^{a} steht für Sauerstoff,
X^{b} steht für Schwefel,
die Reste R¹, G haben die gleiche Bedeutung wie die in den vorherigen Ansprüchen für Formel (I) definierten Reste R¹, G;
Schritt (J): Halogenierung der Verbindungen gemäß allgemeiner Formel (X-a) oder(I-a) zum Erhalt von Verbindungen der allgemeinen Formel (X-d) oder (I-d) gemäß dem nachfolgenden Reaktionsschema (Schema 10): 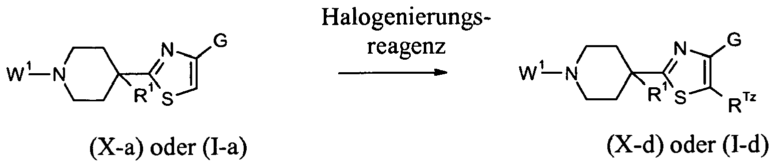 W¹ steht für Acetyl, C₁-C₄-Alkoxycarbonyl, Benzyl, Benzyloxycarbonyl oder [3,5-Bis(difluormethyl)-1H-pyrazol-1-yl]acetyl,
R^{TZ} steht für ein Halogen
die Reste R¹, G haben die gleiche Bedeutung wie die in den vorherigen Ansprüchen für Formel (I) definierten Reste R¹, G.

## Claims

1. Compounds of the formula (I) 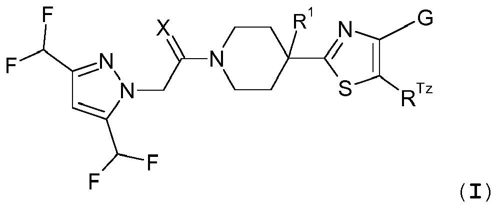 in which the symbols are each defined as follows:
X is oxygen or sulphur, G is G1 = 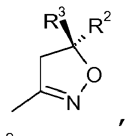 G2 = 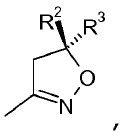 G3 = 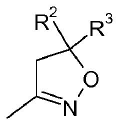 or G4 = 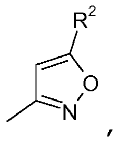
R¹ is hydrogen or halogen,
R² is C₁-C₆-alkyl or C₁-C₆-haloalkyl,
or
R² is a C₃-C₈-cycloalkyl which may contain up to two substituents, where the substituents are each independently selected from the following list:
cyano, halogen, hydroxyl, oxo, C₁-C₄-alkyl, C₁-C₄-haloalkyl, C₁-C₄-alkoxy, C₁-C₄-haloalkoxy, C₁-C₄-alkylthio, C₁-C₄-haloalkylthio or phenyl,
or
R² is a C₅-C₈-cycloalkenyl which may contain up to two substituents, where the substituents are each independently selected from the following list:
cyano, halogen, hydroxyl, oxo, C₁-C₄-alkyl, C₁-C₄-haloalkyl, C₁-C₄-alkoxy, C₁-C₄-haloalkoxy, C₁-C₄-alkylthio, C₁-C₄-haloalkylthio or phenyl,
or
R² is a phenyl which may contain up to two substituents, where the substituents are each independently selected from the following list:
amino, halogen, cyano, hydroxyl, SH, nitro, C(=O)H, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkylcycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl, C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₂-C₆-alkenyloxy, C₂-C₆-haloalkenyloxy, C₂-C₆-alkynyloxy, C₂-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri(C₁-C₄-alkyl)silyl, C₁-C₆-alkylsulphonylamino, C₁-C₆-haloalkylsulphonylamino or -LQ,
or
R² is saturated or partly or fully unsaturated naphthyl or indenyl which may contain up to two substituents, where the substituents are each independently selected from the following list:
cyano, nitro, halogen, C₁-C₆-alkyl, C₁-C₄-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₄-alkyl)silyl, benzyl, phenyl, hydroxyl, SH, oxo, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
or
R² is a 5- or 6-membered heteroaryl radical which may contain up to two substituents, where the substituents are each independently selected from the following list:
substituents on carbon: halogen, cyano, nitro, hydroxyl, SH, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or -LQ,
substituents on nitrogen: C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₁-C₄-alkylsulphonyl, C(=O)H, C(=O)Me, C(=O)OMe, benzyl or phenyl,
or
R² and R³, together with the carbon atom to which they are bonded, form a 5- to 12-membered unsubstituted or substituted, partly saturated or unsaturated, mono- or bicyclic ring system which may contain up to three further heteroatoms selected from N, O and S, where no two oxygen atoms are adjacent and where any possible substituents are selected independently from halogen, C₁-C₄-alkyl, C₁-C₄-haloalkyl, C₁-C₄-alkoxy, C₁-C₄-haloalkoxy, oxo, hydroxyl, benzyl and phenyl,
R³ is hydrogen, cyano, C₁-C₃-alkyl or C₁-C₃-haloalkyl,
R^{Tz} is halogen or hydrogen
L is a direct bond, -CH₂-, -(C=O)-, sulphur or oxygen,
Q is a phenyl which may contain up to two substituents, where the substituents are each independently selected from the following list:
halogen, cyano, nitro, hydroxyl, SH, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₂-C₆-alkoxyalkyl, C₂-C₆-alkylcarbonyl, C₂-C₆-haloalkylcarbonyl, C₂-C₆-alkoxycarbonyl, C₁-C₆-alkoxy, C₁-C₆-haloalkoxy, C₃-C₈-cycloalkoxy, C₃-C₈-halocycloalkoxy, C₂-C₆-alkenyloxy, C₂-C₆-alkynyloxy, C₂-C₆-alkoxyalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, tri(C₁-C₄-alkyl)silyl or phenyl,
or
Q is a 5- or 6-membered heteroaryl radical which may contain up to two substituents, where the substituents are each independently selected from the following list:
substituents on carbon: halogen, cyano, nitro, hydroxyl, SH, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or phenyl,
substituents on nitrogen: C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, C₁-C₄-alkylsulphonyl, C(=O)H, C(=O)Me, C(=O)OMe or phenyl,
and agrochemically active salts, metal complexes and N-oxides thereof.

2. Compounds of the formula (I) according to Claim 1, in which the symbols are each defined as follows:
X is oxygen,
R¹ is hydrogen or fluoroine,
G is G1, G3 and G4,
R² is a C₃-C₈-cycloalkyl which may contain up to two substituents, where the substituents are each independently selected from the following list:
cyano, halogen, hydroxyl, oxo, C₁-C₄-alkyl, C₁-C₄-haloalkyl, C₁-C₄-alkoxy, C₁-C₄-haloalkoxy, C₁-C₄-alkylthio, C₁-C₄-haloalkylthio or phenyl,
R² is also a phenyl which may contain up to two substituents, where the substituents are each independently selected from the following list:
amino, halogen, cyano, hydroxyl, SH, nitro, C(=O)H, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkylcycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl, C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₂-C₆-alkenyloxy, C₂-C₆-haloalkenyloxy, C₂-C₆-alkynyloxy, C₂-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri (C₁-C₄-alkyl)silyl, C₁-C₆-alkylsulphonylamino, C₁-C₆-haloalkylsulphonylamino or -LQ,
R² is also a 5- or 6-membered heteroaryl radical which may contain up to two substituents, where the substituents are each independently selected from the following list:
substituents on carbon: halogen, cyano, nitro, hydroxyl, SH, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or -LQ,
substituents on nitrogen: C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₁-C₄-alkylsulphonyl, C(=O)H, C(=O)Me, C(=O)OMe, benzyl or phenyl,
R³ is hydrogen, cyano or C₁-C₃-alkyl,
L is a direct bond or oxygen,
Q is a phenyl which may contain up to two substituents, where the substituents are each independently selected from the following list:
halogen, cyano, hydroxyl, nitro, SH, C₁-C₆-alkyl, C₁-C₆-haloalkyl, C₃-C₆-cycloalkyl, C₁-C₄-alkoxy, C₁-C₄-haloalkoxy, C₁-C₄-alkylthio, C₁-C₄-haloalkylthio or phenyl,
Q is also a 5- or 6-membered heteroaryl radical which may contain up to two substituents, where the substituents are each independently selected from the following list:
substituents on carbon:
halogen, cyano, hydroxyl, nitro, SH, C₁-C₆-alkyl, C₁-C₆-haloalkyl, C₃-C₆-cycloalkyl, C₁-C₄-alkoxy, C₁-C₄-haloalkoxy, C₁-C₄-alkylthio, C₁-C₄-haloalkylthio or phenyl,
substituents on nitrogen: C₁-C₆-alkyl, C₂-C₆-alkenyl, C₂-C₆-alkynyl, C₃-C₆-cycloalkyl or phenyl,
and agrochemically active salts, metal complexes and N-oxides thereof.

3. Compounds of the formula **(I)** according to either of the preceding claims, in which the symbols are each defined as follows:
G is G3 and/or G4 and is especially G3,
and agrochemically active salts, metal complexes and N-oxides thereof.

4. Compounds of the formula **(I)** according to any of the preceding claims, in which the symbols are each defined as follows:
R² is a phenyl which may contain up to two substituents, where the substituents are each
independently selected from the following list:
amino, halogen, cyano, nitro, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₂-C₆-alkoxyalkyl, C₂-C₆-alkylcarbonyl, C₂-C₆-haloalkylcarbonyl, C₂-C₆-alkoxycarbonyl, C₁-C₆-alkoxy, C₁-C₆-haloalkoxy, C₃-C₈-cycloalkoxy, C₃-C₈-halocycloalkoxy, C₂-C₆-alkenyloxy, C₂-C₆-alkynyloxy, C₂-C₆-alkoxyalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, phenyl,
and agrochemically active salts, metal complexes and N-oxides thereof.

5. Compounds of the formula (I) according to Claim 4, in which
R² is phenyl, 2-fluorophenyl, 2,6-difluorophenyl, 2-acetylphenyl, 3-acetylphenyl, 2-hydroxyphenyl, 2-nitrophenyl, 2-[(2-methoxyethoxy)methyl]phenyl, 2-[(ethylsulphanyl)methyl]phenyl, 2-[(cyclopropylmethoxy)carbonyl]phenyl, 2-(allyloxy)phenyl, 3-(but-2-yn-1-yloxy)phenyl, 2-(butoxymethyl)phenyl, 2-fluoro-6-formylphenyl, 2-[(2-methylprop-2-en-1-yl)oxy]phenyl, 2-(2-methoxyethoxy)phenyl, 2-[(3-methylbut-2-en-1-yl)oxylphenyl, 3-(prop-2-yn-1-yloxy)phenyl, 4-(prop-2-yn-1-yloxy)phenyl, 3-formylphenyl, 2-(cyclohexylmethoxy)phenyl, 2-(pent-2-yn-1-yloxy)phenyl, 2-formylphenyl, 2-(cyclopropylcarbamoyl)phenyl, 2-(but-2-yn-1-yloxy)-6-fluorophenyl, 2-fluoro-6-(prop-2-yn-1-yloxy)phenyl, 2-[(cyclohexylcarbonyl)oxy]phenyl, 2-[(cyclopropylcarbonyl)oxy]phenyl, 3-(pent-2-yn-1-yloxy)phenyl, 2-(but-2-yn-1-yloxy)phenyl, 2-[(3,3,3-trifluoropropanoyl)oxy]phenyl, 2-[(methylsulphonyl)amino]phenyl, 2-ethynylphenyl, 2-(prop-2-yn-1-yloxy)phenyl, 4-[(methylsulphonyl)amino]phenyl, 2-aminophenyl, 3-hydroxyphenyl, 2-(methoxycarbonyl)phenyl, 2-(chloromethyl)phenyl, 4-(pent-2-yn-1-yloxy)phenyl, 4-(but-2-yn-1-yloxy)phenyl, 2-chloro-6-(prop-2-yn-1-yloxy)phenyl, 2-chloro-6-(2-methoxyethoxy)phenyl, 2-(allyloxy)-6-chlorophenyl, 2-[(2,2,2-trifluoroethoxy)methyl]phenyl, 2-[(ethylsulphonyl)methyl]phenyl or 2-(hydroxymethyl)phenyl,
and agrochemically active salts, metal complexes and N-oxides thereof.

6. Compounds of the formula (I) according to any of the preceding claims, in which
R³ is hydrogen,
and agrochemically active salts, metal complexes and N-oxides thereof.

7. Compounds of the formula (I) according to any of the preceding claims, in which
R^{Tz} is hydrogen
and agrochemically active salts, metal complexes and N-oxides thereof.

8. Method for controlling phytopathogenic harmful fungi, **characterized in that** compounds of the formula (**I**) according to any of Claims 1 to 7 are applied to the phytopathogenic harmful fungi and/or their habitat.

9. Composition for controlling phytopathogenic harmful fungi, **characterized by** a content of at least one compound of the formula **(I)** according to any of Claims 1 to 7, in addition to extenders and/or surfactants.

10. Use of bis(difluoromethyl)pyrazole derivatives of the formula **(I)** according to any of Claims 1 to 7 for controlling phytopathogenic harmful fungi.

11. Process for producing compositions for controlling phytopathogenic harmful fungi, **characterized in that** bis(difluoromethyl)pyrazole derivatives of the formula **(I)** according to any of Claims 1 to 7 are mixed with extenders and/or surfactants.

12. Use of compounds of the formula (**I**) according to any of Claims 1 to 7 for treating transgenic plants.

13. Process for preparing the compounds of the formula (I) according to any of Claims 1 to 7, comprising at least one of the following steps (E) to (J):
Step (E): cycloaddition of a compound of the general formula (VIII) with a compound of the general formula (IX) to give compounds of the formula (X-a) or (Ia) in the presence of a base and optionally in the presence of a solvent according to the following reaction scheme (Scheme 5): 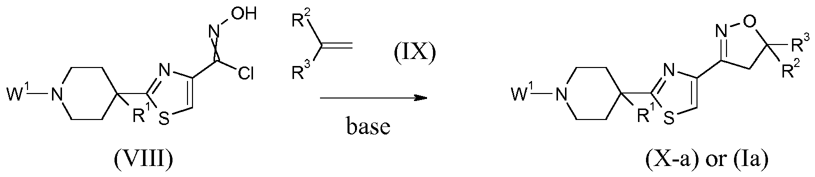 W¹ is acetyl, C₁-C₄-alkoxycarbonyl, benzyl, benzyloxycarbonyl or [3,5-bis(difluoromethyl)-1H-pyrazol-1-yl]acetyl,
the R¹, R², R³ radicals are each as defined for the R¹, R², R³ radicals defined in the preceding claims for formula (I);
Step (F): cycloaddition of a compound of the general formula (VIII) with a compound of the general formula (XI) to give compounds of the formula (X-b) or (Ia) in the presence of a base and optionally in the presence of a solvent according to the following reaction scheme (Scheme 6): 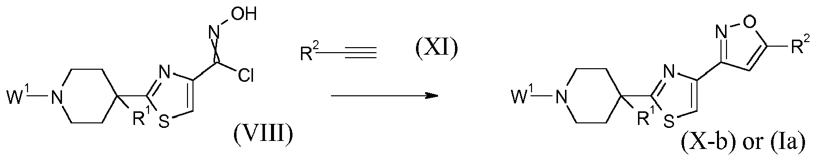 W¹ is acetyl, C₁-C₄-alkoxycarbonyl, benzyl, benzyloxycarbonyl or [3,5-bis(difluoromethyl)-1H-pyrazol-1-yl]acetyl,
the R¹, R² radicals are each as defined for the R¹, R² radicals defined in the preceding claims for formula (I);
Step (G): Removal of the protecting group of the compounds of the general formula (X) to obtain compounds of the general formula (XII) according to the following reaction scheme (Scheme 7): 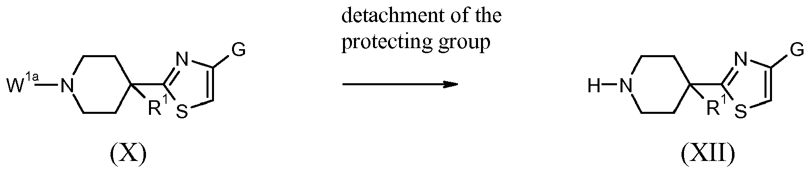 W^{1a} is acetyl, C₁-C₄-alkoxycarbonyl, benzyl or benzyloxycarbonyl, the R¹, G radicals are each as defined for the R¹, G radicals defined in the preceding claims for formula (I);
Step (H): Coupling reaction of the compounds of the general formula (XII) with a substrate of the general formula (XIII) to obtain compounds of the general formula (Ia) according to the following reaction scheme (Scheme 8): 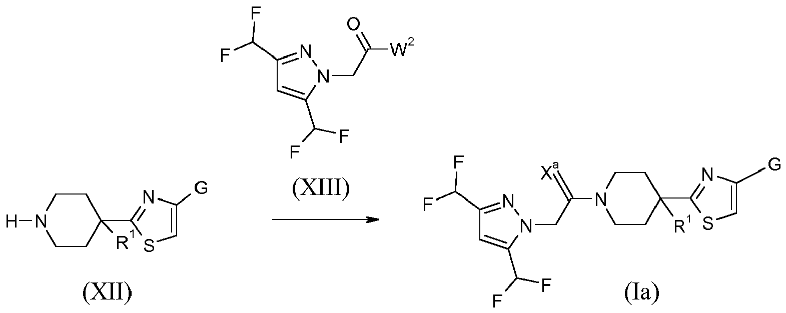 W² is chlorine or OH,
X^{a} is oxygen,
the R¹, G radicals are each as defined for the R¹, G radicals defined for formula (I) in the preceding claims;
Step (I): Thionation of the compounds of the general formula (I-a) to obtain compounds of the general formula (I-c) according to the following reaction scheme (Scheme 9): 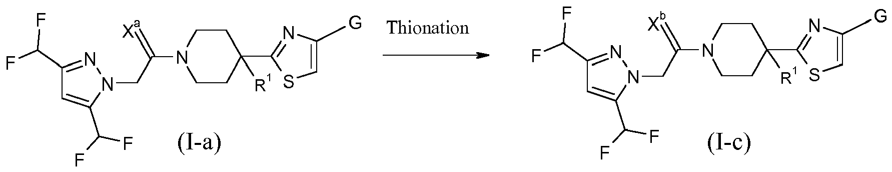 X^{a} is oxygen,
X^{b} is sulphur,
the R¹, G radicals are each as defined for the R¹, G radicals defined for formula (I) in the preceding claims;
Step (J): Halogenation of the compounds of the general formula (X-a) or (I-a) to obtain compounds of the general formula (X-d) or (I-d) according to the following reaction scheme (Scheme 10): 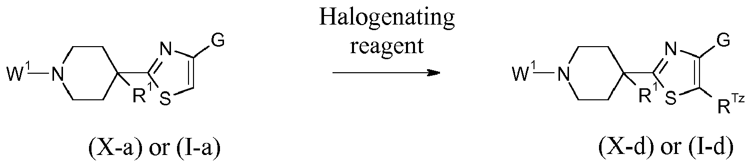 W¹ is acetyl, C₁-C₄-alkoxycarbonyl, benzyl, benzyloxycarbonyl or [3,5-bis(difluoromethyl)-1H-pyrazol-1-yl]acetyl,
R^{TZ} is halogen
the R¹, G radicals are each as defined for the R¹, G radicals defined for formula (I) in the preceding claims.

## Revendications

1. Composés de formule (I) 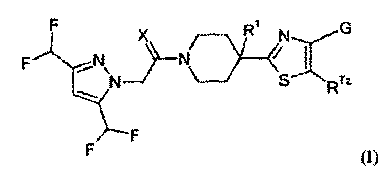 dans lesquels les symboles ont les significations suivantes :
X représente oxygène ou soufre, G représente G1 = 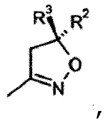 G2 = 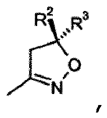 G3 = 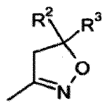 ou G4 = 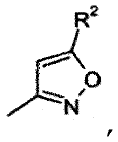
R¹ représente hydrogène ou halogène,
R² représente alkyle en C₁-C₆ ou halogénoalkyle en C₁-C₆,
ou R² représente un cycloalkyle en C₃-C₈, qui peut contenir jusqu'à deux substituants, les substituants étant choisis indépendamment les uns des autres dans la liste suivante :
cyano, halogène, hydroxy, oxo, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄ ou phényle,
ou
R² représente un cycloalcényle en C₅-C₈, qui peut contenir jusqu'à deux substituants, les substituants étant choisis indépendamment les uns des autres dans la liste suivante :
cyano, halogène, hydroxy, oxo, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄ ou phényle, ou
R² représente un phényle, qui peut contenir jusqu'à deux substituants, les substituants étant choisis indépendamment les uns des autres dans la liste suivante :
amino, halogène, cyano, hydroxy, SH, nitro, C(=O)H, alkyle en C₁-C₆, alcényle en C₂-C₆, alcynyle en C₂-C₆, halogénoalkyle en C₁-C₆, halogénalcényle en C₂-C₆, halogénoalcynyle en C₂-C₆, cycloalkyle en C₃-C₈, halogénocycloalkyle en C₃-C₈, alkylcycloalkyle en C₄-C₁₀, cycloalkylalkyle en C₄-C₁₀, cycloalkylcycloalkyle en C₆-C₁₄, halogénocycloalkylalkyle en C₄-C₁₀, alkylcycloalkylalkyle en C₅-C₁₀, cycloalcényle en C₃-C₈, halogénocycloalcényle en C₃-C₈, alcoxyalkyle en C₂-C₆, cycloalcoxyalkyle en C₄-C₁₀, alcoxyalcoxyalkyle en C₃-C₈, alkylthioalkyle en C₂-C₆, alkylsulfinylalkyle en C₂-C₆, alkylsulfonylalkyle en C₂-C₆, alkylaminoalkyle en C₂-C₆, dialkylaminoalkyle en C₃-C₈, halogénoalkylaminoalkyle en C₂-C₆, cycloalkylaminoalkyle en C₄-C₁₀, alkylcarbonyle en C₂-C₆, halogénoalkylcarbonyle en C₂-C₆, cycloalkylcarbonyle en C₄-C₈, alcoxycarbonyle en C₂-C₆, cycloalcoxycarbonyle en C₄-C₈, cycloalkylalcoxycarbonyle en C₅-C₁₀, alkylaminocarbonyle en C₂-C₆, dialkylaminocarbonyle en C₃-C₈, cycloalkylaminocarbonyle en C₄-C₈, halogénoalcoxyalkyle en C₂-C₆, hydroxyalkyle en C₁-C₆, alcoxy en C₁-C₆, halogénoalcoxy en C₁-C₆, cycloalcoxy en C₃-C₈, halogénocycloalcoxy en C₃-C₈, cycloalkylalcoxy en C₄-C₁₀, alcényloxy en C₂-C₆, halogénoalcényloxy en C₂-C₆, alcynyloxy en C₂-C₆, halogénoalcynyloxy en C₂-C₆, alcoxyalcoxy en C₂-C₆, alkylcarbonyloxy en C₂-C₆, halogénoalkylcarbonyloxy en C₂-C₆, cycloalkylcarbonyloxy en C₄-C₈, alkylcarbonylalcoxy en C₃-C₆, alkylthio en C₁-C₆, halogénoalkylthio en C₁-C₆, cycloalkylthio en C₃-C₆, alkylsulfinyle en C₁-C₆, halogénoalkylsulfinyle en C₁-C₆, alkylsulfonyle en C₁-C₆, halogénoalkylsulfonyle en C₁-C₆, cycloalkylsulfonyle en C₃-C₈, tri(alkyle en C₁-C₄)silyle, alkylsulfonylamino en C₁-C₆, halogénoalkylsulfonylamino en C₁-C₆ ou -LQ,
ou
R² représente un naphtyle ou indényle saturé ou partiellement ou complètement insaturé, qui peut contenir jusqu'à deux substituants, les substituants étant choisis indépendament les uns des autres dans la liste suivante :
cyano, nitro, halogène, alkyle en C₁-C₆, halogénoalkyle en C₁-C₄, cycloalkyle en C₃-C₆, alcényle en C₂-C₆, halogénoalcényle en C₂-C₆, alcynyle en C₂-C₆, halogénoalcynyle en C₂-C₆, tri(alkyle en C₁-C₄)silyle, benzyle, phényle, hydroxy, SH, oxo, alcoxy en C₁-C₆, halogénoalcoxy en C₁-C₆, alcényloxy en C₂-C₆, alcynyloxy en C₂-C₆, alkylthio en C₁-C₆ ou halogénoalkylthio en C₁-C₆,
ou
R² représente un radical hétéroaryle à 5 ou 6 éléments, qui peut contenir jusqu'à deux substituants, les substituants étant choisis indépendamment les uns des autres dans la liste suivante :
substituants sur le carbone : halogène, cyano, nitro, hydroxy, SH, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, tri(alkyle en C₁-C₄)silyle ou -LQ,
substituants sur l'azote : alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, alkylsulfonyle en C₁-C₄, C(=O)H, C(=O)Me, C(=O)OMe, benzyle ou phényle,
ou
R² et R³ forment ensemble avec l'atome de carbone auquel ils sont reliés un système cyclique mono- ou bicyclique de 5 à 12 éléments, non substitué ou substitué, partiellement saturé ou insaturé, qui peut contenir jusqu'à trois hétéroatomes supplémentaires, choisis parmi N, O et S, deux atomes d'oxygène n'étant pas voisins et les substituants possibles étant choisis indépendamment les uns des autres parmi halogène, alkyle en C₁-C₄, halogénoalkyle en C₁-C₄, alcoxy en C₁-C₄, halogénoalcoxy en C₁-C₄, oxo, hydroxy, benzyle ou phényle,
R³ représente hydrogène, cyano, alkyle en C₁-C₃ ou halogénoalkyle en C₁-C₃,
R^{Tz} représente halogène ou hydrogène,
L représente une liaison directe, -CH₂-, -(C=O)-, soufre ou oxygène,
Q représente un phényle, qui peut contenir jusqu'à deux substituants, les substituants étant choisis indépendamment les uns des autres dans la liste suivante :
halogène, cyano, nitro, hydroxy, SH, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₈, halogénocycloalkyle en C₃-C₈, alcoxyalkyle en C₂-C₆, alkylcarbonyle en C₂-C₆, halogénoalkylcarbonyle en C₂-C₆, alcoxycarbonyle en C₂-C₆, alcoxy en C₁-C₆, halogénoalcoxy en C₁-C₆, cycloalcoxy en C₃-C₈, halogénocycloalcoxy en C₃-C₈, alcényloxy en C₂-C₆, alcynyloxy en C₂-C₆, alcoxyalcoxy en C₂-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, tri(alkyle en C₁-C₄)silyle ou phényle,
ou
Q représente un radical hétéroaryle à 5 ou 6 éléments, qui peut contenir jusqu'à deux substituants, les substituants étant choisis indépendamment les uns des autres dans la liste suivante :
substituants sur le carbone : halogène, cyano, nitro, hydroxy, SH, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, tri (alkyle en C₁-C₄) silyle ou phényle,
substituants sur l'azote : alkyle en C₁-C₄, alcényle en C₂-C₄, alcynyle en C₂-C₄, halogénoalkyle en C₁-C₄, halogénoalcényle en C₂-C₄, halogénoalcynyle en C₂-C₄, cycloalkyle en C₃-C₆, alkylsulfonyle en C₁-C₄, C(=O)H, C(=O)Me, C(=O)OMe ou phényle, ainsi que leurs sels agrochimiquement actifs, leurs complexes métaliques et leurs N-oxydes.

2. Composés de formule (I) selon la revendication 1, dans lesquels les symboles ont les significations suivantes :
X représente oxygène,
R¹ représente hydrogène ou fluor,
G représente G1, G3 et G4,
R² représente un cycloalkyle en C₃-C₈, qui peut contenir jusqu'à deux substituants, les substituants étant choisis indépendamment les uns des autres dans la liste suivante :
cyano, halogène, hydroxy, oxo, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄ ou phényle,
R² représente également un phényle, qui peut contenir jusqu'à deux substituants, les substituants étant choisis indépendamment les uns des autres dans la liste suivante :
amino, halogène, cyano, hydroxy, SH, nitro, C(=O)H, alkyle en C₁-C₆, alcényle en C₂-C₆, alcynyle en C₂-C₆, halogénoalkyle en C₁-C₆, halogénalcényle en C₂-C₆, halogénoalcynyle en C₂-C₆, cycloalkyle en C₃-C₈, halogénocycloalkyle en C₃-C₈, alkylcycloalkyle en C₄-C₁₀, cycloalkylalkyle en C₄-C₁₀, cycloalkylcycloalkyle en C₆-C₁₄, halogénocycloalkylalkyle en C₄-C₁₀, alkylcycloalkylalkyle en C₅-C₁₀, cycloalcényle en C₃-C₈, halogénocycloalcényle en C₃-C₈, alcoxyalkyle en C₂-C₆, cycloalcoxyalkyle en C₄-C₁₀, alcoxyalcoxyalkyle en C₃-C₈, alkylthioalkyle en C₂-C₆, alkylsulfinylalkyle en C₂-C₆, alkylsulfonylalkyle en C₂-C₆, alkylaminoalkyle en C₂-C₆, dialkylaminoalkyle en C₃-C₈, halogénoalkylaminoalkyle en C₂-C₆, cycloalkylaminoalkyle en C₄-C₁₀, alkylcarbonyle en C₂-C₆, halogénoalkylcarbonyle en C₂-C₆, cycloalkylcarbonyle en C₄-C₈, alcoxycarbonyle en C₂-C₆, cycloalcoxycarbonyle en C₄-C₈, cycloalkylalcoxycarbonyle en C₅-C₁₀, alkylaminocarbonyle en C₂-C₆, dialkylaminocarbonyle en C₃-C₈, cycloalkylaminocarbonyle en C₄-C₈, halogénoalcoxyalkyle en C₂-C₆, hydroxyalkyle en C₁-C₆, alcoxy en C₁-C₆, halogénoalcoxy en C₁-C₆, cycloalcoxy en C₃-C₈, halogénocycloalcoxy en C₃-C₈, cycloalkylalcoxy en C₄-C₁₀, alcényloxy en C₂-C₆, halogénoalcényloxy en C₂-C₆, alcynyloxy en C₂-C₆, halogénoalcynyloxy en C₂-C₆, alcoxyalcoxy en C₂-C₆, alkylcarbonyloxy en C₂-C₆, halogénoalkylcarbonyloxy en C₂-C₆, cycloalkylcarbonyloxy en C₄-C₈, alkylcarbonylalcoxy en C₃-C₆, alkylthio en C₁-C₆, halogénoalkylthio en C₁-C₆, cycloalkylthio en C₃-C₆, alkylsulfinyle en C₁-C₆, halogénalkylsulfinyle en C₁-C₆, alkylsulfonyle en C₁-C₆, halogénalkylsulfonyle en C₁-C₆, cycloalkylsulfonyle en C₃-C₈, tri(alkyle en C₁-C₄) silyle, alkylsulfonylamino en C₁-C₆, halogénoalkylsulfonylamino en C₁-C₆ ou -LQ,
R² représente également un radical hétéroaryle à 5 ou 6 éléments, qui peut contenir jusqu'à deux substituants, les substituants étant choisis indépendamment les uns des autres dans la liste suivante :
substituants sur le carbone : halogène, cyano, nitro, hydroxy, SH, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, tri(alkyle en C₁-C₄)silyle ou -LQ,
substituants sur l'azote : alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, alkylsulfonyle en C₁-C₄, C(=O)H, C(=O)Me, C(=O)OMe, benzyle ou phényle,
R³ représente hydrogène, cyano ou alkyle en C₁-C₃,
L représente une liaison directe ou oxygène,
Q représente un phényle, qui peut contenir jusqu'à deux substituants, les substituants étant choisis indépendamment les uns des autres dans la liste suivante :
halogène, cyano, hydroxy, nitro, SH, alkyle en C₁-C₆, halogénoalkyle en C₁-C₆, cycloalkyle en C₃-C₈, alcoxy en C₁-C₄, halogénoalcoxy en C₁-C₄, alkylthio en C₁-C₄, halogénoalkylthio en C₁-C₄ ou phényle,
Q représente également un radical hétéroaryle à 5 ou 6 éléments, qui peut contenir jusqu'à deux substituants, les substituants étant choisis indépendamment les uns des autres dans la liste suivante :
substituants sur le carbone : halogène, cyano, nitro, SH, alkyle en C₁-C₆, halogénoalkyle en C₁-C₆, cycloalkyle en C₃-C₆, alcoxy en C₁-C₄, halogénoalcoxy en C₁-C₄, alkylthio en C₁-C₄, halogénoalkylthio en C₁-C₄ ou phényle,
substituants sur l'azote : alkyle en C₁-C₆, alcényle en C₂-C₆, alcynyle en C₂-C₆, cycloalkyle en C₃-C₆ ou phényle,
ainsi que leurs sels agrochimiquement actifs, leurs complexes métaliques et leurs N-oxydes.

3. Composés de formule (I) selon l'une qelconque des revendications précédentes, dans lesquels les symboles ont les significations suivantes :
G représente G3 et/ou G4 et notamment G3,
ainsi que leurs sels agrochimiquement actifs, leurs complexes métaliques et leurs N-oxydes.

4. Composés de formule (I) selon l'une qelconque des revendications précédentes, dans lesquels les symboles ont les significations suivantes :
R² représente un phényle, qui peut contenir jusqu'à deux substituants, les substituants étant choisis indépendamment les uns des autres dans la liste suivants :
amino, halogène, cyano, nitro, alkyle en C₁-C₆, alcényle en C₂-C₆, alcynyle en C₂-C₆, halogénoalkyle en C₁-C₆, halogénalcényle en C₂-C₆, halogénoalcynyle en C₂-C₆, cycloalkyle en C₃-C₈, halogénocycloalkyle en C₃-C₈, alcoxyalkyle en C₂-C₆, alkylcarbonyle en C₂-C₆, halogénoalkylcarbonyle en C₂-C₆, alcoxycarbonyle en C₂-C₆, alcoxy en C₁-C₆, halogénoalcoxy en C₁-C₆, cycloalcoxy en C₃-C₈, halogénocycloalcoxy en C₃-C₈, alcényloxy en C₂-C₆, alcynyloxy en C₂-C₆, alcoxyalcoxy en C₂-C₆, alkylthio en C₁-C₆, halogénoalkylthio en C₁-C₆, alkylsulfinyle en C₁-C₆, halogénalkylsulfinyle en C₁-C₆, alkylsulfonyle en C₁-C₆, halogénalkylsulfonyle en C₁-C₆, phényle, ainsi que leurs sels agrochimiquement actifs, leurs complexes métaliques et leurs N-oxydes.

5. Composés de formule (I) selon la revendication 4, dans lesquels
R² représente phényle, 2-fluorophényle, 2,6-difluorophényle, 2-acétylphényle, 3-acétylphényle, 2-hydroxyphényle, 2-nitrophényle, 2-[(2-méthoxyéthoxy)méthyl]phényle, 2-[(éthylsulfanyl)méthyl]phényle, 2-[(cyclopropylméthoxy)carbonyl]phényle, 2-(allyloxy)phényle, 3-(but-2-yn-1-yloxy)phényle, 2-(butoxyméthyl)phényle, 2-fluoro-6-formylphényle, 2-[(2-méthylprop-2-én-1-yl)oxy]phényle, 2-(2-méthoxyéthoxy)phényle, 2-[(3-méthylbut-2-én-1-yl)oxy]phényle, 3-(prop-2-yn-1-yloxy)phényle, 4-(prop-2-yn-1-yloxy)phényle, 3-formylphényle, 2-(cyclohexylméthoxy)phényle, 2-(pent-2-yn-1-yloxy)phényle, 2-formylphényle, 2-(cyclopropylcarbamoyl)phényle, 2-(but-2-yn-1-yloxy)-6-fluorophényle, 2-fluoro-6-(prop-2-yn-1-yloxy)phényle, 2-[(cyclohexylcarbonyl)oxy]phényle, 2-[(cyclopropylcarbonyl)oxy]phényle, 3-(pent-2-yn-1-yloxy)phényle, 2-(but-2-yn-1-yloxy)phényle, 2-[(3,3,3-trifluoropropanoyl)oxy]phényl, 2-[(méthylsulfonyl)amino]phényle, 2-éthynylphényle, 2-(prop-2-yn-1-yloxy)phényle, 4-[(méthylsulfonyl)amino]phényle, 2-aminophényle, 3-hydroxyphényle, 2-(méthoxycarbonyl)phényle, 2-(chlorométhyl)phényl, 4-(pent-2-yn-1-yloxy)phényle, 4-(but-2-yn-1-yloxy)phényle, 2-chloro-6-(prop-2-yn-1-yloxy)phényle, 2-chloro-6-(2-méthoxyéthoxy)phényle, 2-(allyloxy)-6-chlorophényle, 2-[(2,2,2-trifluoroéthoxy)méthyl]phényle, 2-[(éthylsulfonyl)méthyl]phényle ou 2-(hydroxyméthyl)phényle,
ainsi que leurs sels agrochimiquement actifs, leurs complexes métaliques et leurs N-oxydes.

6. Composés de formule (I) selon l'une quelconque des revendications précédentes, dans lesquels
R³ représente hydrogène,
ainsi que leurs sels agrochimiquement actifs, leurs complexes métaliques et leurs N-oxydes.

7. Composés de formule (I) selon l'une quelconque des revendications précédentes, dans lesquels
R^{Tz} représente hydrogène,
ainsi que leurs sels agrochimiquement actifs, leurs complexes métaliques et leurs N-oxydes.

8. Procédé de lutte contre les champignons phytopathogènes, **caractérisé en ce que** des composés de formule (I) selon l'une quelconque des revendications 1 à 7 sont appliqués sur les champignons phytopathogènes et/ou leur habitat.

9. Agent de lutte contre les champignons phytopathogènes, **caractérisé par** une teneur en au moins un composé de formule (I) selon l'une quelconque des revendications 1 à 7, en plus d'extendeurs et/ou de substances tensioactives.

10. Utilisation de dérivés de bis(difluorométhyl)pyrazole de formule (I) selon l'une quelconque des revendications 1 à 7 pour lutter contre les champignons phytopathogènes.

11. Procédé de fabrication d'agents de lutte contre les champignons phytopathogènes, **caractérisé en ce que** des dérivés de bis(difluorométhyl)pyrazole de formule (I) selon l'une quelconque des revendications 1 à 7 sont mélangés avec des extendeurs et/ou des substances tensioactives.

12. Utilisation de composés de formule (I) selon l'une quelconque des revendications 1 à 7 pour le traitement de plantes transgéniques.

13. Procédé de fabrication des composés de formule (I) selon l'une quelconque des revendications 1 à 7, comprenant au moins l'une des étapes (E) à (J) suivantes :
étape (E) : la cycloaddition d'un composé de formule générale (VIII) avec un composé de formule générale (IX) pour former des composés de formule (X-a) ou (Ia) en présence d'une base et éventuellement en présence d'un solvant selon le schéma réactionnel suivant (schéma 5) : 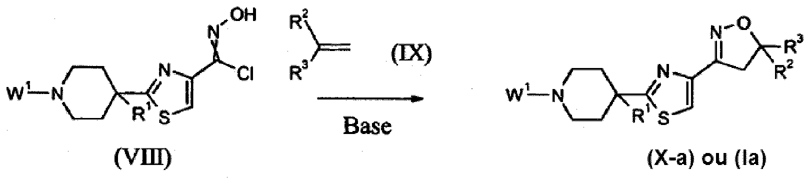 W¹ représentant acétyle, alcoxycarbonyle en C₁-C₄, benzyle, benzyloxycarbonyle ou [3,5-bis(difluorométhyl)-1H-pyrazol-1-yl]acétyle,
les radicaux R¹, R², R³ ayant la même signification que les radicaux R¹, R², R³ définis dans les revendications précédentes pour la formule (I) ;
étape (F) : la cycloaddition d'un composé de formule générale (VIII) avec un composé de formule générale (XI) pour former des composés de formule (X-b) ou (Ia) en présence d'une base et éventuellement en présence d'un solvant selon le schéma réactionnel suivant (schéma 6) : 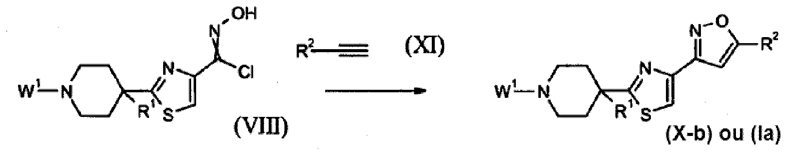 W¹ représentant acétyle, alcoxycarbonyle en C₁-C₄, benzyle, benzyloxycarbonyle ou [3,5-bis(difluorométhyl)-1H-pyrazol-1-yl]acétyle,
les radicaux R¹, R² ayant la même signification que les radicaux R¹, R² définis dans les revendications précédentes pour la formule (I) ;
étape (G) : l'élimination du groupe protecteur des composés de formule générale (X) pour obtenir des composés de formule générale (XII) selon le schéma réactionnel suivant (schéma 7) : 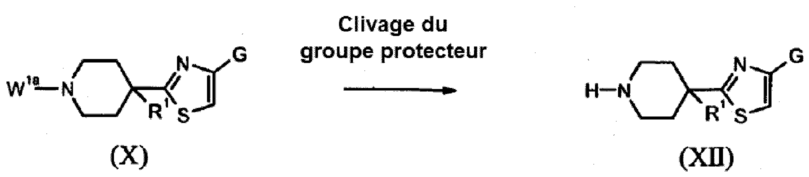 W^{1a} représentant acétyle, alcoxycarbonyle en C₁-C₄, benzyle ou benzyloxycarbonyle, les radicaux R¹, G ayant la même signification que les radicaux R¹, G définis dans les revendications précédentes pour la formule (I) ;
étape (H) : la réaction de couplage des composés de formule générale (XII) avec un substrat de formule générale (XIII) pour obtenir des composés de formule générale (Ia) selon le schéma réactionnel suivant (schéma 8) : 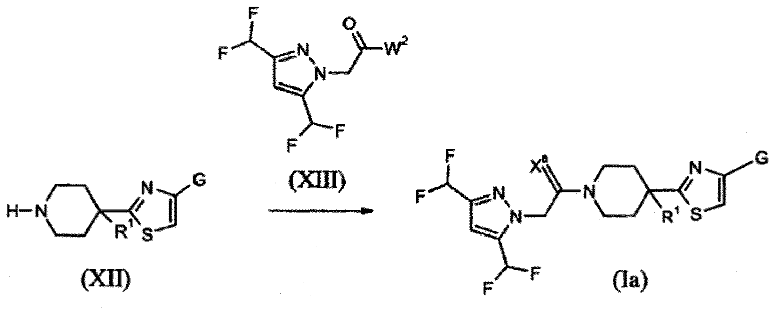 W² représentant chlore ou OH,
X^{a} représentant oxygène,
les radicaux R¹, G ayant la même signification que les radicaux R¹, G définis dans les revendications précédentes pour la formule (I) ;
étape (I) : la sulfuration des composés selon la formule générale (I-a) pour obtenir des composés de formule générale (I-c) selon le schéma réactionnel suivant (schéma 9) : 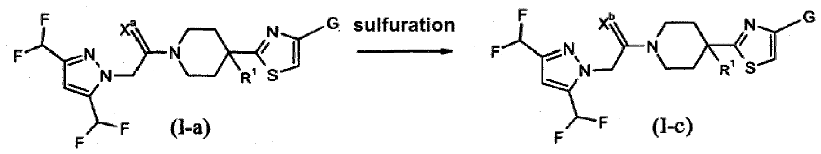
X^{a} représentant l'oxygène,
X^{b} représentant le soufre,
les radicaux R¹, G ayant la même signification que les radicaux R¹, G définis dans les revendications précédentes pour la formule (I) ;
étape (J) : l'halogénation des composés selon la formule générale (X-a) ou (I-a) pour obtenir des composés de formule générale (X-d) ou (I-d) selon le schéma réactionnel suivant (schéma 10) : 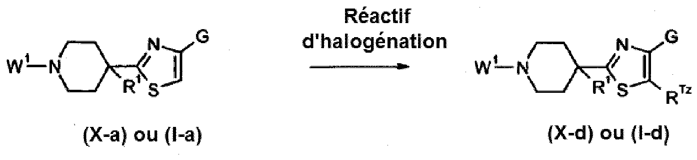 W¹ représentant acétyle, alcoxycarbonyle en C₁-C₄, benzyle, benzyloxycarbonyle ou [3,5-bis(difluorométhyl)-1H-pyrazol-1-yl]acétyle,
R^{Tz} représentant un halogène,
les radicaux R¹, G ayant la même signification que les radicaux R¹, G définis dans les revendications précédentes pour la formule (I).
